# EUROPEAN PATENT SPECIFICATION

(11) **EP 3 436 577 B1**
(45) Date of publication and mention of the grant of the patent: **17.06.2026**
(21) Application number: 17720281.9
(22) Date of filing: 30.03.2017
(51) Int. Cl.: C12N 9/26

(54) **METHOD FOR SELECTION OF HIGH M6P RECOMBINANT PROTEINS**
VERFAHREN ZUR SELEKTION VON HÖHE M6P REKOMBINANTE PROTEINE
METHODE DE SELECTION DE PROTEINE RECOMBINANTE M6P ELEVEES

(30) Priority: 30.03.2016 US 201662315400 P; 10.02.2017 US 201762457584 P
(43) Date of publication of application: 06.02.2019
(60) Divisional application: 26175131.7
(73) Proprietor: Amicus Therapeutics, Inc., Philadelphia, PA 19104 (US)
(72) Inventor: DO, Hung V., Cranbury, New Jersey 08512 (US); GOTSCHALL, Russell, Cranbury, New Jersey 08512 (US)
(74) Representative: Hoffmann Eitle
(86) International application number: PCT/US2017/024981
(87) International publication number: WO 2017/173059

(56) References cited:
- EP-A2- 1 820 862
- WO-A1-2016/054231
- WO-A2-2005/077093
- WO-A2-2006/125141
- WO-A2-2009/102895
- WO-A2-2011/039634
- WO-A2-2012/042386
- WO-A2-2013/136189
- US-A1- 2002 073 438
- VAN HOVE J L K ET AL: "Purification of recombinant human precursor acid alpha-glucosidase", BIOCHEMISTRY AND MOLECULAR BIOLOGY INTERNATIONAL, ACADEMIC PRESS, LONDON, GB, vol. 43, no. 3, 1 October 1997 (1997-10-01), pages 613 - 623, XP002108774, ISSN: 1039-9712
- DATABASE EMBASE [online] ELSEVIER SCIENCE PUBLISHERS, AMSTERDAM, NL; June 2007 (2007-06-01), JONGEN S P ET AL: "N-glycans of recombinant human acid [alpha]-glucosidase expressed in the milk of transgenic rabbits", XP002770407, Database accession no. EMB-2007342042
- TOONKOOL P ET AL: "Expression and purification of dalcochinase, a beta-glucosidase from Dalbergia cochinchinensis Pierre, in yeast and bacterial hosts", PROTEIN EXPRESSION AND PURIFICATION, ACADEMIC PRESS, SAN DIEGO, CA, vol. 48, no. 2, 1 August 2006 (2006-08-01), pages 195 - 204, XP024908821, ISSN: 1046-5928, [retrieved on 20060801]
- DOROTA HOJA-LUKOWICZ ET AL: "Characterization of the oligosaccharide component of microsomal [beta]-glucuronidase from rat liver", BIOCHIMIE, vol. 86, no. 6, 1 June 2004 (2004-06-01), FR, pages 363 - 372, XP055375366, ISSN: 0300-9084, DOI: 10.1016/j.biochi.2004.05.008

## Description

### TECHNICAL FIELD

Principles and embodiments of the present invention as defined in the claims relate generally to the manufacturing of recombinant proteins, particularly lysosomal enzymes that have a high content of mannose-6-phosphate.

### BACKGROUND

Lysosomal storage disorders are a group of autosomal recessive genetic diseases characterized by the accumulation of cellular glycosphingolipids, glycogen, or mucopolysaccharides within intracellular compartments called lysosomes. Individuals with these diseases carry mutant genes coding for enzymes which are defective in catalyzing the hydrolysis of one or more of these substances, which then build up in the lysosomes. For example, Pompe disease, also known as acid maltase deficiency or glycogen storage disease type II, is one of several lysosomal storage disorders. Other examples of lysosomal disorders include Gaucher disease, GM1-gangliosidosis, fucosidosis, mucopolysaccharidoses, Hurlcr-Scheie disease, Niemann-Pick A and B diseases, and Fabry disease. Pompe disease is also classified as a neuromuscular disease or a metabolic myopathy.

Pompe disease is estimated to occur in about 1 in 40,000 births, and is caused by a mutation in the GAA gene, which codes for the enzyme lysosomal α-glucosidase (EC:3.2.1.20), also commonly known as acid α-glucosidase. Acid α-glucosidase is involved in the metabolism of glycogen, a branched polysaccharide which is the major storage form of glucose in animals, by catalyzing its hydrolysis into glucose within the lysosomes. Because individuals with Pompe disease produce mutant, defective acid α-glucosidase which is inactive or has reduced activity, glycogen breakdown occurs slowly or not at all, and glycogen accumulates in the lysosomes of various tissues, particularly in striated muscles, leading to a broad spectrum of clinical manifestations, including progressive muscle weakness and respiratory insufficiency. Tissues such as the heart and skeletal muscles are particularly affected.

Pompe disease can vary widely in the degree of enzyme deficiency, severity and age of onset, and over 500 different mutations in the GAA gene have been identified, many of which cause disease symptoms of varying severity. The disease has been classified into broad types: early onset or infantile and late onset. Earlier onset of disease and lower enzymatic activity are generally associated with a more severe clinical course. Infantile Pompe disease is the most severe, resulting from complete or near complete acid α-glucosidase deficiency, and presents with symptoms that include severe lack of muscle tone, weakness, enlarged liver and heart, and cardiomyopathy. The tongue may become enlarged and protrude, and swallowing may become difficult. Most affected children die from respiratory or cardiac complications before the age of two. Late onset Pompe disease can present at any age older than 12 months and is characterized by a lack of cardiac involvement and better short-term prognosis. Symptoms are related to progressive skeletal muscle dysfunction, and involve generalized muscle weakness and wasting of respiratory muscles in the trunk, proximal lower limbs, and diaphragm. Some adult patients are devoid of major symptoms or motor limitations. Prognosis generally depends on the extent of respiratory muscle involvement. Most subjects with Pompe disease eventually progress to physical debilitation requiring the use of a wheelchair and assisted ventilation, with premature death often occurring due to respiratory failure.

Recent treatment options for Pompe disease include enzyme replacement therapy (ERT) with recombinant human acid α-glucosidase (rhGAA). Conventional rhGAA products are known under the names alglucosidase alfa, Myozyme^{®} or Lumizyme^{®}, from Genzyme, Inc. ERT is a chronic treatment required throughout the lifetime of the patient, and involves administering the replacement enzyme by intravenous infusion. The replacement enzyme is then transported in the circulation and enters lysosomes within cells, where it acts to break down the accumulated glycogen, compensating for the deficient activity of the endogenous defective mutant enzyme, and thus relieving the disease symptoms. In subjects with infantile onset Pompe disease, treatment with alglucosidase alfa has been shown to significantly improve survival compared to historical controls, and in late onset Pompe disease, alglucosidase alfa has been shown to have a statistically significant, if modest, effect on the 6-Minute Walk Test (6MWT) and forced vital capacity (FVC) compared to placebo.

However, the majority of subjects either remain stable or continue to deteriorate while undergoing treatment with alglucosidase alfa. The reason for the apparent sub-optimal effect of ERT with alglucosidase alfa is unclear, but could be partly due to the progressive nature of underlying muscle pathology, or the poor tissue targeting of the current ERT. For example, the infused enzyme is not stable at neutral pH, including at the pH of plasma (about pH 7.4), and can be irreversibly inactivated within the circulation. Furthermore, infused alglucosidase alfa shows insufficient uptake in key disease-relevant muscles, possibly due to inadequate glycosylation with mannose-6-phosphate (M6P) residues. Such residues bind cation-independent mannose-6-phosphate receptors (CIMPR) at the cell surface, allowing the enzyme to enter the cell and the lysosomes within. Therefore, high doses of the enzyme may be required for effective treatment so that an adequate amount of active enzyme can reach the lysosomes, making the therapy costly and time-consuming.

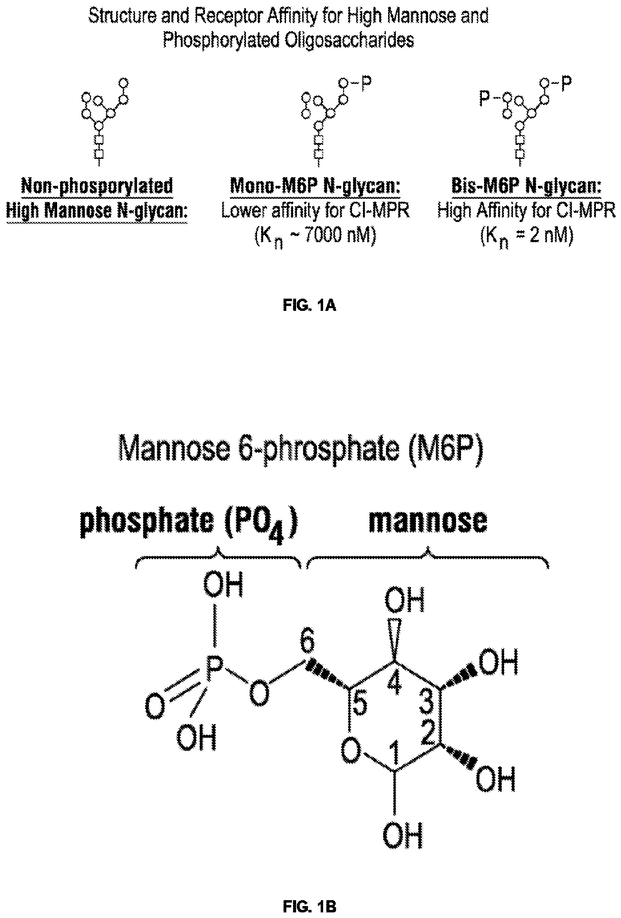

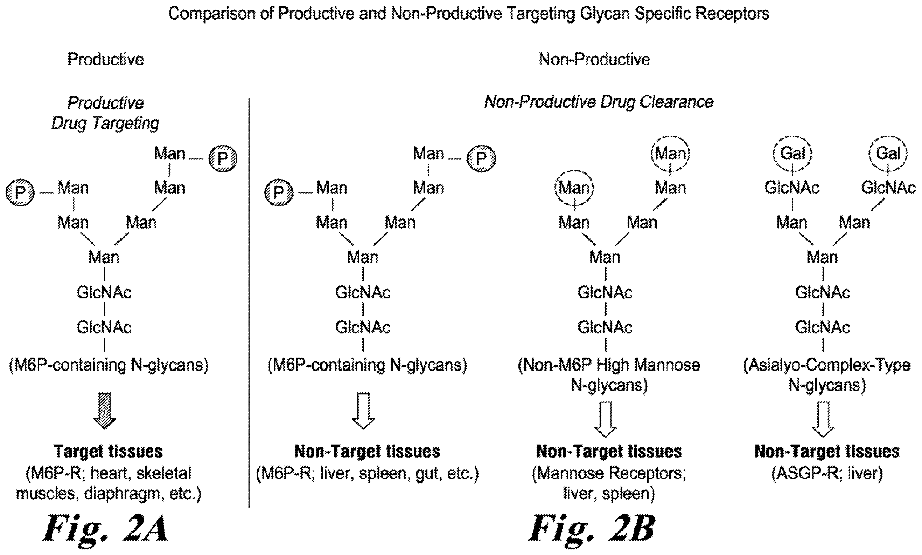

There are seven potential N-linked glycosylation sites on rhGAA. Since each glycosylation site is heterogeneous in the type of N-linked oligosaccharides (N-glycans) present, rhGAA consist of a complex mixture of proteins with N-glycans having varying binding affinities for M6P receptor and other carbohydrate receptors. rhGAA that contains a high mannose N-glycans having one M6P group (mono-M6P) binds to CIMPR with low (~6,000 nM) affinity while rhGAA that contains two M6P groups on same N-glycan (bis-M6P) bind with high (~2 nM) affinity. Representative structures for non-phosphorylated, mono-M6P, and bis-M6P glycans are shown by Figure 1A. The mannose-6-P group is shown by Figure 1B. Once inside the lysosome, rhGAA can enzymatically degrade accumulated glycogen. However, conventional rhGAAs have low total levels of M6P- and bis-M6P bearing glycans and, thus, target muscle cells poorly resulting in inferior delivery of rhGAA to the lysosomes. Productive drug targeting of rhGAA is shown in Figure 2A. The majority of rhGAA molecules in these conventional products do not have phosphorylated N-glycans, thereby lacking affinity for the CIMPR. Non-phosphorylated high mannose glycans can also be cleared by the mannose receptor which results in non-productive clearance of the ERT (Figure 2B).

The other type of N-glycans, complex carbohydrates, which contain galactose and sialic acids, are also present on rhGAA. Since complex N-glycans are not phosphorylated they have no affinity for CIMPR. However, complex-type N-glycans with exposed galactose residues have moderate to high affinity for the asialoglycoprotein receptor on liver hepatocytes which leads to rapid non-productive clearance of rhGAA (Figure 2B).

Current manufacturing processes used to make conventional rhGAA, such as Myozyme^{®}, Lumizyme^{®} or alglucosidase alfa, have not significantly increased the content of M6P or bis-M6P because cellular carbohydrate processing is naturally complex and extremely difficult to manipulate. Accordingly, there remains a need for further improvements to enzyme replacement therapy for treatment of Pompe disease, such as new manufacturing, capturing and purification processes for rhGAA.

Similarly, other recombinant proteins that are targeted to the lysosome, such as other lysosomal enzymes, also bind CIMPR. However, current manufacturing processes used to make other conventional recombinant proteins that are targeted to lysosomes do not provide recombinant proteins with a high content of M6P or bis-M6P. Accordingly, there remains a need for further improvements in the manufacturing, capturing and purification processes for these other recombinant proteins as well.
WO 2013/136189 discloses methods and materials for treatment of Pompe disease. US 2002/073438 discloses methods of purifying human acid alpha-glucosidase. WO 2005/077093 discloses manufacture of highly phosphorylated lysosomal enzymes and uses thereof.

### SUMMARY

One aspect of the present invention is related to a method for producing recombinant human acid alpha-glucosidase, as defined in the claims. In various embodiments of this aspect, the method comprises culturing host cells in a bioreactor that secrete a recombinant human lysosomal protein, removing media from the bioreactor, filtering the media to provide a filtrate, loading the filtrate onto an anion exchange chromatography (AEX) column to capture the lysosomal protein and eluting a first protein product from the AEX column.

The recombinant human lysosomal protein is recombinant human α-glucosidase (rhGAA). The rhGAA comprises an amino acid sequence that is at least 98% identical to SEQ ID NO: 2.

The method further comprises loading the first protein product onto a chromatography column, and eluting the protein product from the column. The column is an immobilized metal affinity chromatography (IMAC) column, as defined in the claims.

The method may further comprise loading the rhGAA eluted from the IMAC column onto a third chromatography column and eluting the rhGAA from the third chromatography column. In some embodiments, the third chromatography column is selected from a cation exchange chromatography (CEX) column and a size exclusion chromatography (SEC) column.

In one or more embodiments, filtering the media is selected from alternating tangential flow filtration (ATF) and tangential flow filtration (TFF).

In one or more embodiments, the method further comprises inactivating viruses in one or more of the first protein product, the second protein product and the third protein product.

In one or more embodiments, the method further comprises filtering the rhGAA eluted from the IMAC column or the rhGAA eluted from the third chromatography column to provide a filtered product and filling a vial with the filtered product. In one or more embodiments, the method further comprises lyophilizing the filtered product.

In one or more embodiments, the host cells comprise Chinese hamster ovary (CHO) cells. In some embodiments, the host cells comprise CHO cell line GA-ATB-200 or ATB-200-X5-14 or a subculture thereof.

In one or more embodiments, (i) at least 90% of the purifed rhGAA binds to cation-independent manosc-6-phosphate receptor (CIMPR) or (ii) at least 90% of the purified rhGAA comprises an N-glycan carrying mono-mannose-6-phosphate (mono-M6P) or bis-mannose-6-phosphate (bis-M6P).

In one or more embodiments, the rhGAA comprising seven potential N-glycosylation sites, at least 50% of molecules of the rhGAA comprise an N-glycan unit bearing two mannose-6-phosphate residues at the first site, at least 30% of molecules of the rhGAA comprise an N-glycan unit bearing one mannose-6-phosphate residue at the second site, at least 30% of molecules of the rhGAA comprise an N-glycan unit bearing two mannose-6-phosphate residue at the fourth site, and at least 20% of molecules of the rhGAA comprise an N-glycan unit bearing one mannose-6-phosphate residue at the fourth site.

Another aspect of the present invention is related to a recombinant protein product made by the method set out in the claims.

Another aspect useful for illustrating the present invention is related to pharmaceutical composition comprising the recombinant protein product and a pharmaceutically acceptable carrier.

Yet another aspect useful for illustrating the present invention is related to medical uses for treating a lysosomal storage disorder, comprising administering the pharmaceutical composition to a patient in need thereof. Pharmaceutical compositions and medical uses are not claimed as such.

In one or more embodiments, the lysosomal storage disorder is Pompe disease and the recombinant protein is rhGAA. In one or more embodiments, the patient is co-administered a pharmacological chaperone for α-glucosidase within 4 hours of the administration of the pharmaceutical composition comprising the rhGAA product. In some embodiments, the pharmacological chaperone is selected from 1-deoxynojirimycin and N-butyl-deoxynojirimycin. In some embodiments, the pharmacological chaperone is co-formulated with the rhGAA product.

### BRIEF DESCRIPTION OF THE DRAWINGS

Further features of the present invention as defined in the claims will become apparent from the following written description and the accompanying figures, in which:
Figure 1A shows non-phosphorylated high mannose glycan, a mono-M6P glycan, and a bis-M6P glycan.
Figure 1B shows the chemical structure of the M6P group.
Figure 2A describes productive targeting of rhGAA via glycans bearing M6P to target tissues (e.g. muscle tissues of subject with Pompe Disease).
Figure 2B describes non-productive drug clearance to non-target tissues (e.g. liver and spleen) or by binding of non-M6P glycans to non-target tissues.

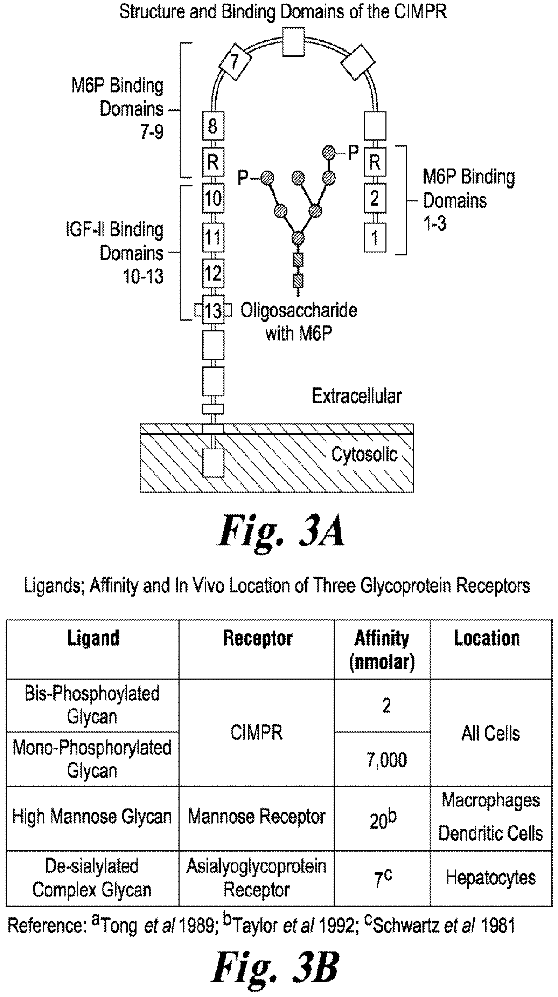

Figure 3A graphically depicts a CIMPR receptor (also known as an IGF2 receptor) and domains of this receptor.
Figure 3B is a table showing binding affinity (nmolar) of glycans bearing bis- and mono-M6P for CIMPR, the binding affinity of high mannose-type glycans to mannose receptors, and the binding affinity of desialylated complex glycan for asialyoglycoprotein receptors. RhGAA that has glycans bearing M6P and bis-M6P can productively bind to CIMPR on muscle.

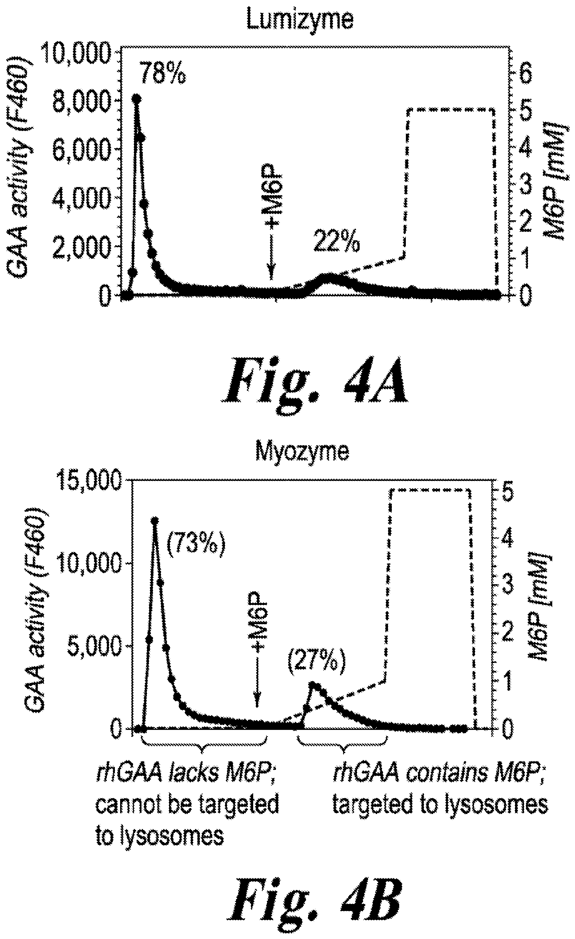

Figures 4A and 4B, respectively, are graphs showing the results of CIMPR affinity chromatography of Lumizyme^{®} and Myozyme^{®}. The dashed lines refer to the M6P elution gradient. Elution with M6P displaces GAA molecules bound via an M6Pcontaining glycan to CIMPR. As shown in Figure 2A, 78% of the GAA activity in Lumizyme^{®} eluted prior to addition of M6P. Figure 2B shows that 73% of the GAA Myozyme^{®} activity eluted prior to addition of M6P. Only 22% or 27% of the rhGAA in Lumizyme^{®} or Myozyme^{®}, respectively, was eluted with M6P. These figures show that most of the rhGAA in these two conventional rhGAA products lack glycans having M6P needed to target CIMPR in target muscle tissues.
Figure 5 shows a DNA construct for transforming CHO cells with DNA encoding rhGAA. CHO cells were transformed with a DNA construct encoding rhGAA.
Figure 6 is a schematic diagram of an exemplary process for the manufacturing, capturing and purification of a recombinant lysosomal protein.
Figures 7A and 7B, respectively, are graphs showing the results of CIMPR affinity chromatography of Myozyme^{®} and ATB200 rhGAA. As apparent from Figure 7B, about 70% of the rhGAA in ATB200 rhGAA contained M6P.
Figure 8 is a graph showing the results of CIMPR affinity chromatography of ATB200 rhGAA with and without capture on an anion exchange (AEX) column.
Figure 9 is a graph showing Polywax elution profiles of Lumizyme^{®} and ATB200 rhGAAs.
Figure 10 is a table showing a summary of N-glycan structures of Lumizyme^{®} compared to three different preparations of ATB200 rhGAA, identified as BP-rhGAA, ATB200-1 and ATB200-2.
Figures 11A-11H show the results of a site-specific N-glycosylation analysis of ATB200 rhGAA.
Figure 12A is a graph comparing the CIMPR binding affinity of ATB200 rhGAA (left trace) with that of Lumizyme^{®} (right trace).
Figure 12B is a table comparing the Bis-M6P content of Lumizyme^{®} and ATB200 rhGAA.
Figure 13A is a graph comparing ATB200 rhGAA activity (left trace) with Lumizyme^{®} rhGAA activity (right trace) inside normal fibroblasts at various GAA concentrations.
Figure 13B is a table comparing ATB200 rhGAA activity (left trace) with Lumizyme^{®} rhGAA activity (right trace) inside fibroblasts from a subject having Pompe Disease at various GAA concentrations.
Figure 13C is a table comparing Kᵤₚₜₐₖₑ of fibroblasts from normal subjects and subjects with Pompe Disease.
Figure 14A is a graph showing the amount of glycogen relative to dose of recombinant human acid α-glucosidase in mouse heart muscle after contact with vehicle (negative control), with 20 mg/ml alglucosidase alfa (Lumizyme^{®}), or with 5, 10 or 20 mg/kg ATB200.
Figure 14B is a graph showing the amount of glycogen relative to dose of recombinant human acid α-glucosidase in mouse quadriceps muscle after contact with vehicle (negative control), with 20 mg/ml alglucosidase alfa (Lumizyme^{®}), or with 5, 10 or 20 mg/kg ATB200.
Figure 14C is a graph showing the amount of glycogen relative to dose of recombinant human acid α-glucosidase in mouse triceps muscle after contact with vehicle (negative control), with 20 mg/ml alglucosidase alfa (Lumizyme^{®}), or with 5, 10 or 20 mg/kg ATB200.
Figure 15 is a table showing that the combination of ATB200 rhGAA and chaperone miglustat provided significantly better glycogen clearance in GAA knock-out mice than treatments with either Lumizyme^{®} or ATB200 rhGAAs without the miglustat chaperone.
Figure 16 is a series of electron micrographs of heart, diaphragm and soleus muscle from wild-type and Gaa-knockout mice treated with vehicle, alglucosidase alfa and ATB200 in the presence and absence of miglustat, showing levels of lysosome associated membrane protein (LAMP-1).
Figure 17 is a series of electron micrographs of heart and soleus muscle from wild-type and Gaa-knockout mice treated with vehicle, alglucosidase alfa and ATB200 in the presence and absence of miglustat, showing glycogen levels by staining with periodic acid - Schiff reagent (PAS).
Figure 18 is a series of electron micrographs (1000x) of quadriceps muscle from wild-type and Gaa-knockout mice treated with vehicle, alglucosidase alfa and ATB200 in the presence and absence of miglustat, stained with methylene blue to show vacuoles (indicated by arrows).
Figure 19 is a series of electron micrographs (40x) of quadriceps muscle from wild-type and Gaa-knockout mice treated with vehicle, alglucosidase alfa and ATB200 in the presence and absence of miglustat, showing levels of the autophagy markers microtubule-associated protein 1A/1B-light chain 3 phosphatidylethanolamine conjugate (LC3A II) and p62, the insulin-dependent glucose transporter GLUT4 and the insulin-independent glucose transporter GLUT1.
Figures 20A and 20B, respectively, are graphs showing the results of CIMPR affinity chromatography of recombinant α-galactosidase A (rhα-Gal A) enzyme before and after capture and purification on an anion exchange (AEX) column.
Figures 21A and 21B are graphs showing wire hand and grip strength muscle data for wild-type and Gaa-knockout mice treated with vehicle, alglucosidase alfa and ATB200 in the presence of miglustat.
Figures 22A-22G are graphs showing glycogen levels in quadriceps, triceps and heart cells from wild-type and Gaa-knockout mice treated with vehicle, alglucosidase alfa and ATB200 in the presence and absence of miglustat.
Figure 23 is a series of photomicrographs (100x and 200x) of muscle fibers of vastus lateralis (VL) from wild-type and Gaa-knockout mice treated with vehicle, alglucosidase alfa and ATB200 in the presence and absence of miglustat, showing dystrophin signals.
Figure 24 shows the study design of an open-label, fixed-sequence, ascending-dose, first-in-human, phase 1/2 study to assess the safety, tolerability, PK, PD, and efficacy of intravenous infusions of ATB200 co-administered with oral miglustat in adults with Pompe disease.
Figures 25A-25B are graphs showing the concentration-time profiles of GAA total protein in plasma in human subjects after dosing of 5, 10 or 20 mg/kg ATB200, 20 mg/kg ATB200 and 130 mg miglustat, or 20 mg/kg ATB200 and 260 mg miglustat.
Figure 25C is a graph showing the AUC of GAA total protein in plasma in human subjects after dosing of 20 mg/kg ATB200, 20 mg/kg ATB200 and 130 mg miglustat, or 20 mg/kg ATB200 and 260 mg miglustat.
Figure 25D is a graph showing the concentration-time profiles of GAA total protein in plasma in two individual human subjects after dosing of 20 mg/kg ATB200 and 260 mg miglustat.
Figure 26 is a graph showing the concentration-time profiles of miglustat in plasma in human subjects after dosing of 130 mg or 260 mg of miglustat.
Figures 27A-27D are graphs showing changes in alanine aminotransferase (ALT), aspartate aminotransferase (AST), creatine phosphokinase (CPK) and hexose tetrasaccharide (Hex4) levels in human patients after administration of ascending doses of ATB200 (5, 10 and 20 mg/kg) followed by co-administration of ATB200 (20 mg/kg) and miglustat (130 and 260 mg).

### DETAILED DESCRIPTION

The invention is defined in the claims.

Although specific reference is made to GAA, it will be understood by a person having ordinary skill in the art that the methods described herein may be used to produce, capture and purify other recombinant proteins that target the lysosome, including but not limited to the lysosomal enzyme α-galactosidase A.

Various aspects of the invention as defined in the claims pertain to new methods for the production, capturing and purification of useful for illustrating recombinant human acid α-glucosidase (rhGAA). Other aspects the invention pertain to recombinant proteins produced by the processes described herein, as well as pharmaceutical compositions, medical uses and uses of such recombinant proteins. Pharmaceutical compositions and medical uses are not claimed as such.

### Definitions

The terms used in this specification generally have their ordinary meanings in the art, within the context of this invention and in the specific context where each term is used. Certain terms are discussed below, or elsewhere in the specification, to provide additional guidance to the practitioner in describing the methods of the invention as defined in the claims and how to make and use them.

In the present specification, except where the context requires otherwise due to express language or necessary implication, the word "comprises", or variations such as "comprises" or "comprising" is used in an inclusive sense i.e. to specify the presence of the stated features but not to preclude the presence or addition of further features in various embodiments of the invention as defined in the claims.

As used herein, the term "lysosomal protein" refers to any protein that is targeted to the lysosome, such as a lysosomal enzyme. Examples of lysosomal enzymes and the associated disease are provided in Table 1 below:

**Table 1**

| **Lysosomal Enzyme** | **Disease** |
|---|---|
| Acid α-glucosidase | Pompe disease |
| α-galactosidase A | Fabry disease |
| Acid β-glucosidase | Gaucher disease |
| α-L-iduronidase | Hurler-Scheie disease |
| Iduronate sulfatase | Hunter disease |
| β-galactosidase | GM1-gangliosidosis; Morquio disease B |
| β-glucuronidase | Sly disease (MPS VI) |
| α-fucosidase | Fucosidosis |
| Acid sphingomyelinase | Niemann-Pick A and B |
| β-hexosaminidase A | Tay Sachs disease |
| β-hexosaminidase B | Sandhoff disease |
| β-galactocerebrosidase | Krabbe disease |
| Acid ceramidase | Farber disease |
| Heparan-N-sulfatase | Sanfilippo disease A (MPS IIIa) |
| α-N-acetyl-glucosaminidase | Sanfilippo disease B (MPS IIIb) |
| α-glucosaminide N-acetyltransferase | Sanfilippo disease C (MPS IIIc) |
| N-acetylglucosamine-6-sulfate sulfatase | Sanfilippo disease D (MPS IIId) |
| N-acetylgalactosamine-6-sulfate sulfatase | Morquio disease A (MPS Ivb) |
| Arylsulfatase A | Metachromatic leukodystrophy |
| Arylsulfatase B | Maroteaux-Lamy (MPS VI) |
| Acid lipase | Wolf disease |
| acid α-mannosidase | α-mannosidosis |
| acid β-mannosidase | β-mannosidosis |
| α-N-acetyl-neuraminidase | Sialidosis |
| α-N-acetylgalactosaminidase | Schindler-Kanzaki disease |
| N-aspartyl-β-glucosaminidase | Aspartylglucosaminuria |

As used herein, the term "Pompe disease," also referred to as acid maltase deficiency, glycogen storage disease type II (GSDII), and glycogenosis type II, is intended to refer to a genetic lysosomal storage disorder characterized by mutations in the GAA gene, which codes for the human acid α-glucosidase enzyme. The term includes but is not limited to early and late onset forms of the disease, including but not limited to infantile, juvenile and adult-onset Pompe disease.

As used herein, the term "acid α-glucosidase" is intended to refer to a lysosomal enzyme which hydrolyzes α-1,4 linkages between the D-glucose units of glycogen, maltose, and isomaltose. Alternative names include but are not limited to lysosomal α-glucosidase (EC:3.2.1.20); glucoamylase; 1,4-α-D-glucan glucohydrolase; amyloglucosidase; gamma-amylase and exo-1,4-α-glucosidase. Human acid α-glucosidase is encoded by the GAA gene (National Centre for Biotechnology Information (NCBI) Gene ID 2548), which has been mapped to the long arm of chromosome 17 (location 17q25.2-q25.3). More than 500 mutations have currently been identified in the human GAA gene, many of which are associated with Pompe disease. Mutations resulting in misfolding or misprocessing of the acid α-glucosidase enzyme include T1064C (Leu355Pro) and C2104T (Arg702Cys). In addition, GAA mutations which affect maturation and processing of the enzyme include Leu405Pro and Met519Thr. The conserved hexapeptide WIDMNE at amino acid residues 516-521 is required for activity of the acid α-glucosidase protein. As used herein, the abbreviation "GAA" is intended to refer to the acid α-glucosidase enzyme, while the italicized abbreviation "GAA" is intended to refer to the human gene coding for the human acid α-glucosidase enzyme The italicized abbreviation "Gaa" is intended to refer to non-human genes coding for non-human acid α-glucosidase enzymes, including but not limited to rat or mouse genes, and the abbreviation "Gaa" is intended to refer to non-human acid α-glucosidase enzymes. Thus, the abbreviation "rhGAA" is intended to refer to the recombinant human acid α-glucosidase enzyme.

As used herein, the term "alglucosidase alfa" is intended to refer to a recombinant human acid α-glucosidase identified as [199-arginine,223-histidine]prepro-α-glucosidase (human); Chemical Abstracts Registry Number 420794-05-0. Alglucosidase alfa is approved for marketing in the United States by Genzyme, as of January 2016, as the products Lumizyme^{®} and Myozyme^{®}.

As used herein, the term "ATB200" is intended to refer to a recombinant human acid α-glucosidase described in co-pending patent application PCT/US2015/053252

As used herein, the term "glycan" is intended to refer to a polysaccharide chain covalently bound to an amino acid residue on a protein or polypeptide. As used herein, the term "N-glycan" or "N-linked glycan" is intended to refer to a polysaccharide chain attached to an amino acid residue on a protein or polypeptide through covalent binding to a nitrogen atom of the amino acid residue. For example, an N-glycan can be covalently bound to the side chain nitrogen atom of an asparagine residue. Glycans can contain one or several monosaccharide units, and the monosaccharide units can be covalently linked to form a straight chain or a branched chain. In at least one embodiment, N-glycan units attached to ATB200 can comprise one or more monosaccharide units each independently selected from N-acetylglucosamine, mannose, galactose or sialic acid. The N-glycan units on the protein can be determined by any appropriate analytical technique, such as mass spectrometry. In some embodiments, the N-glycan units can be determined by liquid chromatography-tandem mass spectrometry (LC-MS/MS) utilizing an instrument such as the Thermo Scientific Orbitrap Velos Pro^{™} Mass Spectrometer, Thermo Scientific Orbitrap Fusion Lumos Tribid^{™} Mass Spectrometer or Waters Xevo^{®} G2-XS QTof Mass Spectrometer.

As used herein, the term "high-mannose N-glycan" is intended to refer to an N-glycan having one to six or more mannose units. In at least one embodiment, a high mannose N-glycan unit can contain a bis(N-acetylglucosamine) chain bonded to an asparagine residue and further bonded to a branched polymannose chain. As used herein interchangeably, the term "M6P" or "mannose-6-phosphate" is intended to refer to a mannose unit phosphorylated at the 6 position; i.e. having a phosphate group bonded to the hydroxyl group at the 6 position. In at least one embodiment, one or more mannose units of one or more N-glycan units are phosphorylated at the 6 position to form mannose-6-phosphate units. In at least one embodiment, the term "M6P" or "mannose-6-phosphate" refers to both a mannose phosphodiester having N-acetylglucosamine (GlcNAc) as a "cap" on the phosphate group, as well as a mannose unit having an exposed phosphate group lacking the GlcNAc cap. In at least one embodiment, the N-glycans of a protein can have multiple M6P groups, with at least one M6P group having a GlcNAc cap and at least one other M6P group lacking a GlcNAc cap.

As used herein, the term "complex N-glycan" is intended to refer to an N-glycan containing one or more galactose and/or sialic acid units. In at least one embodiment, a complex N-glycan can be a high-mannose N-glycan in which one or mannose units are further bonded to one or more monosaccharide units each independently selected from N-acetylglucosamine, galactose and sialic acid.

As used herein, the compound miglustat, also known as N-butyl-1-deoxynojirimycin NB-DNJ or (2R,3R,4R,5S)-1-butyl-2-(hydroxymethyl)piperidine-3,4,5-triol, is a compound having the following chemical formula:

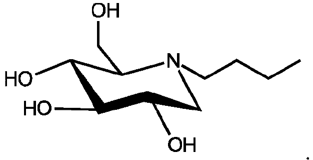

One formulation of miglustat is marketed commercially under the trade name Zavesca^{®} as monotherapy for type 1 Gaucher disease.

As discussed below, pharmaceutically acceptable salts of miglustat may also be useful with the present invention. When a salt of miglustat is used, the dosage of the salt will be adjusted so that the dose of miglustat received by the patient is equivalent to the amount which would have been received had the miglustat free base been used.

As used herein, the compound duvoglustat, also known as 1-deoxynojirimycin or DNJ or (2R,3R,4R,5S)-2-(hydroxymethyl)piperidine-3,4,5-triol, is a compound having the following chemical formula:

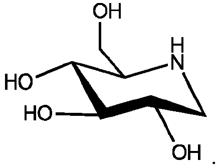

When a salt of duvoglustat is used, the dosage of the salt will be adjusted so that the dose of duvoglustat received by the patient is equivalent to the amount which would have been received had the duvoglustat free base been used.

As used herein, the term "pharmacological chaperone" or sometimes simply the term "chaperone" is intended to refer to a molecule that specifically binds to a lysosomal protein and has one or more of the following effects:
enhances the formation of a stable molecular conformation of the protein;
enhances proper trafficking of the protein from the endoplasmic reticulum to another cellular location, preferably a native cellular location, so as to prevent endoplasmic reticulum-associated degradation of the protein;
prevents aggregation of conformationally unstable or misfolded proteins;
restores and/or enhances at least partial wild-type function, stability, and/or activity of the protein; and/or
improves the phenotype or function of the cell harboring the protein.

Thus, a pharmacological chaperone for acid α-glucosidase is a molecule that binds to acid α-glucosidase, resulting in proper folding, trafficking, non-aggregation, and activity of acid α-glucosidase. As used herein, this term includes but is not limited to active site-specific chaperones (ASSCs) which bind in the active site of the enzyme, inhibitors or The pharmacological chaperone described herein, but not claimed, antagonists, and agonists. The pharmacological chaperone can be an inhibitor or antagonist of acid α-glucosidase. As used herein, the term "antagonist" is intended to refer to any molecule that binds to acid α-glucosidase and either partially or completely blocks, inhibits, reduces, or neutralizes an activity of acid α-glucosidase. The pharmacological chaperone described herein, but not claimed, can be miglustat. Another non-limiting example of a pharmacological chaperone for acid α-glucosidase is duvoglustat.

As used herein, the term "active site" is intended to refer to a region of a protein that is associated with and necessary for a specific biological activity of the protein. In at least one embodiment, the active site can be a site that binds a substrate or other binding partner and contributes the amino acid residues that directly participate in the making and breaking of chemical bonds.

As used herein, the "therapeutically effective dose" and "effective amount" are intended to refer to an amount of rhGAA and/or of chaperone and/or of a combination thereof, which is sufficient to result in a therapeutic response in a subject. A therapeutic response may be any response that a user (for example, a clinician) will recognize as an effective response to the therapy, including any surrogate clinical markers or symptoms described herein and known in the art. Thus, in at least one embodiment, a therapeutic response can be an amelioration or inhibition of one or more symptoms or markers of Pompe disease such as those known in the art. Symptoms or markers of Pompe disease include but are not limited to decreased acid α-glucosidase tissue activity; cardiomyopathy; cardiomegaly; progressive muscle weakness, especially in the trunk or lower limbs; profound hypotonia; macroglossia (and in some cases, protrusion of the tongue); difficulty swallowing, sucking, and/or feeding; respiratory insufficiency; hepatomegaly (moderate); laxity of facial muscles; areflexia; exercise intolerance; exertional dyspnea; orthopnea; sleep apnea; morning headaches; somnolence; lordosis and/or scoliosis; decreased deep tendon reflexes; lower back pain; and failure to meet developmental motor milestones. It should be noted that a concentration of chaperone (e.g. miglustat) that has an inhibitory effect on acid α-glucosidase may constitute an "effective amount" for purposes useful with this invention because of dilution (and consequent shift in binding due to the change in equilibrium), bioavailability and metabolism of the chaperone upon administration in vivo.

As used herein, the term "enzyme replacement therapy" or "ERT" is intended to refer to the introduction of a non-native, purified enzyme into an individual having a deficiency in such enzyme. The administered protein can be obtained from natural sources or by recombinant expression. The term also refers to the introduction of a purified enzyme in an individual otherwise requiring or benefiting from administration of a purified enzyme. In at least one embodiment, such an individual suffers from enzyme insufficiency. The introduced enzyme may be a purified, recombinant enzyme produced in vitro, or a protein purified from isolated tissue or fluid, such as, for example, placenta or animal milk, or from plants.

As used herein, the term "combination therapy" is intended to refer to any therapy wherein two or more individual therapies are administered concurrently or consecutively. In at least one embodiment, the results of the combination therapy are enhanced as compared to the effect of each therapy when it is performed individually. Enhancement may include any improvement of the effect of the various therapies that may result in an advantageous result as compared to the results achieved by the therapies when performed alone. Enhanced effect or results can include a synergistic enhancement, wherein the enhanced effect is more than the additive effects of each therapy when performed by itself; an additive enhancement, wherein the enhanced effect is substantially equal to the additive effect of each therapy when performed by itself; or less than a synergistic effect, wherein the enhanced effect is lower than the additive effect of each therapy when performed by itself, but still better than the effect of each therapy when performed by itself. Enhanced effect may be measured by any means known in the art by which treatment efficacy or outcome can be measured.

As used herein, the term "pharmaceutically acceptable" is intended to refer to molecular entities and compositions that are physiologically tolerable and do not typically produce untoward reactions when administered to a human. Preferably, as used herein, the term "pharmaceutically acceptable" means approved by a regulatory agency of the federal or a state government or listed in the U.S. Pharmacopeia or other generally recognized pharmacopeia for use in animals, and more particularly in humans.

As used herein, the term "carrier" is intended to refer to a diluent, adjuvant, excipient, or vehicle with which a compound is administered. Suitable pharmaceutical carriers are known in the art and, in at least one embodiment, are described in "Remington's Pharmaceutical Sciences" by E. W. Martin, 18th Edition, or other editions.

As used herein, the terms "subject" or "patient" are intended to refer to a human or non-human animal. In at least one embodiment, the subject is a mammal. In at least one embodiment, the subject is a human.

As used herein, the term "anti-drug antibody" is intended to refer to an antibody specifically binding to a drug administered to a subject and generated by the subject as at least part of a humoral immune response to administration of the drug to the subject. In at least one embodiment the drug is a therapeutic protein drug product. The presence of the anti-drug antibody in the subject can cause immune responses ranging from mild to severe, including but not limited to life-threatening immune responses which include but are not limited to anaphylaxis, cytokine release syndrome and cross-reactive neutralization of endogenous proteins mediating critical functions. In addition or alternatively, the presence of the anti-drug antibody in the subject can decrease the efficacy of the drug.

As used herein, the term "neutralizing antibody" is intended to refer to an anti-drug antibody acting to neutralize the function of the drug. In at least one embodiment, the therapeutic protein drug product is a counterpart of an endogenous protein for which expression is reduced or absent in the subject. In at least one embodiment, the neutralizing antibody can act to neutralize the function of the endogenous protein.

As used herein, the terms "about" and "approximately" are intended to refer to an acceptable degree of error for the quantity measured given the nature or precision of the measurements. For example, the degree of error can be indicated by the number of significant figures provided for the measurement, as is understood in the art, and includes but is not limited to a variation of ±1 in the most precise significant figure reported for the measurement. Typical exemplary degrees of error are within 20 percent (%), preferably within 10%, and more preferably within 5% of a given value or range of values. Alternatively, and particularly in biological systems, the terms "about" and "approximately" can mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

The term "concurrently" as used herein is intended to mean at the same time as or within a reasonably short period of time before or after, as will be understood by those skilled in the art. For example, if two treatments are administered concurrently with each other, one treatment can be administered before or after the other treatment, to allow for time needed to prepare for the later of the two treatments. Therefore "concurrent administration" of two treatments includes but is not limited to one treatment following the other by 20 minutes or less, about 20 minutes, about 15 minutes, about 10 minutes, about 5 minutes, about 2 minutes, about 1 minute or less than 1 minute.

The term "pharmaceutically acceptable salt" as used herein is intended to mean a salt which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically-acceptable acid addition salts and pharmaceutically-acceptable base addition salts. Lists of suitable salts are found in, for example, S. M. Birge et al., J. Pharm. Sci., 1977, 66, pp. 1-19

The term "pharmaceutically-acceptable acid addition salt" as used herein is intended to mean those salts which retain the biological effectiveness and properties of the free bases and which are not biologically or otherwise undesirable, formed with inorganic acids including but not limited to hydrochloric acid, hydrobromic acid, sulfuric acid, sulfamic acid, nitric acid, phosphoric acid and the like, and organic acids including but not limited to acetic acid, trifluoroacetic acid, adipic acid, ascorbic acid, aspartic acid, benzenesulfonic acid, benzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, hexanoic acid, formic acid, fumaric acid, 2-hydroxyethanesulfonic acid (isethionic acid), lactic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, pivalic acid, propionic acid, pyruvic acid, salicylic acid, stearic acid, succinic acid, sulfanilic acid, tartaric acid, p-toluenesulfonic acid, undecanoic acid and the like.

The term "pharmaceutically-acceptable base addition salt" as used herein is intended to mean those salts which retain the biological effectiveness and properties of the free acids and which are not biologically or otherwise undesirable, formed with inorganic bases including but not limited to ammonia or the hydroxide, carbonate, or bicarbonate of ammonium or a metal cation such as sodium, potassium, lithium, calcium, magnesium, iron, zinc, copper, manganese, aluminum and the like. Salts derived from pharmaceutically-acceptable organic nontoxic bases include but are not limited to salts of primary, secondary, and tertiary amines, quaternary amine compounds, substituted amines including naturally occurring substituted amines, cyclic amines and basic ion-exchange resins, such as methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperazine, piperidine, N-ethylpiperidine, tetramethylammonium compounds, tetraethylammonium compounds, pyridine, N,N-dimethylaniline, N-methylpiperidine, N-methylmorpholine, dicyclohexylamine, dibenzylamine, N,N-dibenzylphenethylamine, 1-ephenamine, N,N'-dibenzylethylenediamine, polyamine resins and the like.

### ATB200 rhGAA

In at least one embodiment, the rhGAA) is expressed in Chinese hamster ovary (CHO) cells and comprises an increased content of N-glycan units bearing one or more mannose-6-phosphate residues when compared to a content of N-glycan units bearing one or more mannose-6-phosphate residues of a conventional recombinant human lysosomal protein such as alglucosidase alfa. In at least one embodiment, the acid α-glucosidase is a recombinant human acid α-glucosidase referred to herein as ATB200, as described in co-pending international patent application PCT/US2015/053252. ATB200 has been shown to bind cation-independent mannose-6-phosphate receptors (CIMPR) with high affinity *(K_{D}* ~ 2-4 nM) and to be efficiently internalized by Pompe fibroblasts and skeletal muscle myoblasts *(Kᵤₚₜₐₖₑ ~* 7-14 nM). ATB200 was characterized *in vivo* and shown to have a shorter apparent plasma half-life (t_{1/2} ~ 45 min) than alglucosidase alfa (t_{1/2} ~ 60 min).

Recombinant human acid α-glucosidase is an enzyme having an amino acid sequence as set forth in SEQ ID NO: 1 or SEQ ID NO: 2. The rhGAA produced in the present invention is defined in the claims.

SEQ ID NO: 1 Met Gly Val Arg His Pro Pro Cys Ser His Arg Leu Leu Ala Val Cys
Ala Leu Val Ser Leu Ala Thr Ala Ala Leu Leu Gly His Ile Leu Leu
His Asp Phe Leu Leu Val Pro Arg Glu Leu Ser Gly Ser Ser Pro Val
Leu Glu Glu Thr His Pro Ala His Gln Gln Gly Ala Ser Arg Pro Gly
Pro Arg Asp Ala Gln Ala His Pro Gly Arg Pro Arg Ala Val Pro Thr
Gln Cys Asp Val Pro Pro Asn Ser Arg Phe Asp Cys Ala Pro Asp Lys

Ala Ile Thr Gln Glu Gln Cys Glu Ala Arg Gly Cys Cys Tyr Ile Pro
Ala Lys Gln Gly Leu Gln Gly Ala Gln Met Gly Gln Pro Trp Cys Phe
Phe Pro Pro Ser Tyr Pro Ser Tyr Lys Leu Glu Asn Leu Ser Ser Ser
Glu Met Gly Tyr Thr Ala Thr Leu Thr Arg Thr Thr Pro Thr Phe Phe
Pro Lys Asp Ile Leu Thr Leu Arg Leu Asp Val Met Met Glu Thr Glu
Asn Arg Leu His Phe Thr Ile Lys Asp Pro Ala Asn Arg Arg Tyr Glu
Val Pro Leu Glu Thr Pro Arg Val His Ser Arg Ala Pro Ser Pro Leu
Tyr Ser Val Glu Phe Ser Glu Glu Pro Phe Gly Val Ile Val His Arg
Gln Leu Asp Gly Arg Val Leu Leu Asn Thr Thr Val Ala Pro Leu Phe
Phe Ala Asp Gln Phe Leu Gln Leu Ser Thr Ser Leu Pro Ser Gln Tyr
Ile Thr Gly Leu Ala Glu His Leu Ser Pro Leu Met Leu Ser Thr Ser
Trp Thr Arg Ile Thr Leu Trp Asn Arg Asp Leu Ala Pro Thr Pro Gly
Ala Asn Leu Tyr Gly Ser His Pro Phe Tyr Leu Ala Leu Glu Asp Gly
Gly Ser Ala His Gly Val Phe Leu Leu Asn Ser Asn Ala Met Asp Val
Val Leu Gln Pro Ser Pro Ala Leu Ser Trp Arg Ser Thr Gly Gly Ile
Leu Asp Val Tyr Ile Phe Leu Gly Pro Glu Pro Lys Ser Val Val Gln
Gln Tyr Leu Asp Val Val Gly Tyr Pro Phe Met Pro Pro Tyr Trp Gly
Leu Gly Phe His Leu Cys Arg Trp Gly Tyr Ser Ser Thr Ala Ile Thr
Arg Gln Val Val Glu Asn Met Thr Arg Ala His Phe Pro Leu Asp Val
Gln Trp Asn Asp Leu Asp Tyr Met Asp Ser Arg Arg Asp Phe Thr
Phe Asn Lys Asp Gly Phe Arg Asp Phe Pro Ala Met Val Gln Glu
Leu His Gln Gly Gly Arg Arg Tyr Met Met Ile Val Asp Pro Ala Ile
Ser Ser Ser Gly Pro Ala Gly Ser Tyr Arg Pro Tyr Asp Glu Gly Leu
Arg Arg Gly Val Phe Ile Thr Asn Glu Thr Gly Gln Pro Leu Ile Gly
Lys Val Trp Pro Gly Ser Thr Ala Phe Pro Asp Phe Thr Asn Pro Thr
Ala Leu Ala Trp Trp Glu Asp Met Val Ala Glu Phe His Asp Gln Val
Pro Phe Asp Gly Met Trp Ile Asp Met Asn Glu Pro Ser Asn Phe Ile
Arg Gly Ser Glu Asp Gly Cys Pro Asn Asn Glu Leu Glu Asn Pro Pro
Tyr Val Pro Gly Val Val Gly Gly Thr Leu Gln Ala Ala Thr Ile Cys
Ala Ser Ser His Gln Phe Leu Ser Thr His Tyr Asn Leu His Asn Leu
Tyr Gly Leu Thr Glu Ala Ile Ala Ser His Arg Ala Leu Val Lys Ala
Arg Gly Thr Arg Pro Phe Val Ile Ser Arg Ser Thr Phe Ala Gly His

Gly Arg Tyr Ala Gly His Trp Thr Gly Asp Val Trp Ser Ser Trp Glu
Gln Leu Ala Ser Ser Val Pro Glu Ile Leu Gln Phe Asn Leu Leu Gly
Val Pro Leu Val Gly Ala Asp Val Cys Gly Phe Leu Gly Asn Thr Ser
Glu Glu Leu Cys Val Arg Trp Thr Gln Leu Gly Ala Phe Tyr Pro Phe
Met Arg Asn His Asn Ser Leu Leu Ser Leu Pro Gln Glu Pro Tyr Ser
Phe Ser Glu Pro Ala Gln Gln Ala Met Arg Lys Ala Leu Thr Leu Arg
Tyr Ala Leu Leu Pro His Leu Tyr Thr Leu Phe His Gln Ala His Val
Ala Gly Glu Thr Val Ala Arg Pro Leu Phe Leu Glu Phe Pro Lys Asp
Ser Ser Thr Trp Thr Val Asp His Gln Leu Leu Trp Gly Glu Ala Leu
Leu Ile Thr Pro Val Leu Gln Ala Gly Lys Ala Glu Val Thr Gly Tyr
Phe Pro Leu Gly Thr Trp Tyr Asp Leu Gln Thr Val Pro Ile Glu Ala
Leu Gly Ser Leu Pro Pro Pro Pro Ala Ala Pro Arg Glu Pro Ala Ile
His Ser Glu Gly Gln Trp Val Thr Leu Pro Ala Pro Leu Asp Thr Ile
Asn Val His Leu Arg Ala Gly Tyr Ile Ile Pro Leu Gln Gly Pro Gly
Leu Thr Thr Thr Glu Ser Arg Gln Gln Pro Met Ala Leu Ala Val Ala
Leu Thr Lys Gly Gly Glu Ala Arg Gly Glu Leu Phe Trp Asp Asp Gly
Glu Ser Leu Glu Val Leu Glu Arg Gly Ala Tyr Thr Gln Val Ile Phe
Leu Ala Arg Asn Asn Thr Ile Val Asn Glu Leu Val Arg Val Thr Ser
Glu Gly Ala Gly Leu Gln Leu Gln Lys Val Thr Val Leu Gly Val Ala
Thr Ala Pro Gln Gln Val Leu Ser Asn Gly Val Pro Val Ser Asn Phe
Thr Tyr Ser Pro Asp Thr Lys Val Leu Asp Ile Cys Val Ser Leu Leu
Met Gly Glu Gln Phe Leu Val Ser Trp Cys

SEQ ID NO: 2 Gln Gln Gly Ala Ser Arg Pro Gly Pro Arg Asp Ala Gln Ala His Pro
Gly Arg Pro Arg Ala Val Pro Thr Gln Cys Asp Val Pro Pro Asn Ser
Arg Phe Asp Cys Ala Pro Asp Lys Ala Ile Thr Gln Glu Gln Cys Glu
Ala Arg Gly Cys Cys Tyr Ile Pro Ala Lys Gln Gly Leu Gln Gly Ala
Gln Met Gly Gln Pro Trp Cys Phe Phe Pro Pro Ser Tyr Pro Ser Tyr
Lys Leu Glu Asn Leu Ser Ser Ser Glu Met Gly Tyr Thr Ala Thr Leu
Thr Arg Thr Thr Pro Thr Phe Phe Pro Lys Asp Ile Leu Thr Leu Arg
Leu Asp Val Met Met Glu Thr Glu Asn Arg Leu His Phe Thr Ile Lys
Asp Pro Ala Asn Arg Arg Tyr Glu Val Pro Leu Glu Thr Pro Arg Val
His Ser Arg Ala Pro Ser Pro Leu Tyr Ser Val Glu Phe Ser Glu Glu

Pro Phe Gly Val Ile Val His Arg Gln Leu Asp Gly Arg Val Leu Leu
Asn Thr Thr Val Ala Pro Leu Phe Phe Ala Asp Gln Phe Leu Gln Leu
Ser Thr Ser Leu Pro Ser Gln Tyr Ile Thr Gly Leu Ala Glu His Leu
Ser Pro Leu Met Leu Ser Thr Ser Trp Thr Arg Ile Thr Leu Trp Asn
Arg Asp Leu Ala Pro Thr Pro Gly Ala Asn Leu Tyr Gly Ser His Pro
Phe Tyr Leu Ala Leu Glu Asp Gly Gly Ser Ala His Gly Val Phe Leu
Leu Asn Ser Asn Ala Met Asp Val Val Leu Gln Pro Ser Pro Ala Leu
Ser Trp Arg Ser Thr Gly Gly Ile Leu Asp Val Tyr Ile Phe Leu Gly
Pro Glu Pro Lys Ser Val Val Gln Gln Tyr Leu Asp Val Val Gly Tyr
Pro Phe Met Pro Pro Tyr Trp Gly Leu Gly Phe His Leu Cys Arg Trp
Gly Tyr Ser Ser Thr Ala Ile Thr Arg Gln Val Val Glu Asn Met Thr
Arg Ala His Phe Pro Leu Asp Val Gln Trp Asn Asp Leu Asp Tyr
Met Asp Ser Arg Arg Asp Phe Thr Phe Asn Lys Asp Gly Phe Arg
Asp Phe Pro Ala Met Val Gln Glu Leu His Gln Gly Gly Arg Arg Tyr
Met Met Ile Val Asp Pro Ala Ile Ser Ser Ser Gly Pro Ala Gly Ser Tyr
Arg Pro Tyr Asp Glu Gly Leu Arg Arg Gly Val Phe Ile Thr Asn Glu
Thr Gly Gln Pro Leu Ile Gly Lys Val Trp Pro Gly Ser Thr Ala Phe
Pro Asp Phe Thr Asn Pro Thr Ala Leu Ala Trp Trp Glu Asp Met Val
Ala Glu Phe His Asp Gln Val Pro Phe Asp Gly Met Trp Ile Asp Met
Asn Glu Pro Ser Asn Phe Ile Arg Gly Ser Glu Asp Gly Cys Pro Asn
Asn Glu Leu Glu Asn Pro Pro Tyr Val Pro Gly Val Val Gly Gly Thr
Leu Gln Ala Ala Thr Ile Cys Ala Ser Ser His Gln Phe Leu Ser Thr
His Tyr Asn Leu His Asn Leu Tyr Gly Leu Thr Glu Ala Ile Ala Ser
His Arg Ala Leu Val Lys Ala Arg Gly Thr Arg Pro Phe Val Ile Ser
Arg Ser Thr Phe Ala Gly His Gly Arg Tyr Ala Gly His Trp Thr Gly
Asp Val Trp Ser Ser Trp Glu Gln Leu Ala Ser Ser Val Pro Glu Ile
Leu Gln Phe Asn Leu Leu Gly Val Pro Leu Val Gly Ala Asp Val Cys
Gly Phe Leu Gly Asn Thr Ser Glu Glu Leu Cys Val Arg Trp Thr Gln
Leu Gly Ala Phe Tyr Pro Phe Met Arg Asn His Asn Ser Leu Leu Ser
Leu Pro Gln Glu Pro Tyr Ser Phe Ser Glu Pro Ala Gln Gln Ala Met
Arg Lys Ala Leu Thr Leu Arg Tyr Ala Leu Leu Pro His Leu Tyr Thr
Leu Phe His Gln Ala His Val Ala Gly Glu Thr Val Ala Arg Pro Leu

Phe Leu Glu Phe Pro Lys Asp Ser Ser Thr Trp Thr Val Asp His Gln
Leu Leu Trp Gly Glu Ala Leu Leu Ile Thr Pro Val Leu Gln Ala Gly
Lys Ala Glu Val Thr Gly Tyr Phe Pro Leu Gly Thr Trp Tyr Asp Leu
Gln Thr Val Pro Ile Glu Ala Leu Gly Ser Leu Pro Pro Pro Pro Ala
Ala Pro Arg Glu Pro Ala Ile His Ser Glu Gly Gln Trp Val Thr Leu
Pro Ala Pro Leu Asp Thr Ile Asn Val His Leu Arg Ala Gly Tyr Ile Ile
Pro Leu Gln Gly Pro Gly Leu Thr Thr Thr Glu Ser Arg Gln Gln Pro
Met Ala Leu Ala Val Ala Leu Thr Lys Gly Gly Glu Ala Arg Gly Glu
Leu Phe Trp Asp Asp Gly Glu Ser Leu Glu Val Leu Glu Arg Gly Ala
Tyr Thr Gln Val Ile Phe Leu Ala Arg Asn Asn Thr Ile Val Asn Glu
Leu Val Arg Val Thr Ser Glu Gly Ala Gly Leu Gln Leu Gln Lys Val
Thr Val Leu Gly Val Ala Thr Ala Pro Gln Gln Val Leu Ser Asn Gly
Val Pro Val Ser Asn Phe Thr Tyr Ser Pro Asp Thr Lys Val Leu Asp
Ile Cys Val Ser Leu Leu Met Gly Glu Gln Phe Leu Val Ser Trp Cys

In at least one embodiment, the recombinant human acid α-glucosidase has a wild-type GAA amino acid sequence as set forth in SEQ ID NO: 1, as described in US Patent No. 8,592,362 and has GenBank accession number AHE24104.1 (GI:568760974). In at least one embodiment, the recombinant human acid α-glucosidase is glucosidase alfa, the human acid α-glucosidase enzyme encoded by the most predominant of nine observed haplotypes of the *GAA* gene.

In at least one embodiment, the recombinant human acid α-glucosidase is initially expressed as having the full-length 952 amino acid sequence of wild-type GAA as set forth in SEQ ID NO: 1, and the recombinant human acid α-glucosidase undergoes intracellular processing that removes a portion of the amino acids, e.g. the first 56 amino acids. Accordingly, the recombinant human acid α-glucosidase that is secreted by the host cell can have a shorter amino acid sequence than the recombinant human acid α-glucosidase that is initially expressed within the cell. In at least one embodiment, the shorter protein can have the amino acid sequence set forth in SEQ ID NO: 2, which only differs from SEQ ID NO: 1 in that the first 56 amino acids comprising the signal peptide and precursor peptide have been removed, thus resulting in a protein having 896 amino acids. Other variations in the number of amino acids is also possible, such as having 1. 2, 3, 4, 5, 6, 7, 8, 9, 10, 11, 12, 13, 14, 15 or more deletions, substitutions and/or insertions relative to the amino acid sequence described by SEQ ID NO: 1 or SEQ ID NO: 2. In some embodiments, the rhGAA product includes a mixture of recombinant human acid α-glucosidase molecules having different amino acid lengths.

In at least one embodiment, the recombinant human acid α-glucosidase undergoes post-translational and/or chemical modifications at one or more amino acid residues in the protein. For example, methionine and tryptophan residues can undergo oxidation. As another example, the N-terminal glutamine can form pyro-glutamate. As another example, asparagine residues can undergo deamidation to aspartic acid. As yet another example, aspartic acid residues can undergo isomerization to iso-aspartic acid. As yet another example, unpaired cysteine residues in the protein can form disulfide bonds with free glutathione and/or cysteine. Accordingly, in some embodiments the enzyme is initially expressed as having an amino acid sequence as set forth in SEQ ID NO: 1 or SEQ ID NO: 2, and the enzyme undergoes one or more of these post-translational and/or chemical modifications. Such modifications are also within the scope of the present disclosure.

Polynucleotide sequences encoding GAA and such variant human GAAs are also contemplated and may be used to recombinantly express rhGAAs according to the invention.

Preferably, no more than 70, 65, 60, 55, 45, 40, 35, 30, 25, 20, 15, 10, or 5% of the total rhGAA) molecules lack an N-glycan unit bearing one or more mannose-6-phosphate residues or lacks a capacity to bind to the cation independent mannose-6-phosphate receptor (CIMPR). Alternatively, 30, 35, 40, 45, 50, 55, 60, 65, 70, 75, 80, 85, 90, 95, 99%, <100% or more of the rhGAA molecules comprise at least one N-glycan unit bearing one or more mannose-6-phosphate residues or has the capacity to bind to CIMPR.

The rhGAA) molecules may have 1, 2, 3 or 4 mannose-6-phosphate (M6P) groups on their glycans. For example, only one N-glycan on a rhGAA molecule may bear M6P (mono-phosphorylated), a single N-glycan may bear two M6P groups (bis-phosphorylated), or two different N-glycans on the same rhGAA molecule may each bear single M6P groups. rhGAA molecules may also have N-glycans bearing no M6P groups. In another embodiment, on average the N-glycans contain greater than 3 mol/mol of M6P and greater than 4 mol/mol sialic acid, such that the rhGAA comprises on average at least 3 moles of mannose-6-phosphate residues per mole of rhGAA and at least 4 moles of sialic acid per mole of rhGAA On average at least about 3, 4, 5, 6, 7, 8, 9, or 10% of the total glycans on the rhGAA may be in the form of a mono-M6P glycan, for example, about 6.25% of the total glycans may carry a single M6P group and on average, at least about 0.5, 1, 1.5, 2.0, 2.5, 3.0% of the total glycans on rhGAA are in the form of a bis-M6P glycan and on average less than 25% of total rhGAA contains no phosphorylated glycan binding to CIMPR.

The rhGAA may have an average content of N-glycans carrying M6P ranging from 0.5 to 7.0 mol/mol lysosomal protein or any intermediate value of subrange including 0.5, 1.0, 1.5, 2.0, 2.5, 3.0, 3.5, 4.0, 4.5, 5.0, 5.5, 6.0, 6.5, or 7.0 mol/mol lysosomal protein. The rhGAA can be fractionated to provide rhGAA protein preparations with different average numbers of M6P-bearing or bis-M6P-bearing glycans thus permitting further customization of rhGAA protein targeting to the lysosomes in target tissues by selecting a particular fraction or by selectively combining different fractions.

In some embodiments, the rhGAA will bear, on average, 2.0 to 8.0 moles of M6P per mole of rhGAA. This range includes all intermediate values and subranges including 2.0, 2.5, 3.0, 3.5, 4.0, 4.5, 5.0, 5.5, 6.0, 6.5, 7.0, 7.5 and 8.0 mol M6P/mol rhGAA.

Up to 60% of the N-glycans on the rhGAA rhGAA may be fully sialylated, for example, up to 10%, 20%, 30%, 40%, 50% or 60% of the N-glycans may be fully sialylated. In some embodiments from 4 to 20% of the total N-glycans are fully sialylated. In other embodiments no more than 5%, 10%, 20% or 30% of N-glycans on the rhGAA carry sialic acid and a terminal galactose residue (Gal). This range includes all intermediate values and subranges, for example, 7 to 30% of the total N-glycans on the rhGAA can carry sialic acid and terminal galactose. In yet other embodiments, no more than 5, 10, 15, 16, 17, 18, 19 or 20% of the N-glycans on rhGAA have a terminal galactose only and do not contain sialic acid. This range includes all intermediate values and subranges, for example, from 8 to 19% of the total N-glycans on the rhGAA in the composition may have terminal galactose only and do not contain sialic acid.

In other embodiments of the invention, 40, 45, 50, 55 to 60% of the total N-glycans on the e.g. rhGAA are complex type N-glycans; or no more than 1, 2, 3, 4, 5, 6, 7% of total N-glycans on the rhGAA are hybrid-type N-glycans; no more than 5, 10, or 15% of the high mannose-type N-glycans on the rhGAA are non-phosphorylated; at least 5% or 10% of the high mannose-type N-glycans on the rhGAA are mono-M6P phosphorylated; and/or at least 1 or 2% of the high mannose-type N-glycans on the rhGAA are bis-M6P phosphorylated. These values include all intermediate values and subranges. A rhGAA may meet one or more of the content ranges described above.

In some embodiments, the rhGAA will bear, on average, 2.0 to 8.0 moles of sialic acid residues per mole of rhGAA. This range includes all intermediate values and subranges including 2.0, 2.5, 3.0, 3.5, 4.0, 4.5, 5.0, 5.5, 6.0, 6.5, 7.0, 7.5 and 8.0 mol residues/mol rhGAA. Without being bound by theory, it is believed that the presence of N-glycan units bearing sialic acid residues may prevent non-productive clearance of the rhGAA by asialoglycoprotein receptors.

In one or more embodiments, the rhGAA rhGAA has M6P and/or sialic acid units at certain N-glycosylation sites of the rhGAA. For example, as stated above, there are seven potential N-linked glycosylation sites on rhGAA. These potential glycosylation sites are at the following positions of SEQ ID NO: 2: N84, N177, N334, N414, N596, N826 and N869. Similarly, for the full-length amino acid sequence of SEQ ID NO: 1, these potential glycosylation sites are at the following positions: N140, N233, N390, N470, N652, N882 and N925. Other variants of rhGAA can have similar glycosylation sites, depending on the location of asparagine residues. Generally, sequences of ASN-X-SER or ASN-X-THR in the protein amino acid sequence indicate potential glycosylation sites, with the exception that X cannot be HIS or PRO.

In various embodiments, the rhGAA has a certain N-glycosylation profile. In one or more embodiments, at least 20% of the rhGAA is phosphorylated at the first N-glycosylation site (e.g. N84 for SEQ ID NO: 2 and N140 for SEQ ID NO: 1). For example, at least 20%, 25%, 30%, 35%, 40%, 45%, 50%, 55%, 60%, 65%, 70%, 75%, 80%, 85%, 90% or 95% of the rhGAA can be phosphorylated at the first N-glycosylation site. This phosphorylation can be the result of mono-M6P and/or bis-M6P units. In some embodiments, at least 10%, 15%, 20%, 25%, 30%, 35%, 40%, 45%, 50%, 55%, 60%, 65%, 70%, 75%, 80%, 85%, 90% or 95% of the rhGAA bears a mono-M6P unit at the first N-glycosylation site. In some embodiments, at least 10%, 15%, 20%, 25%, 30%, 35%, 40%, 45%, 50%, 55%, 60%, 65%, 70%, 75%, 80%, 85%, 90% or 95% of the rhGAA bears a bis-M6P unit at the first N-glycosylation site.

In one or more embodiments, at least 20% of the rhGAA is phosphorylated at the second N-glycosylation site (e.g. N177 for SEQ ID NO: 2 and N223 for SEQ ID NO: 1). For example, at least 20%, 25%, 30%, 35%, 40%, 45%, 50%, 55%, 60%, 65%, 70%, 75%, 80%, 85%, 90% or 95% of the rhGAA can be phosphorylated at the second N-glycosylation site. This phosphorylation can be the result of mono-M6P and/or bis-M6P units. In some embodiments, at least 10%, 15%, 20%, 25%, 30%, 35%, 40%, 45%, 50%, 55%, 60%, 65%, 70%, 75%, 80%, 85%, 90% or 95% of the rhGAA bears a mono-M6P unit at the second N-glycosylation site. In some embodiments, at least 10%, 15%, 20%, 25%, 30%, 35%, 40%, 45%, 50%, 55%, 60%, 65%, 70%, 75%, 80%, 85%, 90% or 95% of the rhGAA bears a bis-M6P unit at the second N-glycosylation site. In one or more embodiments, at least 5% of the rhGAA is phosphorylated at the third N-glycosylation site (e.g. N334 for SEQ ID NO: 2 and N390 for SEQ ID NO: 1). In other embodiments, less than 5%, 10%, 15%, 20% or 25% of the rhGAA is phosphorylated at the third N-glycosylation site. For example, the third N-glycosylation site can have a mixture of non-phosphorylated high mannose glycans, di-, tri-, and tetra-antennary complex glycans, and hybrid glycans as the major species. In some embodiments, at least 3%, 5%, 8%, 10%, 15%, 20%, 25%, 30%, 35%, 40%, 45% or 50% of the rhGAA is sialylated at the third N-glycosylation site.

In one or more embodiments, at least 20% of the rhGAA is phosphorylated at the fourth N-glycosylation site (e.g. N414 for SEQ ID NO: 2 and N470 for SEQ ID NO: 1). For example, at least 20%, 25%, 30%, 35%, 40%, 45%, 50%, 55%, 60%, 65%, 70%, 75%, 80%, 85%, 90% or 95% of the rhGAA can be phosphorylated at the fourth N-glycosylation site. This phosphorylation can be the result of mono-M6P and/or bis-M6P units. In some embodiments, at least 10%, 15%, 20%, 25%, 30%, 35%, 40%, 45%, 50%, 55%, 60%, 65%, 70%, 75%, 80%, 85%, 90% or 95% of the rhGAA bears a mono-M6P unit at the fourth N-glycosylation site. In some embodiments, at least 10%, 15%, 20%, 25%, 30%, 35%, 40%, 45%, 50%, 55%, 60%, 65%, 70%, 75%, 80%, 85%, 90% or 95% of the rhGAA bears a bis-M6P unit at the fourth N-glycosylation site. In some embodiments, at least 3%, 5%, 8%, 10%, 15%, 20% or 25% of the rhGAA is sialylated at the fourth N-glycosylation site.

In one or more embodiments, at least 5% of the rhGAA is phosphorylated at the fifth N-glycosylation site (e.g. N596 for SEQ ID NO: 2 and N692 for SEQ ID NO: 1). In other embodiments, less than 5%, 10%, 15%, 20% or 25% of the rhGAA is phosphorylated at the fifth N-glycosylation site. For example, the fifth N-glycosylation site can have fucosylated di-antennary complex glycans as the major species. In some embodiments, at least 3%, 5%, 8%, 10%, 15%, 20%, 25%, 30%, 35%, 40%, 45%, 50%, 55%, 60%, 65%, 70%, 75%, 80%, 85%, 90% or 95% of the rhGAA is sialylated at the fifth N-glycosylation site.

In one or more embodiments, at least 5% of the rhGAA is phosphorylated at the sixth N-glycosylation site (e.g. N826 for SEQ ID NO: 2 and N882 for SEQ ID NO: 1). In other embodiments, less than 5%, 10%, 15%, 20% or 25% of the rhGAA is phosphorylated at the sixth N-glycosylation site. For example, the sixth N-glycosylation site can have a mixture of di-, tri-, and tetra-antennary complex glycans as the major species. In some embodiments, at least 3%, 5%, 8%, 10%, 15%, 20%, 25%, 30%, 35%, 40%, 45%, 50%, 55%, 60%, 65%, 70%, 75%, 80%, 85%, 90% or 95% of the rhGAA is sialylated at the sixth N-glycosylation site.

In one or more embodiments, at least 5% of the rhGAA is phosphorylated at the seventh N-glycosylation site (e.g. N869 for SEQ ID NO: 2 and N925 for SEQ ID NO: 1). In other embodiments, less than 5%, 10%, 15%, 20% or 25% of the rhGAA is phosphorylated at the seventh N-glycosylation site. In some embodiments, less than 40%, 45%, 50%, 55%, 60% or 65% % of the rhGAA has any glycan at the seventh N-glycosylation site. In some embodiments, at least 30%, 35% or 40% of the rhGAA has a glycan at the seventh N-glycosylation site.

In various embodiments, the rhGAA has an average fucose content of 0-5 mol per mol of rhGAA, GlcNAc content of 10-30 mol per mol of rhGAA, galactose content of 5-20 mol per mol of rhGAA, mannose content of 10-40 mol per mol of rhGAA, M6P content of 2-8 mol per mol of rhGAA and sialic acid content of 2-8 mol per mol of rhGAA. In various embodiments, the rhGAA has an average fucose content of 2-3 mol per mol of rhGAA, GlcNAc content of 20-25 mol per mol of rhGAA, galactose content of 8-12 mol per mol of rhGAA, mannose content of 22-27 mol per mol of rhGAA, M6P content of 3-5 mol per mol of rhGAA and sialic acid content of 4-7 mol of rhGAA.

The rhGAA is preferably produced by Chinese hamster ovary (CHO) cells, such as CHO cell line GA-ATB-200 or ATB-200-001-X5-14, or by a subculture or derivative of such a CHO cell culture. DNA constructs, which express allelic variants of acid α-glucosidase or other variant acid α-glucosidase amino acid sequences such as those that are at least 90%, 95%, 98% or 99% identical to SEQ ID NO: 1 or SEQ ID NO: 2, may be constructed and expressed in CHO cells. These variant acid α-glucosidase amino acid sequences may contain deletions, substitutions and/or insertions relative to SEQ ID NO: 1 or SEQ ID NO: 2, such as having 1, 2, 3, 4, 5, 6, 7, 8, 9, 10, 11, 12, 13, 14, 15 or more deletions, substitutions and/or insertions relative to the amino acid sequence described by SEQ ID NO: 1 or SEQ ID NO: 2. Those of skill in the art can select alternative vectors suitable for transforming CHO cells for production of such DNA constructs.

Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 90%, 95%, 98% or 99% identity to specific polypeptides described herein and preferably exhibiting substantially the same functions, as well as polynucleotide encoding such polypeptides, are contemplated. Unless otherwise indicated a similarity score will be based on use of BLOSUM62. When BLASTP is used, the percent similarity is based on the BLASTP positives score and the percent sequence identity is based on the BLASTP identities score. BLASTP "Identities" shows the number and fraction of total residues in the high scoring sequence pairs which are identical; and BLASTP "Positives" shows the number and fraction of residues for which the alignment scores have positive values and which are similar to each other. Amino acid sequences having these degrees of identity or similarity or any intermediate degree of identity of similarity to the amino acid sequences disclosed herein are contemplated and encompassed by this disclosure. The polynucleotide sequences of similar polypeptides are deduced using the genetic code and may be obtained by conventional means, in particular by reverse translating its amino acid sequence using the genetic code.

The inventors have found that recombinant human acid α-glucosidase having superior ability to target cation-independent mannose-6-phosphate receptors (CIMPR) and cellular lysosomes as well as glycosylation patterns that reduce its non-productive clearance in vivo can be produced using Chinese hamster ovary (CHO) cells. These cells can be induced to express recombinant human acid α-glucosidase with significantly higher levels of N-glycan units bearing one or more mannose-6-phosphate residues than conventional recombinant human acid α-glucosidase products such as alglucosidase alfa. The recombinant human acid α-glucosidase produced by these cells, for example, as exemplified by ATB200, has significantly more muscle cell-targeting mannose-6-phosphate (M6P) and bis-mannose-6-phosphate N-glycan residues than conventional acid α-glucosidase, such as Lumizyme^{®}. Without being bound by theory, it is believed that this extensive glycosylation allows the ATB200 enzyme to be taken up more effectively into target cells, and therefore to be cleared from the circulation more efficiently than other recombinant human acid α-glucosidases, such as for example, alglucosidase alfa, which has a much lower M6P and bis-M6P content. ATB200 has been shown to efficiently bind to CIMPR and be efficiently taken up by skeletal muscle and cardiac muscle and to have a glycosylation pattern that provides a favorable pharmacokinetic profile and reduces non-productive clearance in vivo.

It is also contemplated that the extensive glycosylation of ATB200 can contribute to a reduction of the immunogenicity of ATB200 compared to, for example, alglucosidase alfa. As will be appreciated by those skilled in the art, glycosylation of proteins with conserved mammalian sugars generally enhances product solubility and diminishes product aggregation and immunogenicity. Glycosylation indirectly alters protein immunogenicity by minimizing protein aggregation as well as by shielding immunogenic protein epitopes from the immune system (Guidance for Industry - Immunogenicity Assessment for Therapeutic Protein Products, US Department of Health and Human Services, Food and Drug Administration, Center for Drug Evaluation and Research, Center for Biologics Evaluation and Research, August 2014). Therefore, in at least one embodiment, administration of the recombinant human acid α-glucosidase does not induce anti-drug antibodies. In at least one embodiment, administration of the recombinant human acid α-glucosidase induces a lower incidence of anti-drug antibodies in a subject than the level of anti-drug antibodies induced by administration of alglucosidase alfa.

As described in co-pending international patent application PCT/US2015/053252, cells such as CHO cells can be used to produce the rhGAA described therein, and this rhGAA can be used in the present invention. Examples of such a CHO cell line are GA-ATB-200 or ATB-200-001-X5-14. or a subculture thereof that produces a rhGAA composition as described therein. Such CHO cell lines may contain multiple copies of a gene, such as 5, 10, 15, or 20 or more copies, of a polynucleotide encoding GAA.

The high M6P and bis-M6P rhGAA, such as ATB200 rhGAA, can be produced by transforming CHO cells with a DNA construct that encodes GAA. While CHO cells have been previously used to make rhGAA, it was not appreciated that transformed CHO cells could be cultured and selected in a way that would produce rhGAA having a high content of M6P and bis-M6P glycans which target the CIMPR.

Surprisingly, it was found that it was possible to transform CHO cell lines, select transformants that produce rhGAA containing a high content of glycans bearing M6P or bis-M6P that target the CIMPR, and to stably express this high-M6P rhGAA. Thus, methods for making these CHO cell lines are also described in co-pending international patent application PCT/US2015/053252. This method involves transforming a CHO cell with DNA encoding GAA or a GAA variant, selecting a CHO cell that stably integrates the DNA encoding GAA into its chromosome(s) and that stably expresses GAA, and selecting a CHO cell that expresses GAA having a high content of glycans bearing M6P or bis-M6P, and, optionally, selecting a CHO cell having N-glycans with high sialic acid content and/or having N-glycans with a low non-phosphorylated high-mannose content.

These CHO cell lines may be used to produce rhGAA and rhGAA compositions by culturing the CHO cell line and recovering said composition from the culture of CHO cells.

### Production, Capturing and Purification of Recombinant Human Lysosomal Protein

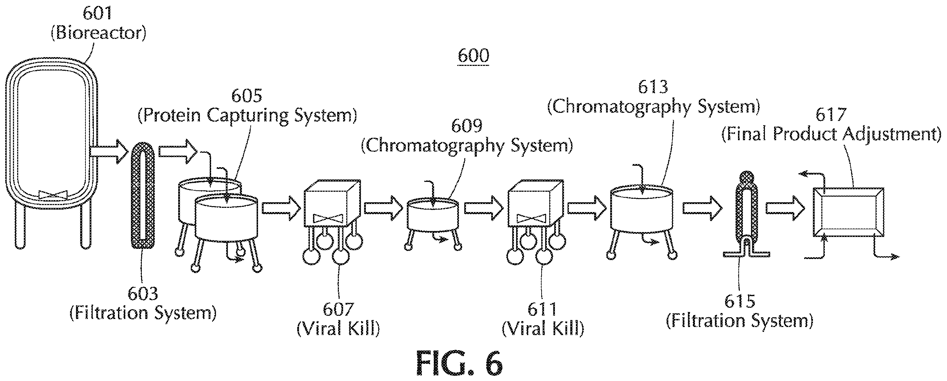

Various embodiments of the present invention pertain to methods for the production and/or capturing and/or purification of rhGAA rhGAA). An exemplary process 600 for producing, capturing and purifying recombinant human lysosomal protein is shown in Figure 6. The invention is defined in the claims.

In Figure 6, the arrows indicate the direction of movement for the various liquid phases containing the recombinant human lysosomal protein. Bioreactor 601 contains a culture of cells, such as CHO cells, that express and secrete recombinant human lysosomal protein (e.g. rhGAA) into the surrounding liquid culture media. The bioreactor 601 can be any appropriate bioreactor for culturing the cells, such as a perfusion, batch or fed-batch bioreactor. In various embodiments, the bioreactor has a volume between about 1 L and about 20,000 L. Exemplary bioreactor volumes include about 1 L, about 10 L, about 20 L, about 30 L, about 40 L, about 50 L, about 60 L, about 70 L, about 80 L, about 90 L, about 100 L, about 150 L, about 200 L, about 250 L, about 300 L, about 350 L. about 400 L, about 500 L, about 600 L, about 700 L, about 800 L, about 900 L, about 1,000 L, about 1,500 L, about 2,000 L, about 2,500 L, about 3,000 L, about 3,500 L, about 4,000 L, about 5,000 L, about 6,000 L, about 7,000 L, about 8,000 L, about 9,000 L, about 10,000 L, about 15,000 L and about 20,000 L.

As shown in Figure 6, the media can be removed from the bioreactor. Such media removal can be continuous for a perfusion bioreactor or can be batchwise for a batch or fed-batch reactor. The media is filtered by filtration system 603 to remove cells. In some embodiments, the cells removed from the media are re-introduced to the bioreactor and the media comprising the secrete recombinant human lysosomal protein can be further processed. Filtration system 603 can be any suitable filtration system, including an alternating tangential flow filtration (ATF) system, a tangential flow filtration (TFF) system, centrifugal filtration system, etc. In various embodiments, the filtration system utilizes a filter having a pore size between about 10 nanometers and about 2 micrometers. Exemplary filter pore sizes include about 10 nm, about 20 nm, about 30 nm, about 40 nm, about 50 nm, about 60 nm, about 70 nm, about 80 nm, about 90 nm, about 100 nm, about 150 nm, about 200 nm, about 250 nm, about 300 nm, about 350 nm, about 400 nm, about 500 nm, about 600 nm, about 700 nm, about 800 nm, about 900 nm, about 1 µm, about 1.5 µm and about 2 µm.

In various embodiments, the media removal rate is between about 1 L/day and about 20,000 L/day. Exemplary media removal rates include about 1 L/day, about 10 L/day, about 20 L/day, about 30 L/day, about 40 L/day, about 50 L/day, about 60 L/day, about 70 L/day, about 80 L/day, about 90 L/day, about 100 L/day, about 150 L/day, about 200 L/day, about 250 L/day, about 300 L/day, about 350 L/day, about 400 L/day, about 500 L/day, about 600 L/day, about 700 L/day, about 800 L/day, about 900 L/day, about 1,000 L/day, about 1,500 L/day, about 2,000 L/day, about 2,500 L/day, about 3,000 L/day, about 3,500 L/day, about 4,000 L/day, about 5,000 L/day, about 6,000 L/day, about 7,000 L/day, about 8,000 L/day, about 9,000 L/day, about 10,000 L/day, about 15,000 L/day and about 20,000 L/day. Alternatively, the media removal rate can be expressed as a function of the bioreactor volume, such as about 0.1 to about 3 reactor volumes per day. Exemplary media removal rates include about 0.1, about 0.2, about 0.3, about 0.4, about 0.5, about 0.6, about 0.7, about 0.8, about 0.9, about 1, about 1.1, about 1.2, about 1.3, about 1.4, about 1.5, about 2, about 2.5 and about 3 reactor volumes per day.

For a continuous or fed-batch process, the rate at which fresh media is provided to the bioreactor can be between about 1 L/day and about 20,000 L/day. Exemplary media introduction rates include about 1 L/day, about 10 L/day, about 20 L/day, about 30 L/day, about 40 L/day, about 50 L/day, about 60 L/day, about 70 L/day, about 80 L/day, about 90 L/day, about 100 L/day, about 150 L/day, about 200 L/day, about 250 L/day, about 300 L/day, about 350 L/day, about 400 L/day, about 500 L/day, about 600 L/day, about 700 L/day, about 800 L/day, about 900 L/day, about 1,000 L/day, about 1,500 L/day, about 2,000 L/day, about 2,500 L/day, about 3,000 L/day, about 3,500 L/day, about 4,000 L/day, about 5,000 L/day, about 6,000 L/day, about 7,000 L/day, about 8,000 L/day, about 9,000 L/day, about 10,000 L/day, about 15,000 L/day and about 20,000 L/day. Alternatively, the media introduction rate can be expressed as a function of the bioreactor volume, such as about 0.1 to about 3 reactor volumes per day. Exemplary media introduction rates include about 0.1, about 0.2, about 0.3, about 0.4, about 0.5, about 0.6, about 0.7, about 0.8, about 0.9, about 1, about 1.1, about 1.2, about 1.3, about 1.4, about 1.5, about 2, about 2.5 and about 3 reactor volumes per day.

After filtration, the filtrate is loaded onto a protein capturing system 605. The protein capturing system 605 can include one or more chromatography columns. If more than one chromatography column is used, then the columns may be placed in series so that the next column can begin loading once the first column is loaded. Alternatively, the media removal process can be stopped during the time that the columns are switched.

In various embodiments, the protein capturing system 605 includes one or more anion exchange (AEX) columns for the direct product capture of recombinant human lysosomal protein, particularly lysosomal protein having a high M6P content. While not wishing to be bound by any particular theory, it is believed that using AEX chromatography to capture the recombinant human lysosomal protein from the filtered media ensures that the captured recombinant protein product has a higher M6P content, due to the more negative charge of the recombinant protein having one or more M6P groups. As a result, non-phosphorylated recombinant protein and host cell impurities do not bind the column resin as well as the highly phosphorylated recombinant protein, and the non-phosphorylated recombinant protein and host cell impurities passes through the column. Accordingly, the AEX chromatography can be used to enrich the M6P content of the protein product (i.e. select for proteins having more M6P) due to the high affinity of the M6P-containing proteins for the AEX resin.

Furthermore, while not wishing to be bound by any particular theory, it is also believed that having a direct product capture of recombinant protein using AEX chromatography ensures that the recombinant proteins having high M6P content are removed from the media containing proteases and other enzymes that can degrade the protein and/or dephosphorylate the protein. As a result, the high quality product is preserved.

Suitable AEX chromatography columns have functional chemical groups that bind negatively charged proteins. Exemplary functional groups include, but are not limited to, primary, secondary, tertiary, and quaternary ammonium or amine groups. These functional groups may be bound to membranes (e.g. cellulose membranes) or conventional chromatography resins. Exemplary column media include SP, CM, Q and DEAE Sepharose^{®} Fast Flow media from GE Healthcare Lifesciences.

The volume of the AEX chromatography column can be any suitable volume, such as between 1 L and 1,000 L. Exemplary column volumes include about 1 L, about 2 L, about 3 L, about 4 L, about 5 L, about 6 L, about 7 L, about 8 L, about 9 L, about 10 L, about 20 L, about 30 L, about 40 L, about 50 L, about 60 L, about 70 L, about 80 L, about 90 L, about 100 L, about 150 L, about 200 L, about 250 L, about 300 L, about 350 L, about 400 L and about 500 L, about 600 L, about 700 L, about 800 L, about 900 L and about 1,000 L.

Exemplary conditions for an anion-exchange column are provided in Table 2 below:

**Table 2**

| **Procedure** | **Buffer** | **Flow rate (cm/h)** | **Volume (CV)** | **Temperature (°C)** |
|---|---|---|---|---|
| Pre-used Sanitization | 0.1-10 M NaOH | ≤ 25-2500 | ≥ 1-3 (≥ 10-120 min) | 15 - 25 |
| Pre-Equilibration | 20-2000 mM phosphate buffer (PB), pH 6.9-7.3 | ≤ 25-2500 | ≥ 1-5 | 15 - 25 |
| Equilibration | 4-400 mM PB, pH 6.9-7.3 | ≤ 25-2500 | ≥ 1-5 | 2 - 15 |
| Load | NA | ≤ 10-1000 | NA | 2 - 15 |
| Wash1 | 4-400 mM PB, pH 6.9-7.3 | ≤ 25-2500 | ≥ 2-10 | 2 - 15 |
| Wash2 | 4-400 mM PB, pH 6.9-7.3 | ≤ 25-2500 | ≥ 2-10 | 15 - 25 |
| Elution | 4-400 mM PB, 20-2000 mM NaCl, pH 6.1-6.5 | ≤ 25-2500 | NA | 15 - 25 |
| Strip | 4-400 mM PB, 0.1-10 M NaCl, pH 6.1-6.5 | ≤ 25-2500 | ≥ 1-5 | 15 - 25 |
| Post-use Sanitization | 0.1-10 M NaOH | ≤ 25-2500 | ≥ 1-3 (≥ 10-120 min) | 15 - 25 |
| Storage | 0.01-1.0 M NaOH | ≤ 25-2500 | ≥ 1-5 | 15 - 25 |

After the recombinant human lysosomal protein is loaded onto the protein capturing system 605, the recombinant human lysosomal protein is eluted from the column(s) by changing the pH and/or salt content in the column.

The eluted recombinant human lysosomal protein can be subjected to further purification steps and/or quality assurance steps. For example, as shown in Figure 6, the eluted recombinant human lysosomal protein can be subjected to a virus kill step 607. Such a virus kill 607 can include one or more of a low pH kill, a detergent kill, or other technique known in the art.

The recombinant protein product from the virus kill step 607 can be introduced into a second chromatography system 609 to further purify the recombinant protein product. Alternatively, the eluted recombinant protein from the protein capturing system 605 can be fed directly to the second chromatography system 609. In various embodiments, the second chromatography system 609 includes one or more immobilized metal affinity chromatography (IMAC) columns for further removal of impurities. Exemplary metal ions include cobalt, nickel, copper, iron, zinc or gallium.

The volume of the second chromatography column (e.g. IMAC column) can be any suitable volume, such as between 0.1 L and 100 L. Exemplary column volumes include about 0.1 L, about 0.2 L, about 0.3 L, about 0.4 L, about 0.5 L, about 0.6 L, about 0.7 L, about 0.8 L, about 0.9 L, about 1 L, about 1.5 L, about 2 L, about 2.5L, about 3 L, about 3.5 L, about 4 L, about 4.5 L, about 5 L, about 6 L, about 7 L, about 8 L, about 9 L, about 10 L, about 15 L, about 20 L, about 25 L, about 30 L, about 35 L, about 40 L and about 50 L, about 60 L, about 70 L, about 80 L, about 90 L and about 100 L.

Exemplary conditions for an IMAC column are provided in Table 3 below:

**Table 3**

| Procedure | Buffer | Flow rate (cm/h) | Vol (CV) |
|---|---|---|---|
| Rinse | 4-400 mM PB, pH 6.3-6.7 | ≤ 25-2500 | ≥ 1-5 |
| Pre-use Sanitization | 0.01-1.0 M NaOH | ≤ 25-2500 | ≥ 1-3 (10 - 30 min) |
| Equilibration | 4-400 mM PB, pH 6.5 | ≤ 25-2500 | ≥ 1-5 |
| Wash with WFI | Water For Injection (WFI) | ≤ 25-2500 | ≥ 1-3 |
| Chelating | 0.01-1.0 M Copper Acetate | ≤ 25-2500 | ≥ 1-5 |
| Wash with WFI | WFI | ≤ 25-2500 | ≥ 2-10 |
| Wash with acidic buffer | 2-200 mM Sodium Acetate, 0.05-5 M NaCl, pH 3.5-4.5 | ≤ 25-2500 | ≥ 2-10 |
| Equilibration | 4-400 mM PB, pH 6.3-6.7 | ≤ 25-2500 | ≥ 1-5 |
| Blank run with elution buffer | 4-400 mM PB, 15-1500 mM Glycine, pH 6.1-6.5 | ≤ 25-2500 | ≥ 2-20 |
| Equilibration | 4-400 mM PB, pH 6.3-6.7 | ≤ 25-2500 | ≥ 1-5 |
| Load | NA | ≤ 25-2500 | ≥ 1-5 |
| Wash1 | 4-400 mM PB, pH 6.3-6.7 | ≤ 25-2500 | ≥ 2-10 |
| Wash2 | 4-400 mM PB, 0.1-10 M NaCl, 5-30% propylene glycol, pH 6.3-6.7 | ≤ 25-2500 | ≥ 2-10 |
| Wash3 | 4-400 mM PB, pH 6.3-6.7 | ≤ 25-2500 | ≥ 2-10 |
| Elution | 4-400 mM PB, 15-1500 mM Glycine, pH 6.1-6.5 | ≤ 25-2500 | NA |
| Strip | 4-400 mM PB, 50-5000 mM imidazole, pH 6.3-6.7 | ≤ 25-2500 | ≥ 1-5 |
| Post-use Sanitization | 0.01-1M NaOH | ≤ 25-2500 | ≥ 1-3 (10 - 30 min) |
| Rinse | 4-400 mM PB, pH 6.3-6.7 | ≤ 25-2500 | ≥ 1-5 |
| Storage | 5-30% ethanol | ≤ 25-2500 | ≥ 1-5 |

After the recombinant protein is loaded onto the second chromatography system 609, the recombinant protein is eluted from the column(s). As shown in Figure 6, the eluted recombinant protein can be subjected to a virus kill step 611. As with virus kill 607, virus kill 611 can include one or more of a low pH kill, a detergent kill, or other technique known in the art. In some embodiments, only one of virus kill 607 or 611 is used, or the virus kills are performed at the same stage in the purification process.

As shown in Figure 6, the recombinant protein product from the virus kill step 611 can be introduced into a third chromatography system 613 to further purify the recombinant protein product. Alternatively, the eluted recombinant protein from the second chromatography system 609 can be fed directly to the third chromatography system 613. In various embodiments, the third chromatography system 613 includes one or more cation exchange chromatography (CEX) columns and/or size exclusion chromatography (SEC) columns for further removal of impurities. The recombinant protein product is then eluted from the third chromatography system 613.

The volume of the third chromatography column (e.g. CEX or SEC column) can be any suitable volume, such as between 0.1 L and 200 L. Exemplary column volumes include about 0.1 L, about 0.2 L, about 0.3 L, about 0.4 L, about 0.5 L, about 0.6 L, about 0.7 L, about 0.8 L, about 0.9 L, about 1 L, about 1.5 L, about 2 L, about 2.5L, about 3 L, about 3.5 L, about 4 L, about 4.5 L, about 5 L, about 6 L, about 7 L, about 8 L, about 9 L, about 10 L, about 15 L, about 20 L, about 25 L, about 30 L, about 35 L, about 40 L and about 50 L, about 60 L, about 70 L, about 80 L, about 90 L, about 100 L, about 150 L and about 200 L.

Exemplary conditions for a CEX column are provided in Table 4 below:

**Table 4**

| Procedure | Buffer | Flow rate (cm/h) | Vol (CV) |
|---|---|---|---|
| Pre-used Sanitization | 0.1-10 M NaOH | ≤ 25-2500 | ≥ 1-3 (≥ 10-120 min) |
| Equilibration | 2-200 mM Sodium citrate, pH 4.0-5.0 | ≤ 30-3000 | ≥ 2-10 |
| Load | NA | ≤ 30-3000 | NA |
| Wash | 2-200 mM Sodium citrate, pH 4.0-5.0 | ≤ 30-3000 | ≥ 2-10 |
| Elution | 2-200 mM Sodium citrate, 15-1500 mM NaCl, pH 4.0-5.0 | ≤ 30-3000 | ≥ 2-10 |
| Strip | 2-200 mM Sodium citrate, 0.1-10 M NaCl, pH 4.0-5.0 | ≤ 30-3000 | ≥ 1-5 |
| Post-use Sanitization | 0.1-10 M NaOH | ≤ 25-2500 | ≥ 1-3 (≥ 10-120 min) |
| Storage | 0.01-1.0 M NaOH | ≤ 30-3000 | ≥ 1-5 |

The recombinant protein product may also be subjected to further processing. For example, another filtration system 615 may be used to remove viruses. In some embodiments, such filtration can utilize filters with pore sizes between 5 and 50 µm. Other product processing can include a product adjustment step 617, in which the recombinant protein product may be sterilized, filtered, concentrated, stored and/or have additional components for added for the final product formulation. For example, the recombinant protein product can be concentrated by a factor of 2-10 times, such as from an initial protein concentration of about 2 to about 20 mg/ml to a final protein concentration of about 4 to about 200 mg/ml. This final product can be used to fill vials and may be lyophilized for future use.

### Administration of Recombinant Protein

Medical uses comprising administration of rhGAA are aspects that are useful to illustrate the present invention, but are not claimed as such.

The rhGAA or a pharmaceutically acceptable salt thereof, can be formulated in accordance with the routine procedures as a pharmaceutical composition adapted for administration to human beings. For example, in a preferred embodiment, a composition for intravenous administration is a solution in sterile isotonic aqueous buffer. Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampule or sachct indicating the quantity of active agent. Where the composition is to be administered by infusion, it can be dispensed with an infusion bottle containing sterile pharmaceutical grade water, saline or dextrose/water. Where the composition is administered by injection, an ampule of sterile water for injection or saline can be provided so that the ingredients may be mixed prior to administration.

rhGAA (or a composition or medicament containing rhGAA is administered by an appropriate route. In one embodiment, the rhGAA is administered intravenously. In other embodiments, rhGAA) is administered by direct administration to a target tissue, such as to heart or skeletal muscle (e.g. intramuscular), or nervous system (e.g. direct injection into the brain; intraventricularly; intrathecally). More than one route can be used concurrently, if desired.

The rhGAA (or a composition or medicament containing rhGAA is administered in a therapeutically effective amount (e.g. a dosage amount that, when administered at regular intervals, is sufficient to treat the disease, such as by ameliorating symptoms associated with the disease, preventing or delaying the onset of the disease, and/or lessening the severity or frequency of symptoms of the disease). The amount which will be therapeutically effective in the treatment of the disease will depend on the nature and extent of the disease's effects, and can be determined by standard clinical techniques. In addition, in vitro or in vivo assays may optionally be employed to help identify optimal dosage ranges. The precise dose to be employed will also depend on the route of administration, and the seriousness of the disease, and should be decided according to the judgment of a practitioner and each patient's circumstances. Effective doses may be extrapolated from dose-response curves derived from in vitro or animal model test systems. In at least one embodiment, the recombinant human acid α-glucosidase is administered by intravenous infusion at a dose of about about 1 mg/kg to about 100 mg/kg, such as about 5 mg/kg to about 30 mg/kg, typically about 5 mg/kg to about 20 mg/kg. In at least one embodiment, the recombinant human acid α-glucosidase is administered by intravenous infusion at a dose of about 5 mg/kg, about 10 mg/kg, about 15 mg/kg, about 20 mg/kg, about 25 mg/kg, about 30 mg/kg, about 35 mg/kg, about 40 mg/kg, about 50 mg/kg, about 50 mg/kg, about 60 mg/kg, about 70 mg/kg, about 80 mg/kg, about 90 mg/kg or about 100 mg/kg. In at least one embodiment, the recombinant human acid α-glucosidase is administered by intravenous infusion at a dose of about 20 mg/kg. The effective dose for a particular individual can be varied (e.g. increased or decreased) over time, depending on the needs of the individual. For example, in times of physical illness or stress, or if anti-acid α-glucosidase antibodies become present or increase, or if disease symptoms worsen, the amount can be increased.

The therapeutically effective amount of recombinant human acid α-glucosidase (or composition or medicament containing recombinant human acid α-glucosidase) is administered at regular intervals, depending on the nature and extent of the disease's effects, and on an ongoing basis. Administration at a "regular interval," as used herein, indicates that the therapeutically effective amount is administered periodically (as distinguished from a one-time dose). The interval can be determined by standard clinical techniques. In preferred embodiments, recombinant human acid α-glucosidase is administered monthly, bimonthly; weekly; twice weekly; or daily. The administration interval for a single individual need not be a fixed interval, but can be varied over time, depending on the needs of the individual. For example, in times of physical illness or stress, if anti-recombinant human acid α-glucosidase antibodies become present or increase, or if disease symptoms worsen, the interval between doses can be decreased. In some embodiments, a therapeutically effective amount of 5, 10, 20, 50, 100, or 200 mg enzyme/kg body weight is administered twice a week, weekly or every other week with or without a chaperone.

The rhGAA may be prepared for later use, such as in a unit dose vial or syringe, or in a bottle or bag for intravenous administration. Kits containing the rhGAA as well as optional excipients or other active ingredients, such as chaperones or other drugs, may be enclosed in packaging material and accompanied by instructions for reconstitution, dilution or dosing for use in a method of treating a subject in need of treatment, such as a patient having Pompe disease.

### Combination Therapy of rhGAA and Pharmacological Chaperone

Medical uses comprising combination therapy of rhGAA and phamacological chaperone are aspects that are useful to illustrate the present invenition, but are not claimed as such. In various embodiments, the rhGAA (e.g. ATB200) produced by the processes described herein can be used in combination therapy with a pharmacological chaperone such as miglustat or duvoglustat.

In at least one embodiment, the pharmacological chaperone (e.g. miglustat) is administered orally. In at least one embodiment, the miglustat is administered at an oral dose of about 200 mg to about 400 mg, or at an oral dose of about 200 mg, about 250 mg, about 300 mg, about 350 mg or about 400 mg. In at least one embodiment, the miglustat is administered at an oral dose of about 233 mg to about 400 mg. In at least one embodiment, the miglustat is administered at an oral dose of about 250 to about 270 mg, or at an oral dose of about 250 mg, about 255 mg, about 260 mg, about 265 mg or about 270 mg. In at least one embodiment, the miglustat is administered as an oral dose of about 260 mg.

It will be understood by those skilled in the art that an oral dose of miglustat in the range of about 200 mg to 400 mg or any smaller range therewithin can be suitable for an adult patient with an average body weight of about 70 kg. For patients having a significantly lower body weight than about 70 kg, including but not limited to infants, children or underweight adults, a smaller dose may be considered suitable by a physician. Therefore, in at least one embodiment, the miglustat is administered as an oral dose of from about 50 mg to about 200 mg, or as an oral dose of about 50 mg, about 75 mg, about 100 mg, 125 mg, about 150 mg, about 175 mg or about 200 mg. In at least one embodiment, the miglustat is administered as an oral dose of from about 65 mg to about 195 mg, or as an oral dose of about 65 mg, about 130 mg or about 195 mg.

In at least one embodiment, the miglustat is administered as a pharmaceutically acceptable dosage form suitable for oral administration, and includes but is not limited to tablets, capsules, ovules, elixirs, solutions or suspensions, gels, syrups, mouth washes, or a dry powder for reconstitution with water or other suitable vehicle before use, optionally with flavoring and coloring agents for immediate-, delayed-, modified-, sustained-, pulsed- or controlled-release applications. Solid compositions such as tablets, capsules, lozenges, pastilles, pills, boluses, powder, pastes, granules, bullets, dragées or premix preparations can also be used. In at least one embodiment, the miglustat is administered as a tablet. In at least one embodiment, the miglustat is administered as a capsule. In at least one embodiment, the dosage form contains from about 50 mg to about 300 mg of miglustat. In at least one embodiment, the dosage form contains about 65 mg of miglustat. In at least one embodiment, the dosage form contains about 130 mg of miglustat. In at least one embodiment, the dosage form contains about 260 mg of miglustat. It is contemplated that when the dosage form contains about 65 mg of miglustat, the miglustat can be administered as a dosage of four dosage forms, or a total dose of 260 mg of miglustat. However, for patients who have a significantly lower weight than an average adult weight of 70 kg, including but not limited to infants, children or underweight adults, the miglustat can be administered as a dosage of one dosage form (a total dose of 65 mg of miglustat), two dosage forms (a total dose of 130 mg of miglustat), or three dosage forms (a total dose of 195 mg of miglustat).

Solid and liquid compositions for oral use can be prepared according to methods well known in the art. Such compositions can also contain one or more pharmaceutically acceptable carriers and excipients which can be in solid or liquid form. Tablets or capsules can be prepared by conventional means with pharmaceutically acceptable excipients, including but not limited to binding agents, fillers, lubricants, disintegrants or wetting agents. Suitable pharmaceutically acceptable excipients are known in the art and include but are not limited to pregelatinized starch, polyvinylpyrrolidone, povidone, hydroxypropyl methylcellulose (HPMC), hydroxypropyl ethylcellulose (HPEC), hydroxypropyl cellulose (HPC), sucrose, gelatin, acacia, lactose, microcrystalline cellulose, calcium hydrogen phosphate, magnesium stearate, stearic acid, glyceryl behenate, talc, silica, corn, potato or tapioca starch, sodium starch glycolate, sodium lauryl sulfate, sodium citrate, calcium carbonate, dibasic calcium phosphate, glycine croscarmellose sodium and complex silicates. Tablets can be coated by methods well known in the art. In at least one embodiment, the miglustat is administered as a formulation available commercially as Zavesca^{®} (Actelion Pharmaceuticals).

In at least one embodiment, the miglustat and the recombinant human acid α-glucosidase are administered simultaneously. In at least one embodiment, the miglustat and the recombinant human acid α-glucosidase are administered sequentially. In at least one embodiment, the miglustat is administered prior to administration of the recombinant human acid α-glucosidase. In at least one embodiment, the miglustat is administered less than three hours prior to administration of the recombinant human acid α-glucosidase. In at least one embodiment, the miglustat is administered about two hours prior to administration of the recombinant human acid α-glucosidase. In at least one embodiment, the miglustat is administered less than two hours prior to administration of the recombinant human acid α-glucosidase. In at least one embodiment, the miglustat is administered about 1.5 hours prior to administration of the recombinant human acid α-glucosidase. In at least one embodiment, the miglustat is administered about one hour prior to administration of the recombinant human acid α-glucosidase. In at least one embodiment, the miglustat is administered from about 50 minutes to about 70 minutes prior to administration of the recombinant human acid α-glucosidase. In at least one embodiment, the miglustat is administered from about 55 minutes to about 65 minutes prior to administration of the recombinant human acid α-glucosidase. In at least one embodiment, the miglustat is administered about 30 minutes prior to administration of the recombinant human acid α-glucosidase. In at least one embodiment, the miglustat is administered from about 25 minutes to about 35 minutes prior to administration of the recombinant human acid α-glucosidase. In at least one embodiment, the miglustat is administered from about 27 minutes to about 33 minutes prior to administration of the recombinant human acid α-glucosidase.

In at least one embodiment, the miglustat is administered concurrently with administration of the recombinant human acid α-glucosidase. In at least one embodiment, the miglustat is administered within 20 minutes before or after administration of the recombinant human acid α-glucosidase. In at least one embodiment, the miglustat is administered within 15 minutes before or after administration of the recombinant human acid α-glucosidase. In at least one embodiment, the miglustat is administered within 10 minutes before or after administration of the recombinant human acid α-glucosidase. In at least one embodiment, the miglustat is administered within 5 minutes before or after administration of the recombinant human acid α-glucosidase.

In at least one embodiment, the miglustat is administered after administration of the recombinant human acid α-glucosidase. In at least one embodiment, the miglustat is administered up to 2 hours after administration of the recombinant human acid α-glucosidase. In at least one embodiment, the miglustat is administered about 30 minutes after administration of the recombinant human acid α-glucosidase. In at least one embodiment, the miglustat is administered about one hour after administration of the recombinant human acid α-glucosidase. In at least one embodiment, the miglustat is administered about 1.5 hours after administration of the recombinant human acid α-glucosidase. In at least one embodiment, the miglustat is administered about 2 hours after administration of the recombinant human acid α-glucosidase.

Another aspect useful for illustrating the invention, but not claimed as such, provides a kit for use in combination therapy of Pompe disease in a patient in need thereof. The kit includes a pharmaceutically acceptable dosage form comprising miglustat, a pharmaceutically acceptable dosage form comprising a recombinant human acid α-glucosidase as defined herein, and instructions for administering the pharmaceutically acceptable dosage form comprising miglustat and the pharmaceutically acceptable dosage form comprising the recombinant acid α-glucosidase to a patient in need thereof. In at least one embodiment, the pharmaceutically acceptable dosage form comprising miglustat is an oral dosage form as described herein, including but not limited to a tablet or a capsule. In at least one embodiment, the pharmaceutically acceptable dosage form comprising a recombinant human acid α-glucosidase is a sterile solution suitable for injection as described herein. In at least one embodiment, the instructions for administering the dosage forms include instructions to administer the pharmaceutically acceptable dosage form comprising miglustat orally prior to administering the pharmaceutically acceptable dosage form comprising the recombinant human acid α-glucosidase by intravenous infusion, as described herein.

Without being bound by theory, it is believed that miglustat acts as a pharmacological chaperone for the recombinant human acid α-glucosidase ATB200 and binds to its active site. For example, miglustat has been found to decrease the percentage of unfolded ATB200 protein and stabilize the active conformation of ATB200, preventing denaturation and irreversible inactivation at the neutral pH of plasma and allowing it to survive conditions in the circulation long enough to reach and be taken up by tissues. However, the binding of miglustat to the active site of ATB200 also can result in inhibition of the enzymatic activity of ATB200 by preventing the natural substrate, glycogen, from accessing the active site. It is believed that when miglustat and the recombinant human acid α-glucosidase are administered to a patient under the conditions described herein, the concentrations of miglustat and ATB200 within the plasma and tissues are such that ATB200 is stabilized until it can be taken up into the tissues and targeted to lysosomes, but, because of the rapid clearance of miglustat, hydrolysis of glycogen by ATB200 within lysosomes is not overly inhibited by the presence of miglustat, and the enzyme retains sufficient activity to be therapeutically useful.

All the embodiments described above may be combined. This includes in particular embodiments relating to:
- the nature of the pharmacological chaperone, for example miglustat; and the active site for which it is specific;
- the dosage, route of administration of the pharmacological chaperone (e.g. miglustat) and the type of pharmaceutical composition including the nature of the carrier and the use of commercially available compositions;
- the nature of the drug, e.g. therapeutic protein drug product, which may be a counterpart of an endogenous protein for which expression is reduced or absent in the subject, suitably rhGAA, for example the recombinant human acid α-glucosidase expressed in Chinese hamster ovary (CHO) cells and comprising an increased content of N-glycan units bearing one or more mannose-6-phosphate residues when compared to a content of N-glycan units bearing one or more mannose-6-phosphate residues of alglucosidase alfa; and suitably having an amino acid sequence as set forth in SEQ ID NO: 1 or SEQ ID NO: 2;
- the number and type of N-glycan units on the rhGAA rhGAA), such as the N-acetylglucosamine, galactose, sialic acid or complex N-glycans formed from combinations of these, attached to the rhGAA;
- the degree of phosphorylation of mannose units on the rhGAA to form mannose-6-phosphate and/or bis-mannose-6-phosphate;
- the dosage and route of administration (e.g. intravenous administration, especially intravenous infusion, or direct administration to the target tissue) of the recombinant human acid α-glucosidase and the type of formulation including carriers and therapeutically effective amount;
- the dosage interval of the pharmacological chaperone (miglustat) and the recombinant human acid α-glucosidase;
- the nature of the therapeutic response and the results of the combination therapy (e.g. enhanced results as compared to the effect of each therapy performed individually);
- the timing of the administration of the combination therapy, e.g. simultaneous administration of miglustat and the recombinant human acid α-glucosidase or sequential administration, for example wherein the miglustat is administered prior to the recombinant human acid α-glucosidase or after the recombinant human acid α-glucosidase or within a certain time before or after administration of the recombinant human acid α-glucosidase; and
- the nature of the patient treated (e.g. mammal such as human) and the condition suffered by the individual (e.g. enzyme insufficiency).

Any of the embodiments in the list above may be combined with one or more of the other embodiments in the list.

### EXAMPLES

Other features of the present invention or useful for illustrating the invention will become apparent from the following non-limiting examples which illustrate, by way of example, the principles of the invention as defined in the claims.

### Example 1: Limitations of existing Myozyme^{®} and Lumizyme^{®} rhGAA products

To evaluate the ability of the rhGAA in Myozyme^{®} and Lumizyme^{®}, the only currently approved treatments for Pompe disease, these rhGAA preparations were injected onto a CIMPR column (which binds rhGAA having M6P groups) and subsequently eluted with a free M6 gradient. Fractions were collected in 96-well plate and GAA activity assayed by 4MU-α-glucose substrate. The relative amounts of bound and unbound rhGAA were determined based on GAA activity and reported as the fraction of total enzyme.

Figures 4A-B describe the problems associated with conventional ERTs (Myozyme^{®} and Lumizyme^{®}): 73% of the rhGAA in Myozyme^{®} (Figure 4B) and 78% of the rhGAA in Lumizyme^{®} (Figure 4A) did not bind to the CIMPR, see the left-most peaks in each figure. Only 27% of the rhGAA in Myozyme^{®} and 22% of the rhGAA in Lumizyme^{®} contained M6P that can productive to target it to the CIMPR on muscle cells.

An effective dose of Myozyme^{®} and Lumizyme^{®} corresponds to the amount of rhGAA containing M6P which targets the CIMPR on muscle cells. However, most of the rhGAA in these two conventional products does not target the CIMPR receptor on target muscle cells. The administration of a conventional rhGAA where most of the rhGAA is not targeted to muscle cells increases the risk of allergic reaction or induction of immunity to the non-targeted rhGAA.

### Example 2: Preparation of CHO Cells Producing ATB200 rhGAA having a high content of mono- or bis-M6P-bearing N-glycans

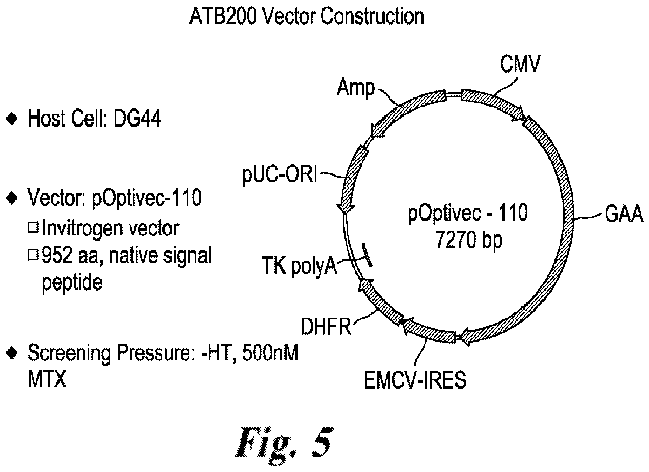

CHO cells were transfected with DNA that expresses rhGAA followed by selection of transformants producing rhGAA. A DNA construct for transforming CHO cells with DNA encoding rhGAA is shown in Figure 5. CHO cells were transfected with DNA that expresses rhGAA followed by selection of transformants producing rhGAA.

After transfection, DG44 CHO (DHFR-) cells containing a stably integrated GAA gene were selected with hypoxanthine/thymidine deficient (-HT) medium). Amplification of

GAA expression in these cells was induced by methotrexate treatment (MTX, 500 nM). Cell pools that expressed high amounts of GAA were identified by GAA enzyme activity assays and were used to establish individual clones producing rhGAA. Individual clones were generated on semisolid media plates, picked by ClonePix system, and were transferred to 24-deep well plates. The individual clones were assayed for GAA enzyme activity to identify clones expressing a high level of GAA. Conditioned media for determining GAA activity used a 4-MU-α-Glucosidase substrate. Clones producing higher levels of GAA as measured by GAA enzyme assays were further evaluated for viability, ability to grow, GAA productivity, N-glycan structure and stable protein expression. CHO cell lines, including CHO cell line GA-ATB-200, expressing rhGAA with enhanced mono-M6P or bis-M6P N-glycans were isolated using this procedure.

### Example 3: Capturing and Purification of ATB200 rhGAA

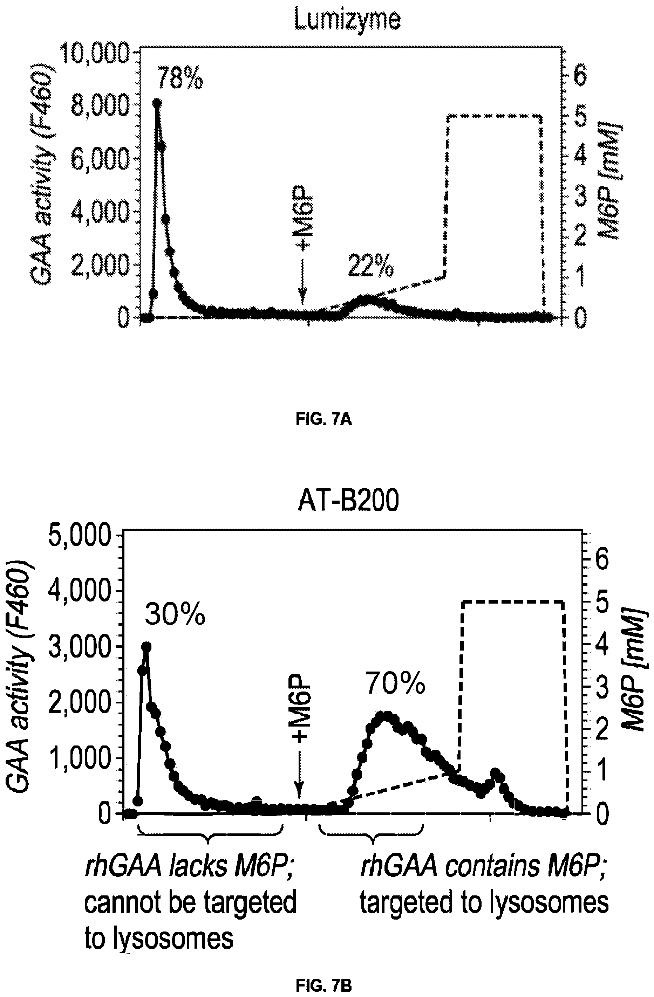

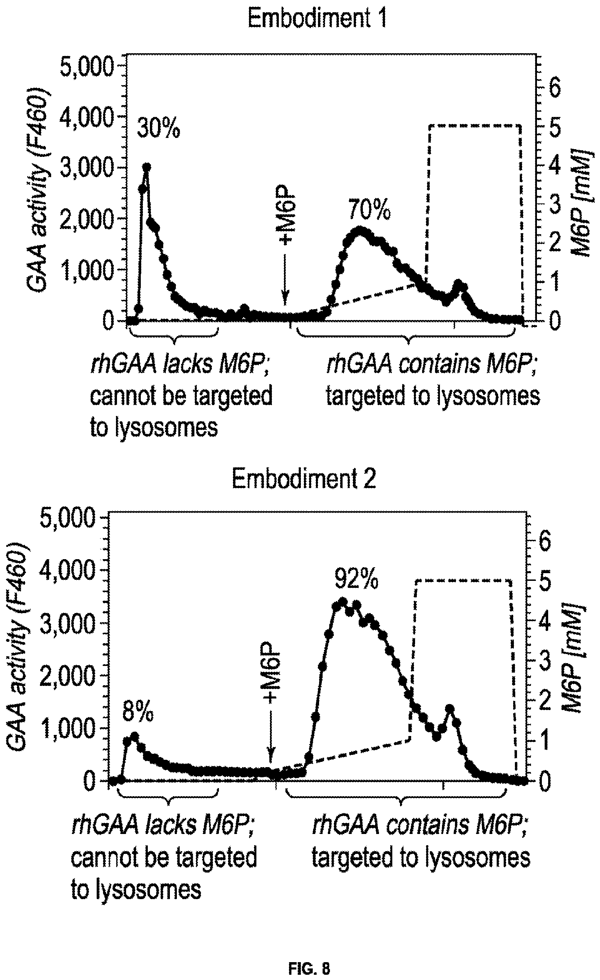

Multiple batches of the rhGAA according to the invention were produced in shake flasks and in perfusion bioreactors using CHO cell line GA-ATB-200 and CIMPR binding was measured. Similar CIMPR receptor binding (~70%) to that shown in Figure 7B and Figure 8 was observed for purified ATB200 rhGAA from different production batches indicating that ATB200 rhGAA can be consistently produced. As shown by Figures 4A, 4B, 7A and 7B, Myozyme^{®} and Lumizyme^{®} rhGAAs exhibited significantly less CIMPR binding than ATB200 rhGAA.

### Example 4: Analytical Comparison of ATB200 to Lumizyme^{®}

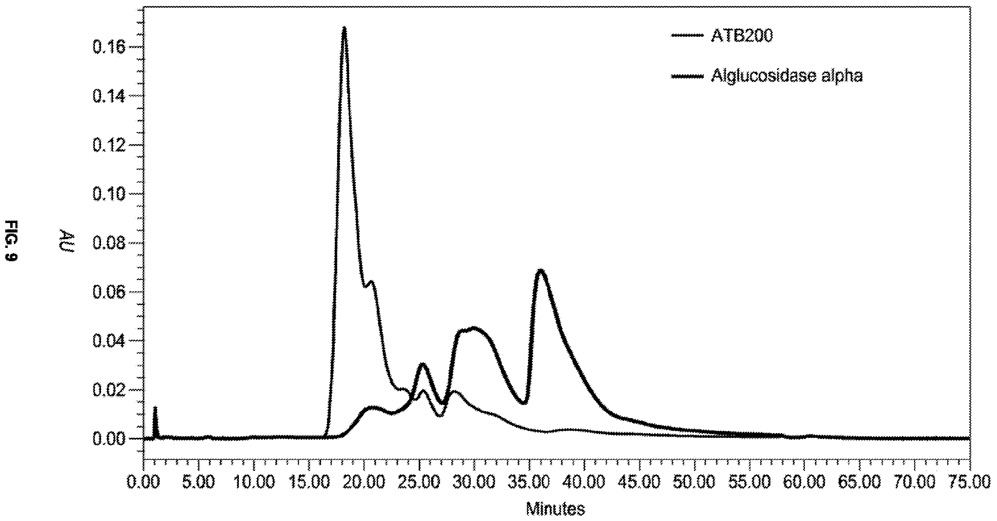

Weak anion exchange ("WAX") liquid chromatography was used to fractionate ATB200 rhGAA according to terminal phosphate. Elution profiles were generated by eluting the ERT with increasing amount of salt. The profiles were monitored by UV (A280nm). ATB200 rhGAA was obtained from CHO cells and purified. Lumizyme^{®} was obtained from a commercial source. Lumizyme^{®} exhibited a high peak on the left of its elution profile. ATB200 rhGAA exhibited four prominent peaks eluting to the right of Lumizyme^{®} (Figure 9). This confirms that ATB200 rhGAA was phosphorylated to a greater extent than Lumizyme^{®} since this evaluation is by terminal charge rather than CIMPR affinity.

### Example 5: Oligosaccharide Characterization of ATB200 rhGAA

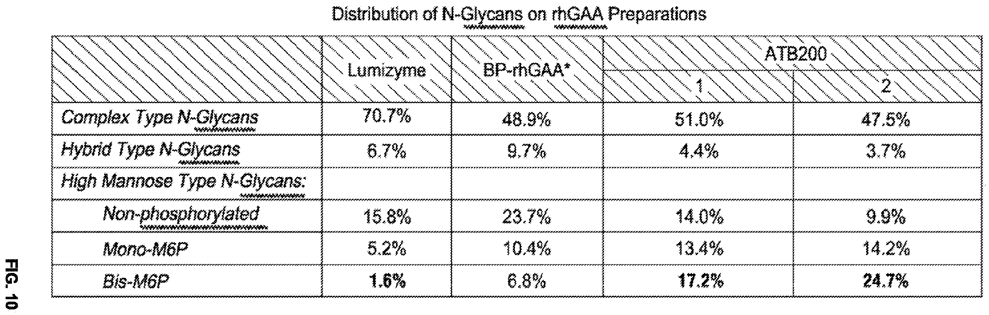

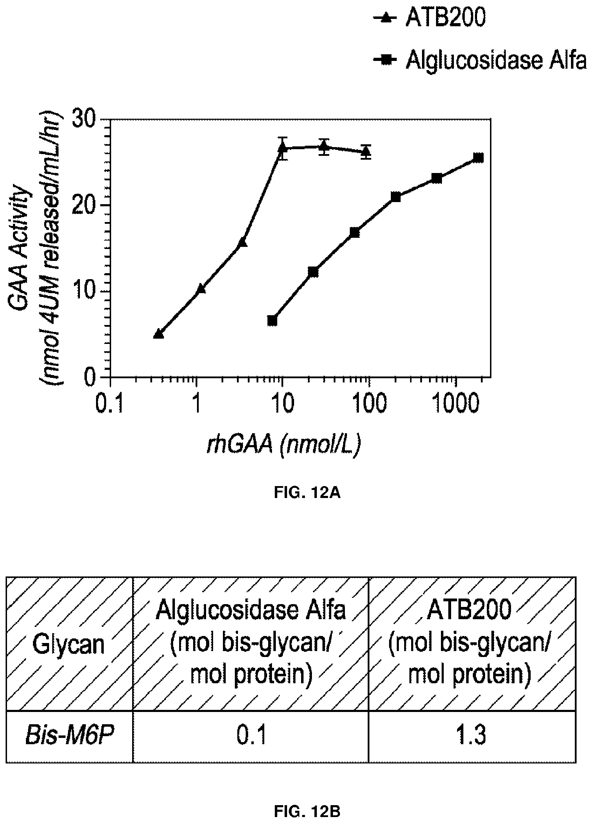

Purified ATB200 rhGAA and Lumizyme^{®} glycans were evaluated by MALDI-TOF to determine the individual glycan structures found on each ERT (Figure 10). ATB200 samples were found to contain lower amounts of non-phosphorylated high-mannose type N-glycans than Lumizyme^{®}. The higher content of M6P glycans in ATB200 than in Lumizyme^{®}, targets ATB200 rhGAA to muscle cells more effectively. The high percentage of mono-phosphorylated and bis-phosphorylated structures determined by MALDI agree with the CIMPR profiles which illustrated significantly greater binding of ATB200 to the CIMPR receptor. N-glycan analysis via MALDI-TOF mass spectrometry confirmed that on average each ATB200 molecule contains at least one natural bis-M6P N-glycan structure. This higher bis-M6P N-glycan content on ATB200 rhGAA directly correlated with high-affinity binding to CIMPR in M6P receptor plate binding assays (KD about 2-4 nM) Figure 12A.

ATB200 rhGAA was also analyzed for site-specific N-glycan profiles using two different LC-MS/MS analytical techniques. In the first analysis, the protein was denatured, reduced, alkylated and digested prior to LC-MS/MS analysis. During protein denaturation and reduction, 200 µg of protein sample, 5 µL 1 mol/L tris-HCl (final concentration 50mM), 75 µL 8 mol/L guanidine HCl (final concentration 6 M), 1 µL 0.5 mol/L EDTA (final concentration 5 mM), 2 µL 1 mol/L DTT (final concentration 20 mM) and Milli-Q^{®} water were added to a 1.5 mL tube to provide a total volume of 100 µL. The sample was mixed and incubated at 56°C for 30 minutes in a dry bath. During alkylation, the denatured and reduced protein sample was mixed with 5 µL 1 mol/L iodoacetamide (IAM, final concentration 50 mM), then incubated at 10-30°C in the dark for 30 minutes. After alkylation, 400 µL of precooled acetone was added to the sample and the mixture was frozen at -80°C refrigeration for 4 hours. The sample was then centrifuged for 5 min at 13000 rpm at 4°C and the supernatant was removed. 400 µL of precooled acetone was added to the pellets, which was then centrifuged for 5 min at 13000 rpm at 4°C and the supernatant was removed. The sample was then air dried on ice in the dark to remove acetone residue. 40 µL of 8M urea and 160 µL of 100 mM NH₄HCO₃ were added to the sample to dissolve the protein. During trypsin digestion, 50 µg of the protein was then added with trypsin digestion buffer to a final volume of 100 µL, and 5 µL 0.5 mg/mL trypsin (protein to enzyme ratio of 20/1 w/w) was added. The solution was mixed well and incubated overnight (16 ± 2 hours) at 37°C. 2.5 µL 20% TFA (final concentration 0.5%) was added to quench the reaction. The sample was then analyzed using the Thermo Scientific Orbitrap Velos Pro^{™} Mass Spectrometer.

In the second LC-MS/MS analysis, the ATB200 sample was prepared according to a similar denaturation, reduction, alkylation and digestion procedure, except that iodoacetic acid (IAA) was used as the alkylation reagent instead of IAM, and then analyzed using the Thermo Scientific Orbitrap Fusion Lumos Tribid^{™} Mass Spectrometer.

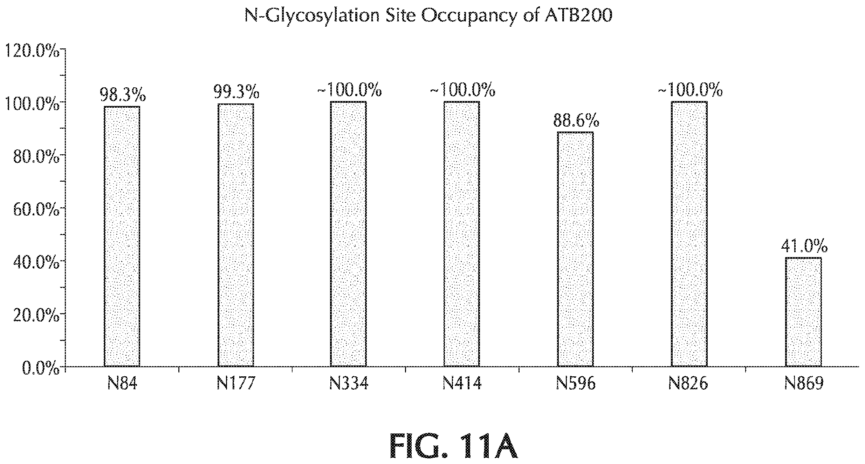

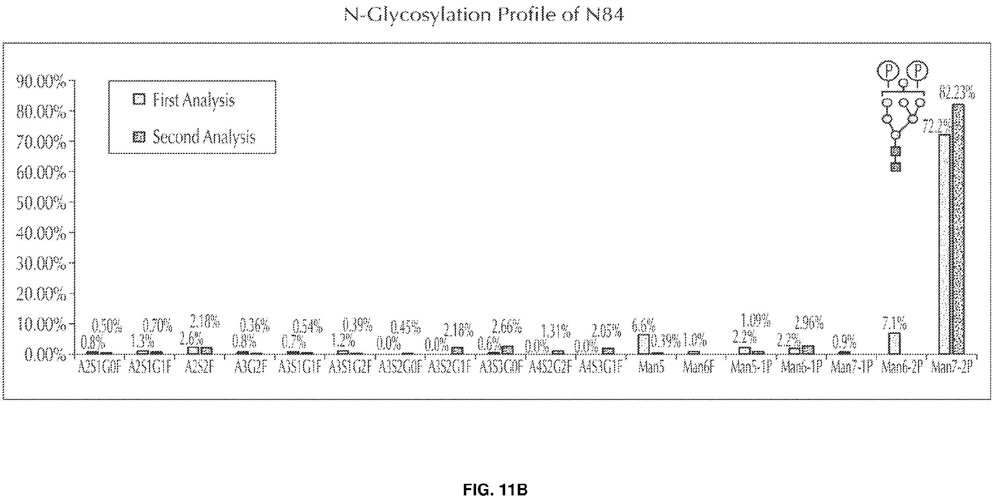

The results of the first and second analyses are shown in Figures 11A-11H. In Figures 11A-11H, the results of the first analysis are represented by left bar (dark grey) and the results from the second analysis are represented by the right bar (light grey). In Figures 11B-11G, the symbol nomenclature for glycan representation is in accordance with Varki, A., Cummings, R.D., Esko J.D., et al., Essentials of Glycobiology, 2nd edition (2009).

As can be seen from Figures 8A-8I, the two analyses provided similar results, although there was some variation between the results. This variation can be due to a number of factors, including the instrument used and the completeness of N-glycan analysis. For example, if some species of phosphorylated glycans were not identified and/or not quantified, then the total number of phosphorylated glycans may be underrepresented, and the percentage of rhGAA bearing the phosphorylated glycans at that site may be underrepresented. As another example, if some species of non-phosphorylated glycans were not identified and/or not quantified, then the total number of non-phosphorylated glycans may be underrepresented, and the percentage of rhGAA bearing the phosphorylated glycans at that site may be overrepresented. Figure 11A shows the N-glycosylation site occupancy of ATB200. As can be seen from Figure 11A, the first, second, third, fourth, fifth and sixth N-glycosylation sites are mostly occupied, with both analyses detecting over 90% and up to about 100% of the ATB200 enzyme having a glycan detected at each potential site. However, the seventh potential N-glycosylation site is glycosylated about half of the time.

Figure 11B shows the N-glycosylation profile of the first site, N84. As can be seen from Figure 11B, the major glycan species is bis-M6P glycans. Both the first and second analyses detected over 75% of the ATB200 had a bis-M6P glycan at the first site.

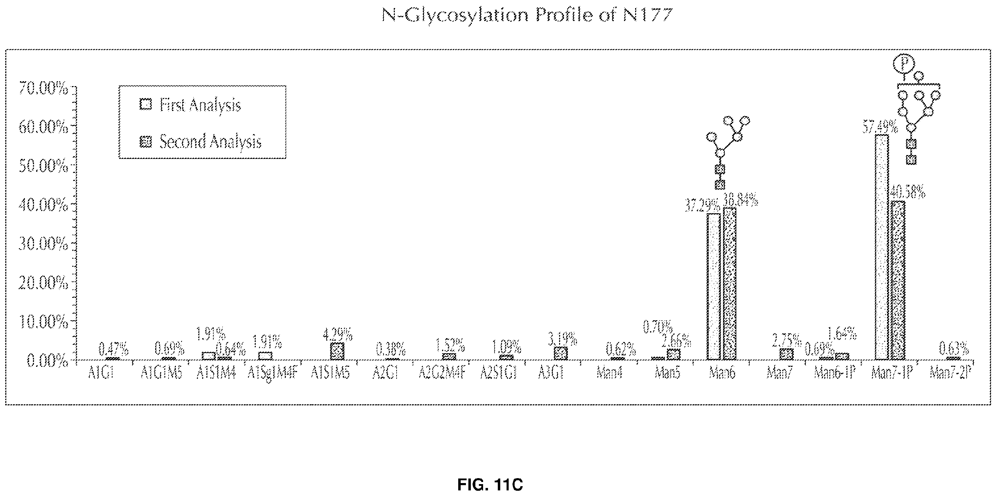

Figure 11C shows the N-glycosylation profile of the second site, N177. As can be seen from Figure 11C, the major glycan species are mono-M6P glycans and non-phosphorylated high mannose glycans. Both the first and second analyses detected over 40% of the ATB200 had a mono-M6P glycan at the second site.

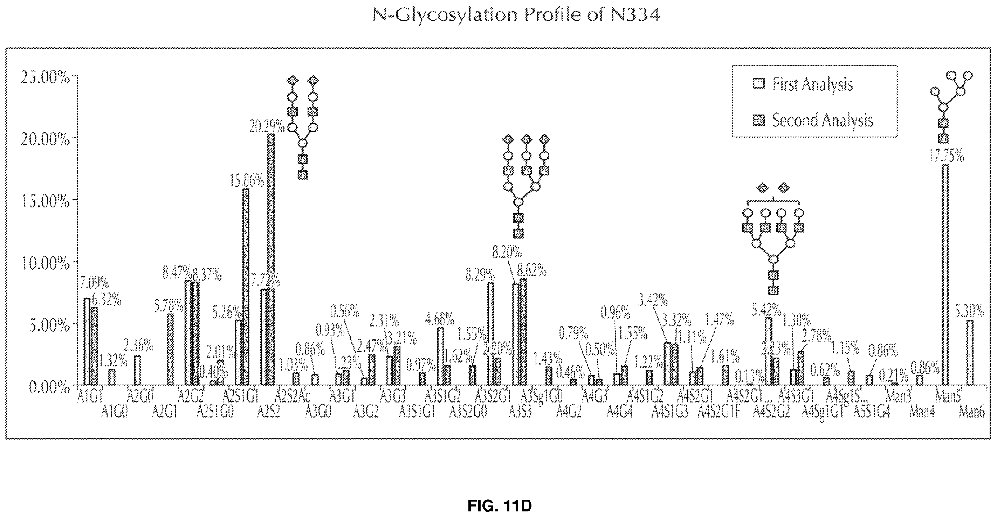

Figure 11D shows the N-glycosylation profile of the third site, N334. As can be seen from Figure 11D, the major glycan species are non-phosphorylated high mannose glycans, di-, tri-, and tetra-antennary complex glycans, and hybrid glycans. Both the first and second analyses detected over 20% of the ATB200 had a sialic acid residue at the third site.

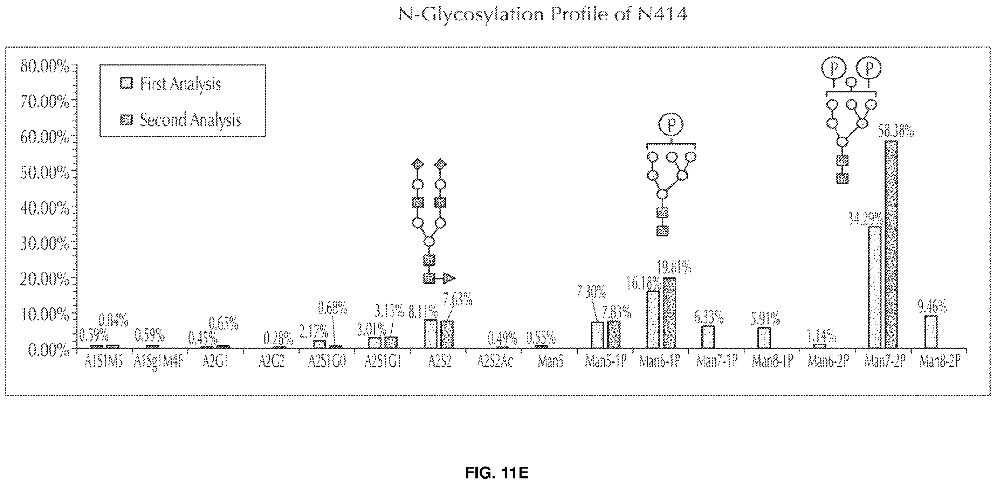

Figure 11E shows the N-glycosylation profile of the fourth site, N414. As can be seen from Figure 11E, the major glycan species are bis-M6P and mono-MGP glycans. Both the first and second analyses detected over 40% of the ATB200 had a bis-M6P glycan at the fourth site. Both the first and second analyses also detected over 25% of the ATB200 had a mono-M6P glycan at the fourth site.

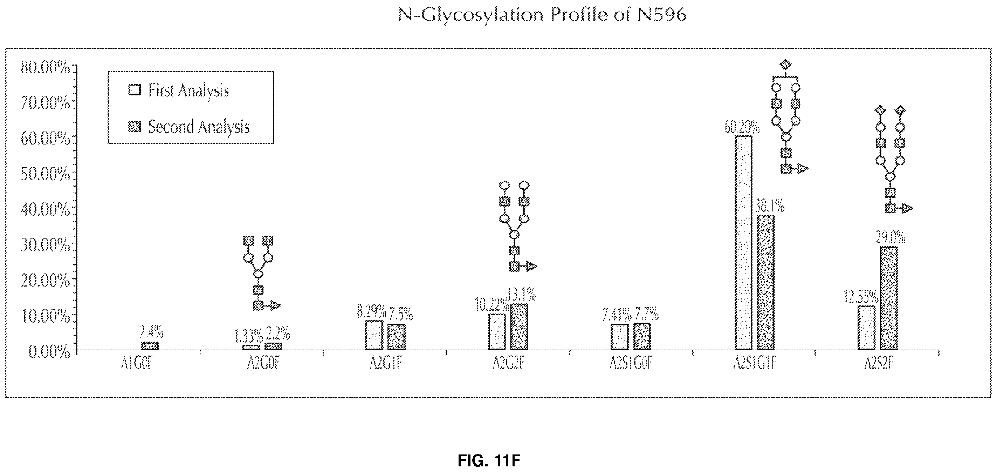

Figure 11F shows the N-glycosylation profile of the fifth site, N596. As can be seen from Figure 11F, the major glycan species are fucosylated di-antennary complex glycans. Both the first and second analyses detected over 70% of the ATB200 had a sialic acid residue at the fifth site.

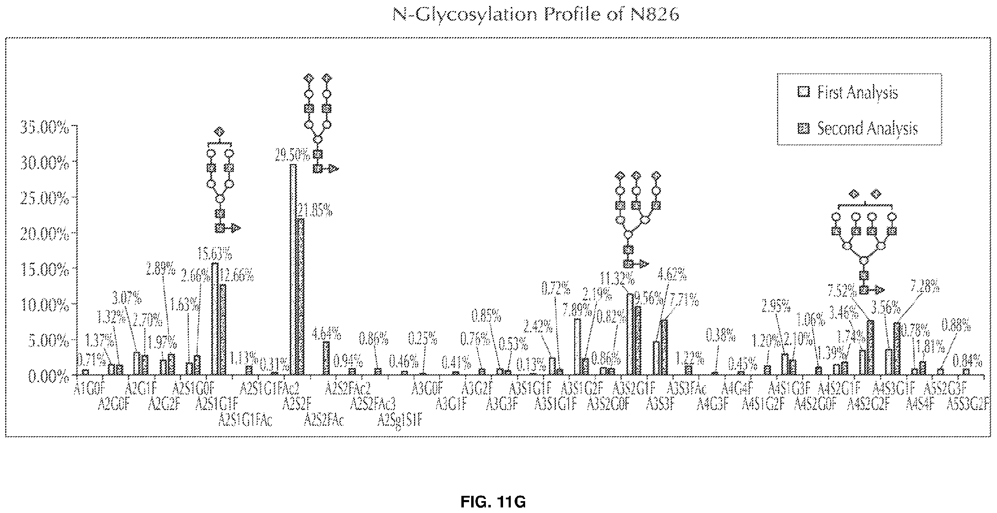

Figure 11G shows the N-glycosylation profile of the sixth site, N826. As can be seen from Figure 11G, the major glycan species are di-, tri-, and tetra-antennary complex glycans. Both the first and second analyses detected over 80% of the ATB200 had a sialic acid residue at the sixth site.

An analysis of the glycosylation at the seventh site, N869, showed approximately 40% glycosylation, with the most common glycans being A4S3S3GF (12%), A5S3G2F (10%), A4S2G2F (8%) and A6S3G3F (8%).

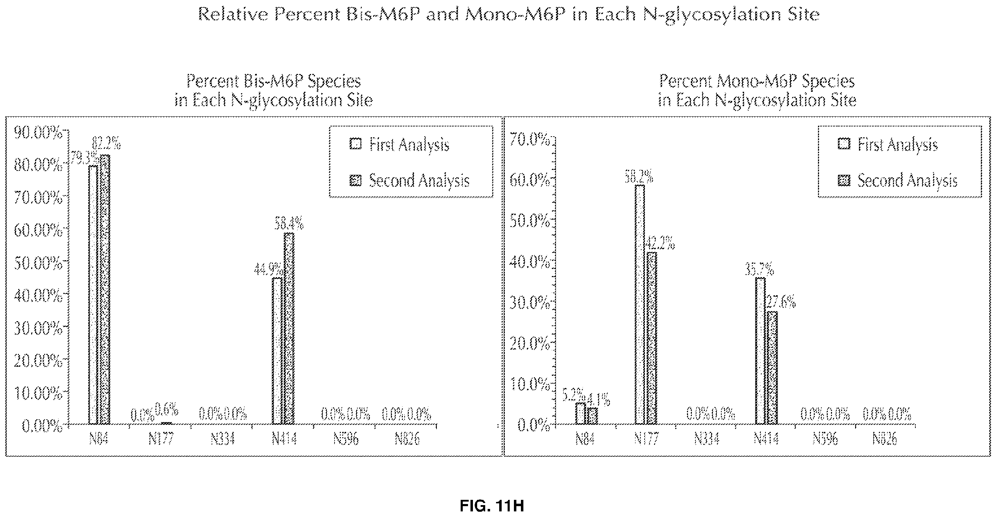

Figure 11H shows a summary of the phosphorylation at each of the seven potential N-glycosylation sites. As can be seen from Figure 11H, both the first and second analyses detected high phosphorylation levels at the first, second and fourth sites. Both analyses detected over 80% of the ATB200 was mono- or di-phosphorylated at the first site, over 40% of the ATB200 was mon-phosphorylated at the second site, and over 80% of the ATB200 was mono- or di-phosphorylated at the fourth site.

Another glycan analysis of ATB200 was performed according to a hydrophilic interaction liquid chromatography-fluorescent detection-mass spectrometery (HILIC-FLD-MS) method.

The results of HILIC-FLD-MS analysis are provided in Table 5 below: In Table 5, the first number in the three-digit number indicates the number of branches in the glycan, the second number indicates the number of core fucose units and the third number indicates the number of terminal sialic acid units. Using this nomenclature, "303" represents a tri-antennary glycan (the first 3) with 0 core fucose (the 2nd 0) and 3 terminal sialic acids (the last 3), "212" represents a bi-antennary glycan with 1 core fucose and 2 terminal sialic acids, "404" represents a tetra-antennary glycan with 0 core fucose and 4 terminal sialic acids, etc.

**Table 5**

| **FLD Peak Number** | **MS Peak Number** | **RT (min)** | **Glycan Structure** | **% Peak Area** |
|---|---|---|---|---|
| **1** | 1 | | BisP_Man 8 | 2.83% |
| **2** | 2 | 13.41 | BisP_Man 7 | 17.58% |
| | 3 | 14.30 | BisP_Man 6 | 1.02% |
| **3** | 4 | 20.89 | MonoP_Man 6 | 2.34% |
| **4** | 5 | 21.65 | MonoP_Man 5 | 1.16% |
| **5** | 6 | 23.51 | MonoP_Man 8 | 1.28% |
| **6** | 7 | 24.33 | MonoP_Man 7 | 4.35% |
| **7** | 8 | 25.61 | MonoP_Man 7 _(+)GlcNAc | 0.50% |
| **8** | 9 | 28.76 | MonoP_hMan6_101 | 0.48% |
| **9** | 10 | 30.54 | MonoP_Man 6_(+)GlcNAc | 0.68% |
| **10** | 11 | 33.50 | Man 6 | 3.97% |
| | 12 | 33.65 | 303 | 0.74% |
| **11** | 13 | 34.97 | Man 7 | 0.20% |
| **12** | 14 | 35.64 | 403 | 0.39% |
| **13** | 15 | 36.61 | 302 | 0.36% |
| **14** | 16 | 38.07 | 302 | 0.61% |
| **15** | 17 | 38.53 | Man 5 | 1.85% |
| **16** | 18 | 39.57 | 302 | 0.48% |
| | 19 | 39.78 | hMan 5_101 | 0.42% |
| | 20 | 40.05 | hMan 5_100_(-)Gal | 0.30% |
| **17** | 21 | 40.77 | 301_(-)Gal | 0.52% |
| | 22 | 40.58 | 301 | 0.50% |
| **18** | 23 | 41.47 | 300_(-)Gal | 0.80% |
| **19** | 24 | 42.17 | 301_(-)Gal | 0.11% |
| | 25 | 42.13 | 301 | 0.58% |
| **20** | 26 | 42.89 | 301_(-)Gal | 0.07% |
| | 27 | 42.79 | 301 | 0.80% |
| **21** | 28 | 43.41 | 300 | 0.85% |
| | 29 | 43.28 | 101 | 0.39% |
| **22** | 30 | 43.94 | 202 | 0.63% |
| **23** | 31 | 44.45 | 401 | 0.39% |
| **24** | 32 | 45.04 | MonoP_hMan6_111 | 0.36% |
| **25** | 33 | 45.69 | MonoP_hMan6_111 | 1.45% |
| | 34 | 45.90 | 100 | 0.23% |
| | 35 | 45.90 | 400 | 0.19% |
| **26** | 36 | 46.87 | 201 | 0.49% |
| | 37 | 47.15 | 202 | 0.34% |
| **27** | 38 | 48.19 | 414 | 0.37% |
| **28** | 39 | 48.94 | 202 | 1.97% |
| **29** | 40 | 50.79 | MonoP_Man 6 _110_(-)Gal | 1.31% |
| | 41 | 51.37 | 414 | 0.62% |
| **30** | 42 | 52.22 | 313 | 0.74% |
| | 43 | 52.42 | 201_(-)Gal | 0.46% |
| | 44 | 52.42 | 201 | 1.18% |
| | 45 | 53.11 | hMan6_111 | 0.20% |
| **31** | 46 | 53.83 | 200_(-)Gal | 0.80% |
| | 47 | 54.23 | 201 | 1.27% |
| | 48 | 54.75 | 413 | 0.30% |
| **32** | 49 | 55.47 | 200 | 1.30% |
| **33** | 50 | 57.45,58.34 | 414_(+)GlcNAcGal | 0.14% |
| | 51 | 56.62,56.91,57.99 | 413 | 0.94% |
| | 52 | 56.11,57.26,57.99 | 312 | 0.98% |
| **34** | 53 | 60.19 | 413 | 0.33% |
| | 54 | 59.39 | 413_(+)GlcNAcGal | 0.42% |
| | 55 | 59.80 | 312 | 0.52% |
| | 56 | 59.49 | 412 | 0.18% |
| **35** | 57 | 60.75 | 413 | 0.78% |
| | 58 | 60.89 | 413_(+)GlcNAcGal | 0.07% |
| **36** | 59 | 61.79 | 413 | 0.20% |
| | 60 | 61.75 | 312 | 0.16% |
| | 61 | 62.12 | 412 | 0.64% |
| **37** | 62 | 63.87 | 311 | 0.73% |
| | 63 | 63.18,64.32 | 412 | 0.29% |
| | 64 | 63.84 | 413_(+)GlcNAcGal | 0.45% |
| | 65 | 63.5, 64.36 | 311_(-)Gal | 0.42% |
| **38** | 66 | 65.73, 66.20 | 311 | 0.68% |
| | 67 | 65.85, 66.49 | 412 | 0.72% |
| | 68 | 65.91 | 310_(-)Gal | 0.28% |
| **39** | 69 | 67.37 | 212 | 1.42% |
| | 70 | 67.57 | 310 | 0.34% |
| **40** | 71 | 68.67 | 412_(+)GlcNAcGal | 0.24% |
| | 72 | 68.36 | 412 | 0.53% |
| **41** | 73 | 68.36 | 412_(+)GlcNAcGal | 0.17% |
| | 74 | 69.03 | 412 | 0.35% |
| | 75 | 69.30 | 413_(+)2(GlcNAcGal) | 0.16% |
| **42** | 76 | 70.66 | 412_(+)GlcNAcGal | 0.73% |
| **43** | 77 | 71.74 | 211 | 1.09% |
| | 78 | 71.23 | 211_(-)Gal | 0.19% |
| **44** | 79 | 72.46 | 212 | 3.66% |
| **45** | 80 | 74.82 | 221_(-)Gal(+)GalNAc | 0.38% |
| | 81 | 74.43,74.96 | 411_(+)GlcNAcGal | 0.66% |
| **46** | 82 | 75.92 | 410 | 0.42% |
| **47** | 83 | 76.73,77.87 | 211_(-)Gal | 1.24% |
| | 84 | 77.23 | 211 | 3.64% |
| **48** | 85 | 79.05 | 211 | 1.52% |
| | 86 | 79.38 | 210_(-)2Gal | 0.45% |
| **49** | 87 | 80.11 | 210_(-)Gal | 1.58% |
| **50** | 88 | 81.15 | 210 | 2.41% |
| **51** | 89 | 84.22-87.15 | 311 | 1.26% |
| **52** | 90 | 95.35 | Mono_Acetyl_NANA_212 | 0.99% |
| **53** | 91 | 96.23 | Mono_Acetyl_NANA_211 | 0.76% |
| **54** | 92 | 97.37 | Bis_Acetyl_NANA_212 | 0.42% |

Based on this HILIC-FLD-MS analysis, the ATB200 tested is expected to have an average fucose content of 2-3 mol per mol of ATB200, GlcNAc content of 20-25 mol per mol of ATB200, galactose content of 8-12 mol per mol of ATB200, mannose content of 22-27 mol per mol of ATB200, M6P content of 3-5 mol per mol of ATB200 and sialic acid content of 4-7 mol of ATB200.

### Example 6: Characterization of CIMPR Affinity of ATB200

In addition to having a greater percentage of rhGAA that can bind to the CIMPR, it is important to understand the quality of that interaction. Lumizyme^{®} and ATB200 rhGAA receptor binding was determined using a CIMPR plate binding assay. Briefly, CIMPR-coated plates were used to capture GAA. Varying concentrations of rhGAA were applied to the immobilized receptor and unbound rhGAA was washed off. The amount of remaining rhGAA was determined by GAA activity. As shown by Figure 12A, ATB200 rhGAA bound to CIMPR significantly better than Lumizyme^{®}.

Figure 12B shows the relative content of bis-M6P glycans in Lumizyme^{®}, a conventional rhGAA, and ATB200 according to the invention. For Lumizyme^{®} there is on average only 10% of molecules have a bis-phosphorylated glycan. Contrast this with ATB200 where on average every rhGAA molecule has at least one bis-phosphorylated glycan.

### Example 7: ATB200 rhGAA was more efficiently internalized by fibroblast than Lumizyme

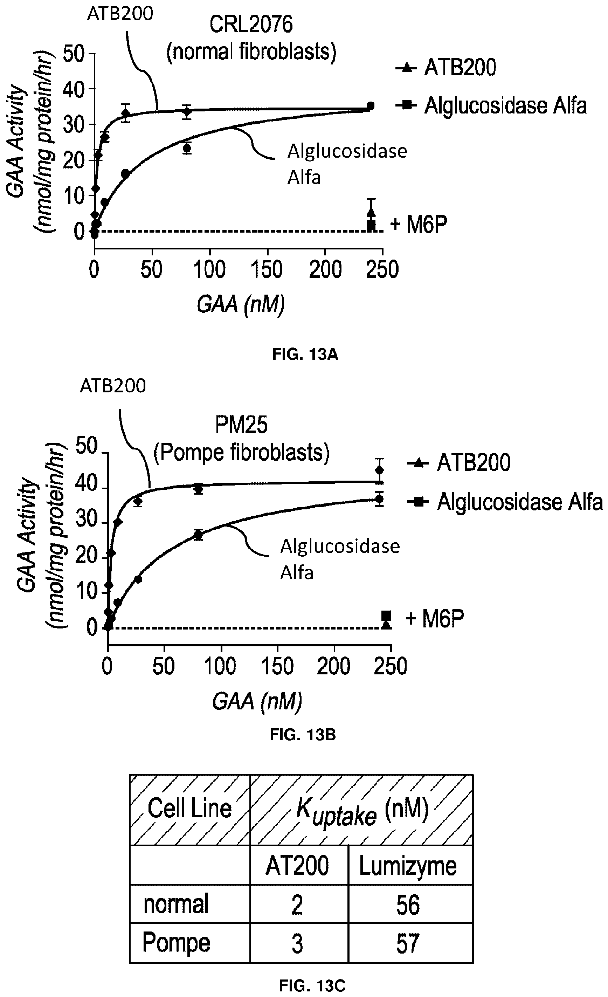

The relative cellular uptake of ATB200 and Lumizyme^{®} rhGAA were compared using normal and Pompe fibroblast cell lines. Comparisons involved 5-100 nM of ATB200 rhGAA according to the invention with 10-500 nM conventional rhGAA Lumizyme^{®}. After 16-hr incubation, external rhGAA was inactivated with TRIS base and cells were washed 3-times with PBS prior to harvest. Internalized GAA measured by 4MU-α-Glucoside hydrolysis and was graphed relative to total cellular protein and the results appear in Figures 13A-B.

ATB200 rhGAA was also shown to be efficiently internalized into cells (Figure 13A and 13B), respectively, show that ATB200 rhGAA is internalized into both normal and Pompe fibroblast cells and that it is internalized to a greater degree than conventional Lumizyme^{®} rhGAA. ATB200 rhGAA saturates cellular receptors at about 20 nM, while about 250 nM of Lumizyme^{®} is needed. The uptake efficiency constant (Kᵤₚₜₐₖₑ) extrapolated from these results is 2-3 nm for ATB200 and 56 nM for Lumizyme^{®} as shown by Figure 13C. These results suggest that ATB200 rhGAA is a well-targeted treatment for Pompe disease.

### Example 8: Glycogen reduction in Gaa-knockout mice

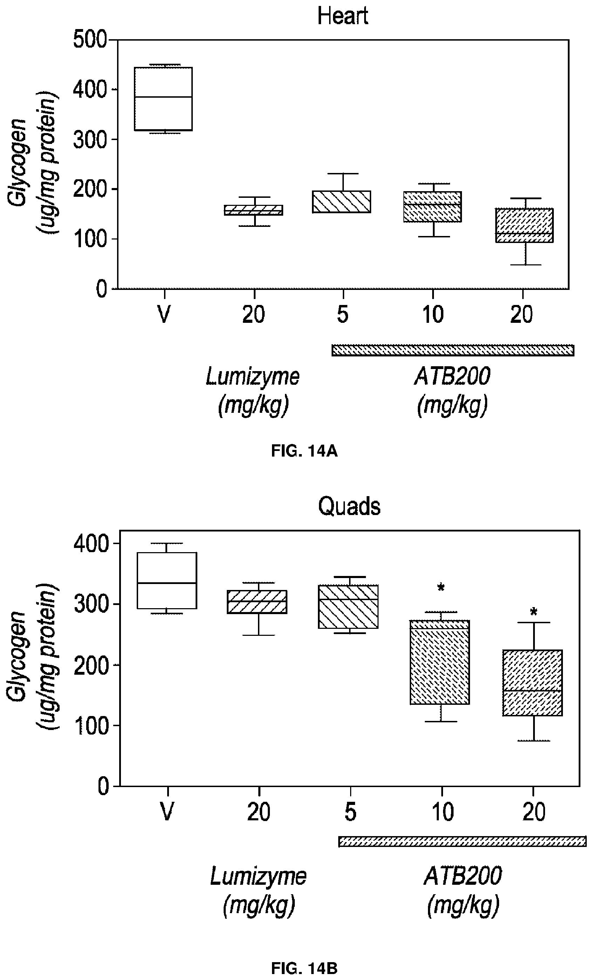

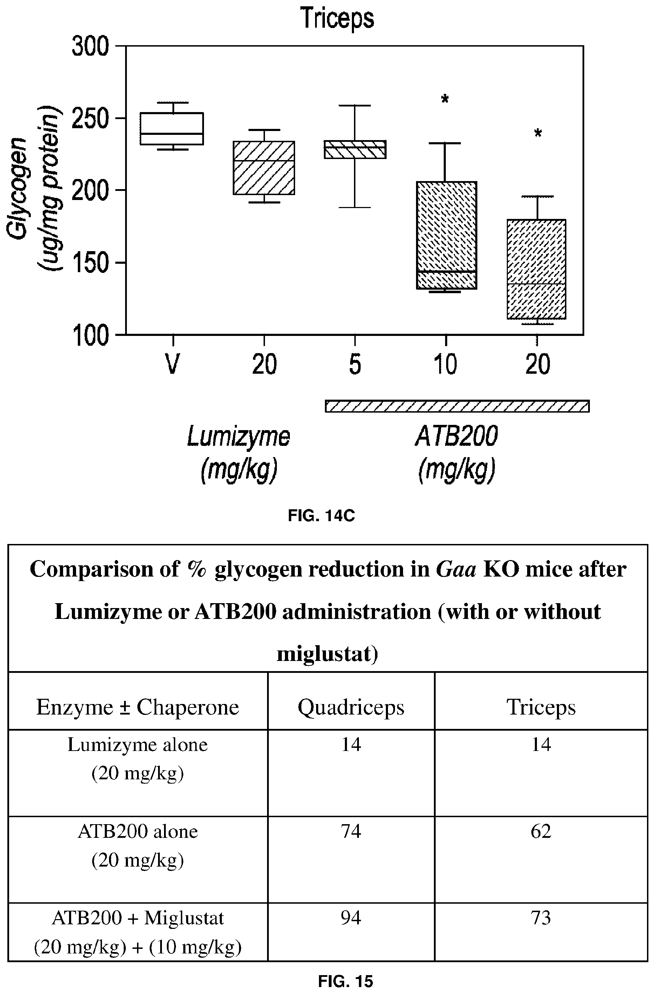

Figures 14A to 14C show the effects of administering alglucosidase alfa (Lumizyme^{®}) and ATB200 on glycogen clearance in *Gaa* knockout mice. Animals were given two IV bolus administrations (every other week); tissues were harvested two weeks after the last dose and analyzed for acid α-glucosidase activity and glycogen content.

As seen from Figures 14A to 14C, ATB200 was found to deplete tissue glycogen in acid α-glucosidase (*Gaa*) knockout mice in a dose-dependent fashion. The 20 mg/kg dose of ATB200 consistently removed a greater proportion of stored glycogen in *Gaa* knockout mice than the 5 and 10 mg/kg dose levels. However, as seen in Figures 14A to 14C, ATB200 administered at 5 mg/kg showed a similar reduction of glycogen in mouse heart and skeletal muscles (quadriceps and triceps) to Lumizyme^{®} administered at 20 mg/kg, while ATB200 dosed at 10 and 20 mg/kg showed significantly better reduction of glycogen levels in skeletal muscles than Lumizyme^{®}.

Figure 15 shows the effects of administering alglucosidase alfa (Lumizyme^{®}) and ATB200 on glycogen clearance in *Gaa* knockout mice, as well as the effect of co-administration of ATB200 and miglustat on glycogen clearance. Twelve week *old* GAA KO mice treated with Lumizyme^{®} or ATB200, 20 mg/kg IV every other week 4 injections; miglustat was co-administered at 10 mg/kg PO, 30 min prior to rhGAA as indicated. Tissues were collected 14 days after last enzyme dose for glycogen measurement. Figure 15 shows the relative reduction of glycogen in quadriceps and triceps skeletal muscle, with ATB200 providing a greater reduction of glycogen than Lumizyme^{®}, and ATB200/miglustat providing an even greater reduction of glycogen.

### Example 9: Muscle physiology and morphology in Gaa-knockout mice

Gaa knockout mice were given two IV bolus administrations of recombinant human acid α-glucosidase (alglucosidase alfa or ATB200) at 20 mg/kg every other week. Miglustat was orally administered at dosages of 10 mg/kg to a subset of animals treated with ATB200 30 mins prior to administration of ATB200. Control mice were treated with vehicle alone. Soleus, quadriceps and diaphragm tissue is harvested two weeks after the last dose of recombinant human acid α-glucosidase. Soleus and diaphragm tissue were analyzed for glycogen levels, by staining with periodic acid - Schiff reagent (PAS), and for lysosome proliferation, by measuring levels of the lysosome-associated membrane protein (LAMP1) marker, which is upregulated in Pompe disease. Semi-thin sections of quadriceps muscle embedded in epoxy resin (Epon) were stained with methylene blue and observed by electron microscopy (1000x) to determine the extent of the presence of vacuoles. Quadriceps muscle samples were analyzed immunohistochemically to determine levels of the autophagy markers microtubule-associated protein 1A/1B-light chain 3 phosphatidylethanolamine conjugate (LC3A II) and p62, the insulin-dependent glucose transporter GLUT4 and the insulin-independent glucose transporter GLUT1.

In a similar study, Gaa knockout mice were given four IV bolus administrations of recombinant human acid α-glucosidase (alglucosidase alfa or ATB200) at 20 mg/kg every other week. Miglustat was orally administered at dosages of 10 mg/kg to a subset of animals treated with ATB200 30 mins prior to administration of ATB200. Control mice were treated with vehicle alone. Cardiac muscle tissue was harvested two weeks after the last dose of recombinant human acid α-glucosidase and analyzed for glycogen levels, by staining with periodic acid - Schiff reagent (PAS), and for lysosome proliferation, by measuring levels of LAMP1.

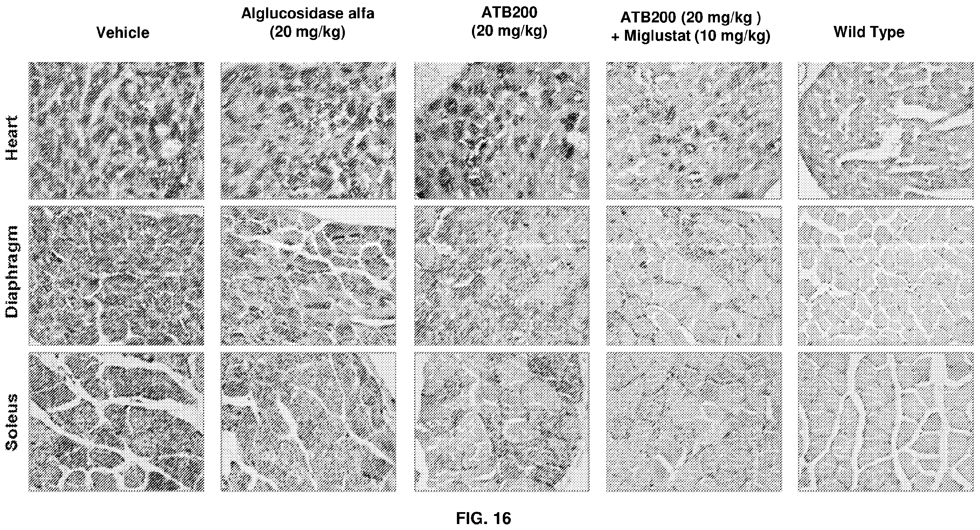

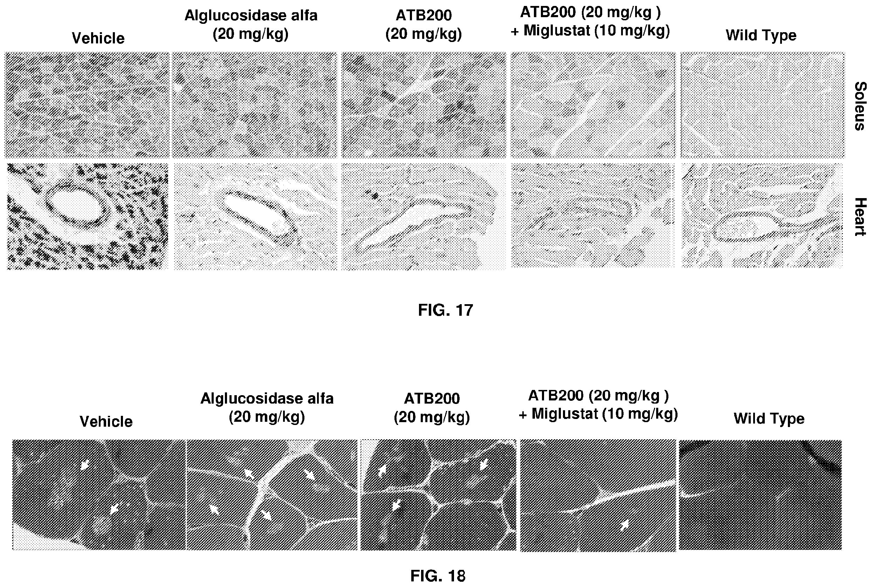

As seen in Figure 16, administration of ATB200 showed a reduction in lysosome proliferation in heart, diaphragm and skeletal muscle (soleus) tissue compared to conventional treatment with alglucosidase alfa, and co-administration of miglustat with ATB200 showed a significant further reduction in lysosomal proliferation, approaching the levels seen in wild type (WT) mice. In addition, as seen in Figure 17, administration of ATB200 showed a reduction in punctate glycogen levels in heart and skeletal muscle (soleus) tissue compared to conventional treatment with alglucosidase alfa, and co-administration of miglustat with ATB200 showed a significant further reduction, again approaching the levels seen in wild type (WT) mice.

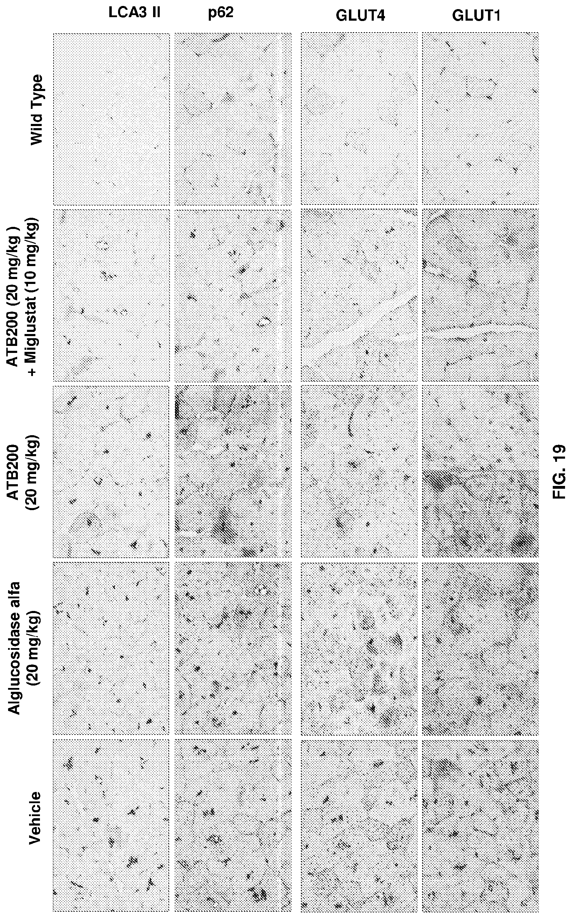

As well, as seen in Figure 18, co-administration of miglustat with ATB200 significantly reduced the number of vacuoles in muscle fiber in the quadriceps of Gaa knockout mice compared to untreated mice and mice treated with alglucosidase alfa. As seen in Figure 19, levels of both LC3 II and p62 are increased in Gaa knockout mice compared to wild type mice, but are reduced significantly upon treatment with ATB200 and miglustat, indicating that the increase in autophagy associated with acid α-glucosidase deficiency is reduced upon co-administration of ATB200 and miglustat. In addition, levels of the insulin-dependent glucose transporter GLUT4 and the insulin-independent glucose transporter GLUT1 are increased in Gaa knockout mice compared to wild type mice, but again, are reduced significantly upon treatment with ATB200 and miglustat. The elevated GLUT4 and GLUT1 levels associated with acid α-glucosidase deficiency can contribute to increased glucose uptake into muscle fibers and increased glycogen synthesis both basally and after food intake. Thus, combination treatment with ATB200 and miglustat has been found to improve skeletal muscle morphology and physiology in a mouse model of Pompe disease.

### Example 10: Muscle function in Gaa-knockout mice

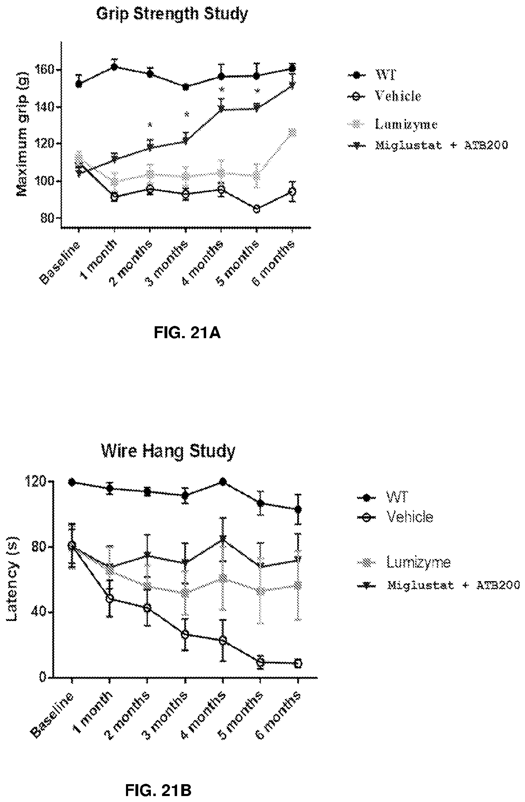

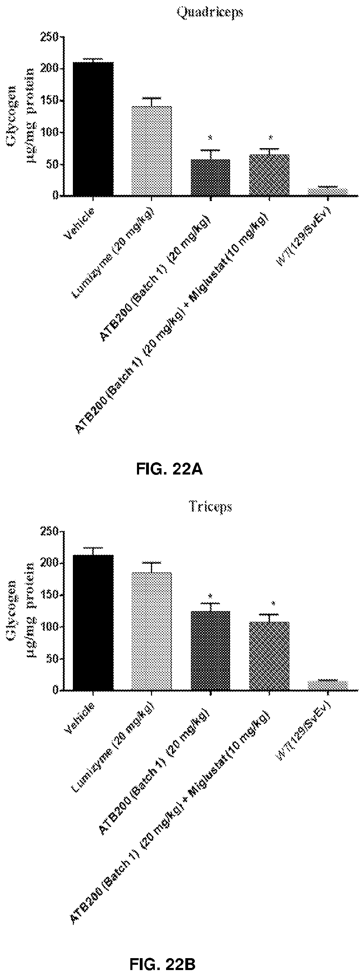

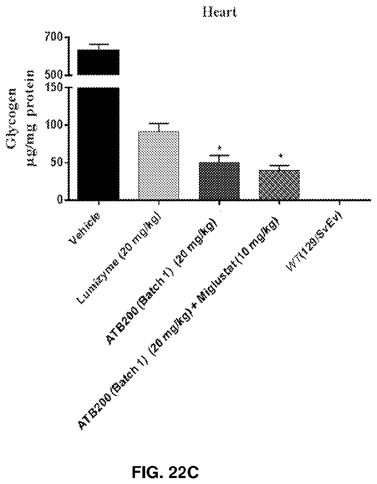

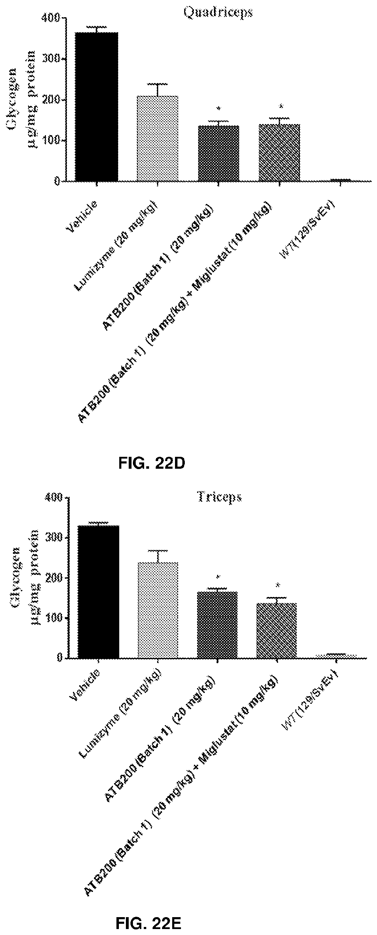

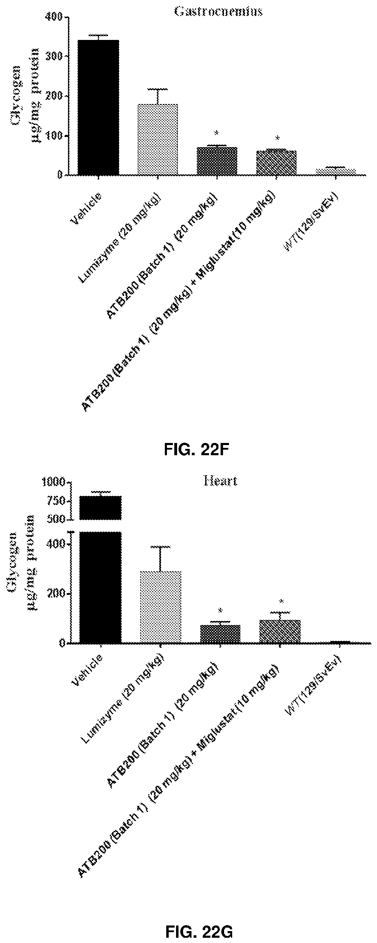

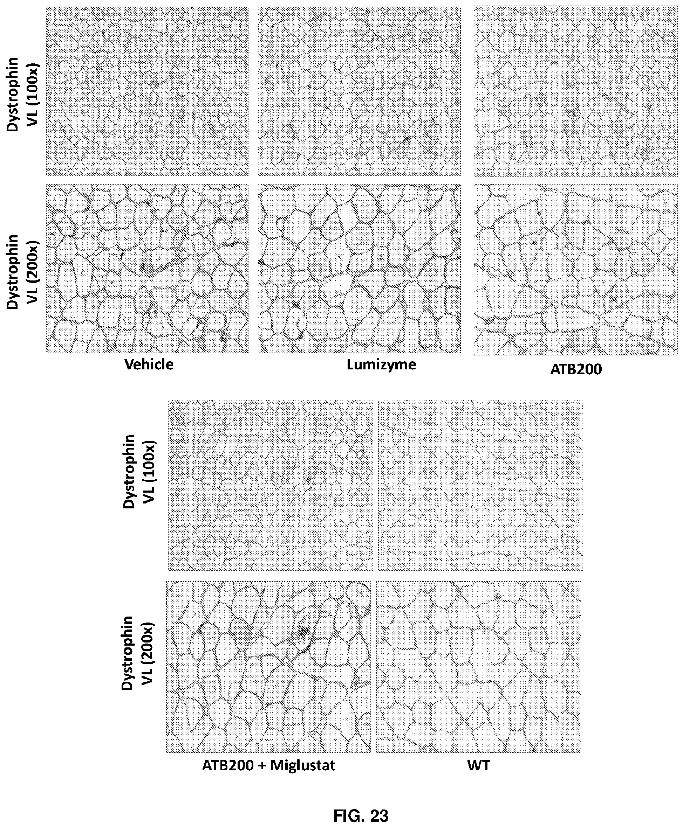

In longer-term studies of 12 biweekly administrations, 20 mg/kg ATB200 plus 10 mg/kg miglustat progressively increased functional muscle strength in *Gaa* KO mice from baseline as measured by both grip strength and wire hang tests (Figures 21A-21B). Alglucosidase alfa (Lumizyme^{®})-treated mice receiving the same ERT dose (20 mg/kg) were observed to decline under identical conditions throughout most of the study (Figures 21A-21B). As with the shorter-term study, ATB200/miglustat had substantially better glycogen clearance after 3 months (Figures 22A-22C) and 6 months (Figures 22D-22G) of treatment than alglucosidase alfa. ATB200/miglustat also reduced autophagy and intracellular accumulation of LAMP1 and dysferlin after 3 months of treatment (Figure 23) compared to alglucosidase alfa. In Figure 21A, * indicates statistically significant compared to Lumizyme^{®} alone (p<0.05, 2-sided t-test). In Figures 22A-22G, * indicates statistically significant compared to Lumizyme^{®} alone (p<0.05, multiple comparison using Dunnett's method under one-way ANOVA analysis).

Taken together, these data indicate that ATB200/miglustat was efficiently targeted to muscles to reverse cellular dysfunction and improve muscle function. Importantly, the apparent improvements in muscle architecture and reduced autophagy and intracellular accumulation of LAMP1 and dysferlin may be good surrogates for improved muscle physiology that correlate with improvements in functional muscle strength. These results suggest that monitoring autophagy and these key muscle proteins may be a rational, practical method to assess the effectiveness therapeutic treatments for Pompe disease in *Gaa* KO mice that may prove to be useful biomarkers from muscle biopsies in clinical studies.

Figure 23 shows that 6 months of ATB200 administration with or without miglustat lowered intracellular accumulation of dystrophin in *Gaa* KO mice. There was a greater reduction for dystrophin accumulation for ATB200 ± miglustat than with Lumizyme^{®}.

### Example 11: Capturing of rhα-Gal A

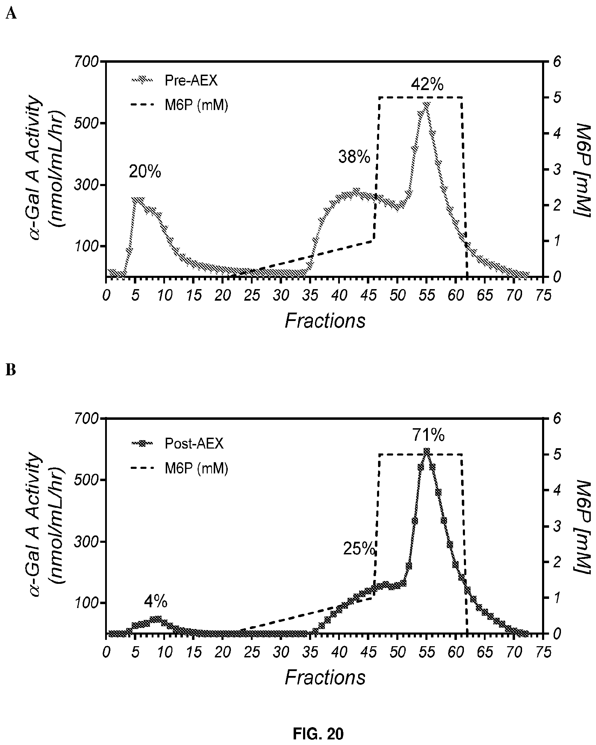

The CIMPR binding profile of recombinant human α-galactosidase A (rhα-Gal A) in spent cell culture medium was measured before product capture using AEX chromatography (Figure 20A) and after product capture using AEX chromatography (Figure 20B). The dashed line in both graphs refers to the M6P elution gradient. Prior to AEX product capture, 80% of the rhα-Gal A is able to bind to the CIMPR. After AEX product capture, the total rhα-Gal A bound increases to 96%.

### Example 12: Pharmacokinetic and Safety Data on Recombinant Acid α-Glucosidase ATB200 Co-administered With Migluststat in ERT-Experienced and ERT-Naive Patients With Pompe Disease

This study was designed to primarily evaluate the safety, tolerability, and pharmacokinetics (PK) of ATB200 co-administered with miglustat. A PK/pharmacodynamic (PD) translational model from Gaa knockout mouse predicted that a combination of ATB200 20 mg/kg with a high dose (e.g. 260 mg) of miglustat in humans would provide optimal glycogen reduction.

In the description below, "high dose" of miglustat refers to a dose of about 260 mg and "low dose" of miglustat refers to a dose of about 130 mg.

The objective was to evaluate the preliminary total GAA protein, ATB200 and miglustat PK data, and safety markers from 10 patients in this of this phase 1/2 study.

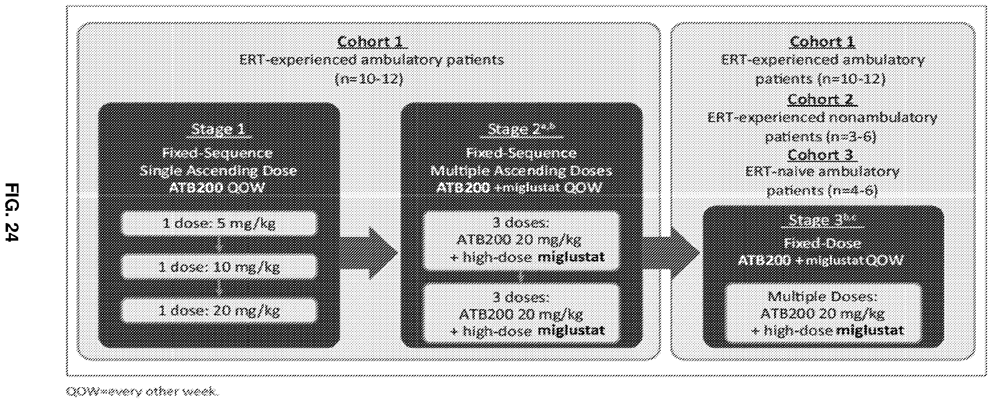

This is an open-label, fixed-sequence, ascending-dose, first-in-human, phase 1/2 study to assess the safety, tolerability, PK, PD, and efficacy of intravenous infusions of ATB200 co-administered with oral miglustat in adults with Pompe disease (Figure 24). Mean total GAA protein and miglustat PK results from the first 8 Cohort 1 patients through Visit 9 and the first 2 Cohort 3 patients were assessed.
^{a}Safety data from 2 sentinel patients from Cohort 1 were reviewed at each dose level before dosing in Cohorts 2 and 3.
^{b}During Stages 2 and 3, miglustat was orally administered prior to the start of ATB200 intravenous infusion. For all doses, ATB200 was intravenously infused for a 4-hour duration.
^{c}The first 2 patients in Cohorts 2 and 3 served as sentinel patients for their respective cohorts.

### Key inclusion criteria:

- Males and females aged 18-65 years who were diagnosed with Pompe disease based on documented deficiency of GAA enzyme activity or by GAA genotyping
   - Received ERT with alglucosidase alfa for 2-6 years (or ≥2 years for Cohort 2) prior to trial initiation (Cohort 1)
   - Currently receiving alglucosidase alfa at a frequency of every other week and completed the last 2 infusions without a drug-related adverse event resulting in dose interruption (Cohorts 1 and 2)
   - Must be able to walk between 200 and 500 meters on the 6-Minute Walk Test (Cohorts 1 and 3)
   - Upright forced vital capacity must be 30%-80% of predicted normal value (Cohorts 1 and 3)
   - Must be wheelchair-bound and unable to walk unassisted (Cohort 2)

### PK Analysis:

- Blood samples for plasma total GAA protein and activity concentration were collected
- Stage 1: prior to start of ATB200 infusion and 1, 2, 3, 3.5, 4, 4.5, 5, 6, 8, 10, 12, and 24 hour(s) post-start of infusion
- Stages 2 and 3: 1, 2, 3, 4, 4.5, 5, 6, 7, 9, 11, 13, and 25 hour(s) post-miglstat oral administration
- Blood samples for plasma miglustat concentrations were taken just prior to miglustat oral administration (time 0) and 1, 1.5, 2, 2.5, 3, 4, 5, 6, 9, 11, and 25 hour(s) after miglustat oral administration. Plasma miglustat is determined by a validated LC-MS/MS assay
- Total GAA protein concentrations in plasma for ATB200 5, 10, and 20 mg/kg were determined by a validated LC-MS/MS quantification of rhGAA-specific "signature" peptide(s)

A preliminary analysis was completed in 8 patients in Cohort 1 who completed Stages 1 and 2 and 2 patients in Cohort 3 who started Stage 3
- Initial ERT-switch patients are represent ative of the Pompe disease population, with mean 5.02 years on ERT (Table 6)

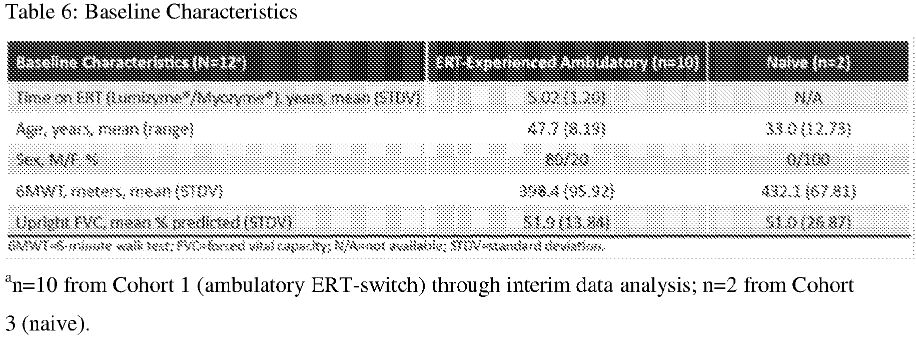

### Total GAA Protein

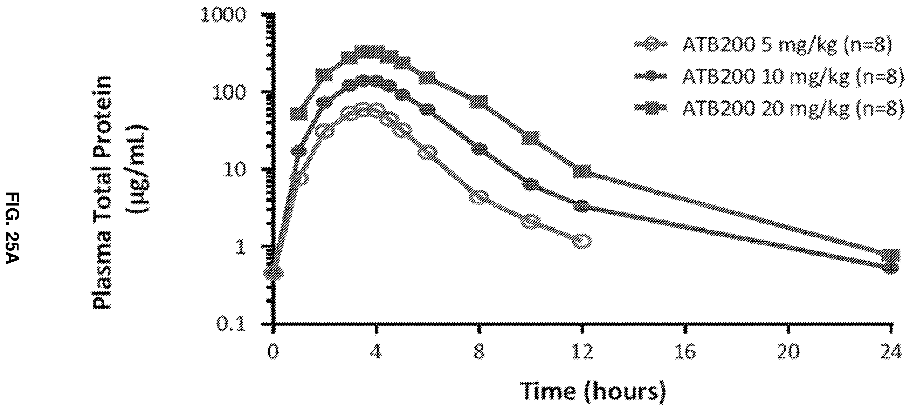

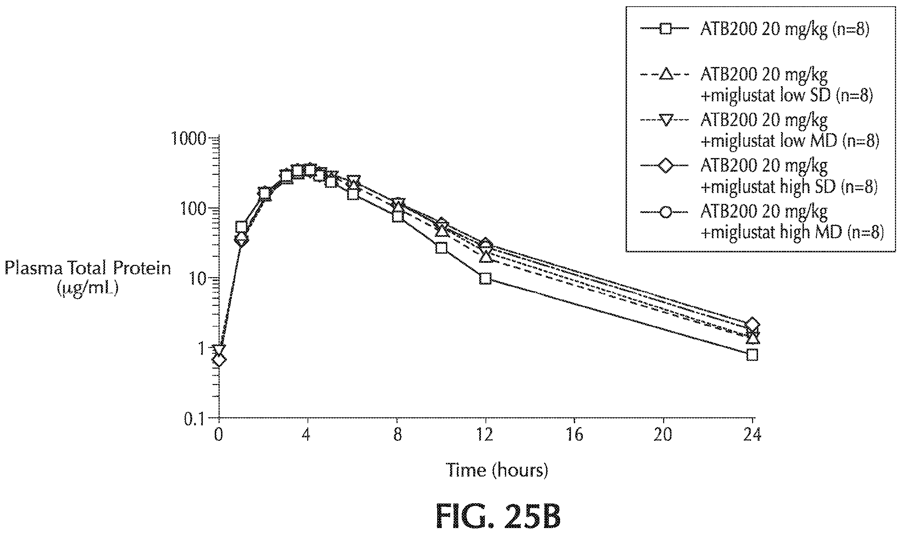

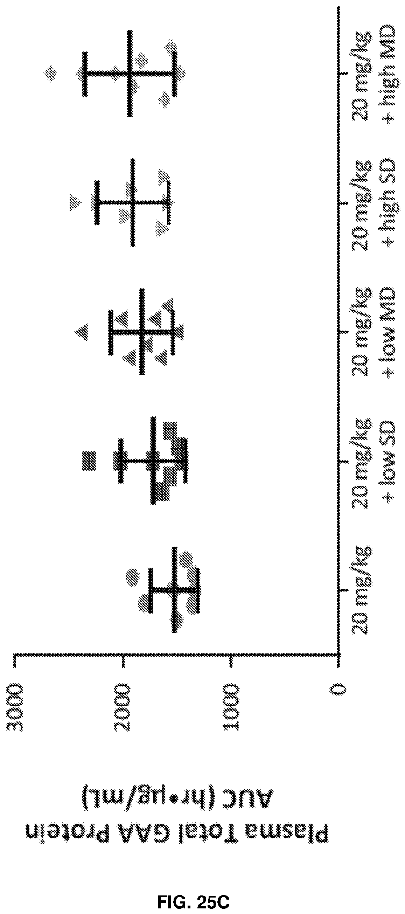

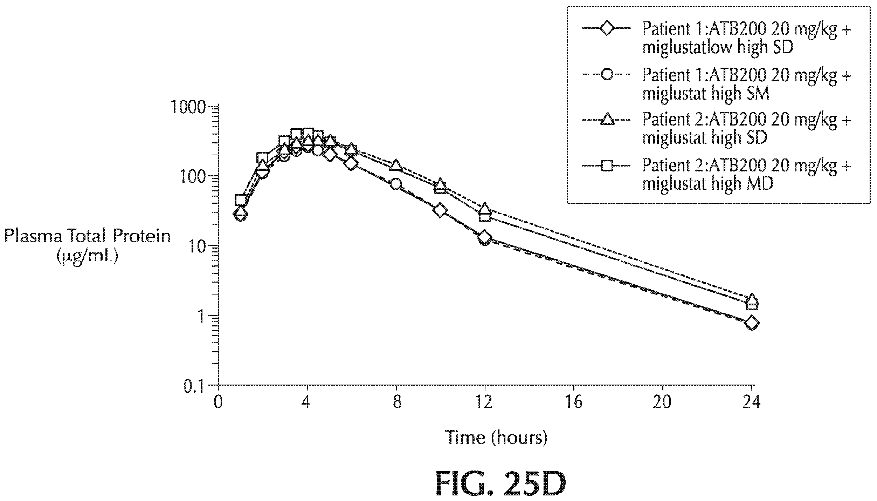

When given alone, ATB200 increases in a slightly greater-than-dose-proportional manner (Table 7 and Figures 25A-25D). Variability appears to increase with miglustat dose (Figure 25C). Co-administration of ATB200 20 mg/kg with the high dose of miglustat (260 mg) increased total GAA protein exposure (AUC) by approximately 25% relative to ATB200 alone at 20 mg/kg. The distribution half-life (α-phase) increased by 45%, suggesting that the high dose of miglustat stabilizes ATB200 in plasma. An increase in the distribution half-life is accompanied by an increase in AUC from time to maximum plasma concentration to approximately 12 hours post-dose. The increases in AUC and half-life can be observed on the log scale, during the terminal elimination phase (Figure 25B). ATB200 demonstrated a relatively high volume of distribution. The disposition of plasma total GAA protein appears similar between ERT-naive (Cohort 3) and ERT-experienced patients (Cohort 1) (Figures 25A and 25D).

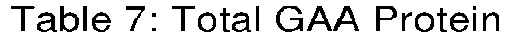

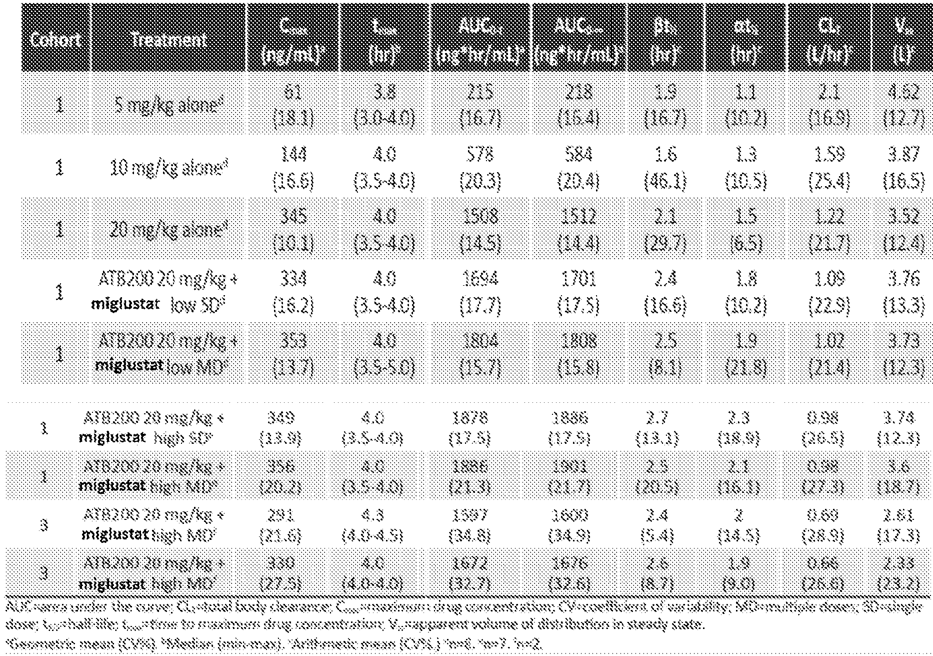

### Miglustat PK

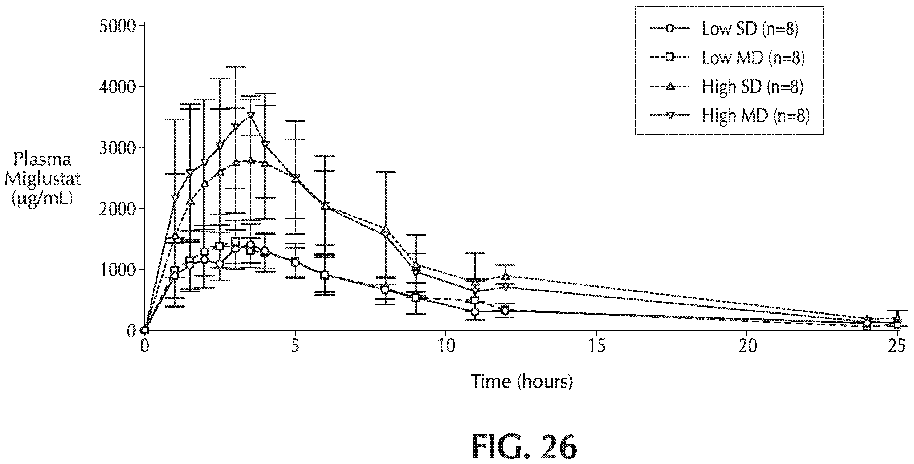

Miglustat demonstrated dose-proportional kinetics (Table 8 and Figure 26). Plasma miglustat appears similar between single and multiple doses.

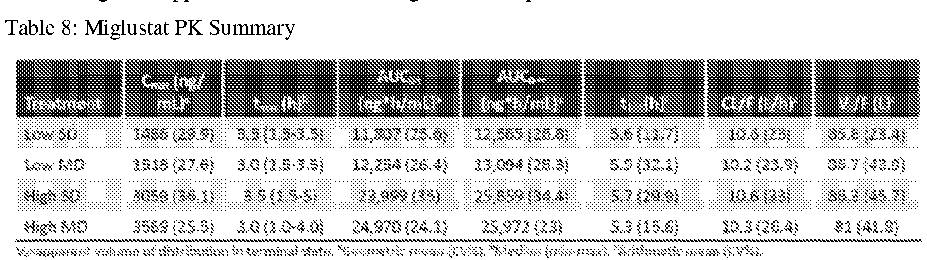

### Pharmacodynamics

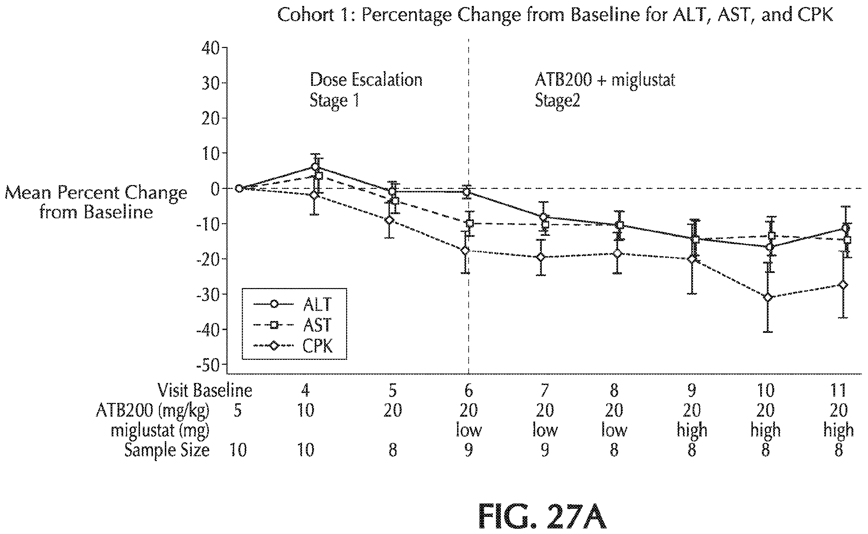

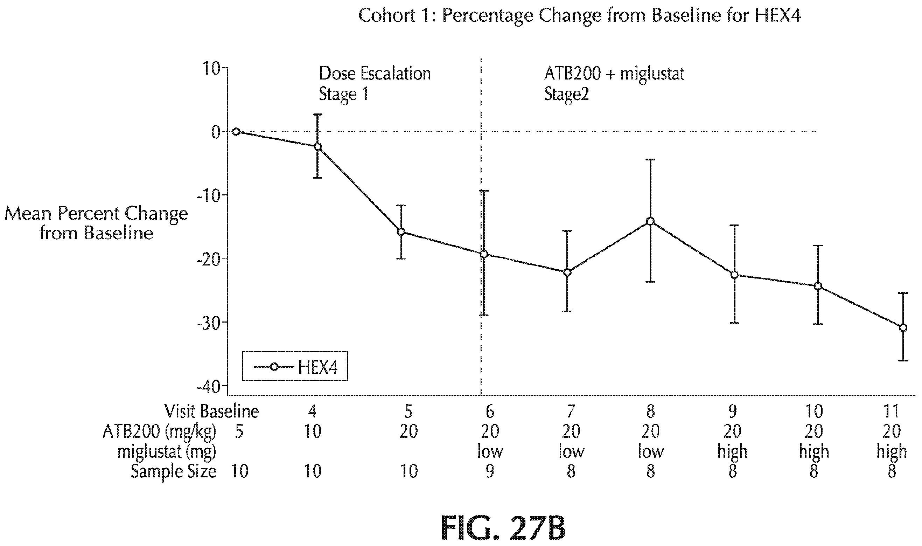

By the 11th visit in ERT-experienced patients from Cohort 1 (Figures 27A and 27B):
- Alanine aminotransferase (ALT) decreased in 5 of 8 patients; 4/4 patients with elevated baseline levels normalized
- Aspartate aminotransferase (AST) decreased in 6 of 8 patients; 3/4 patients with elevated baseline levels normalized
- Creatine phosphokinase (CPK) decreased in 6 of 8 patients; 2/6 patients with elevated baseline levels normalized
- Urine glucose tetrasaccharide (HEX4) levels decreased in 8 of 8 patients

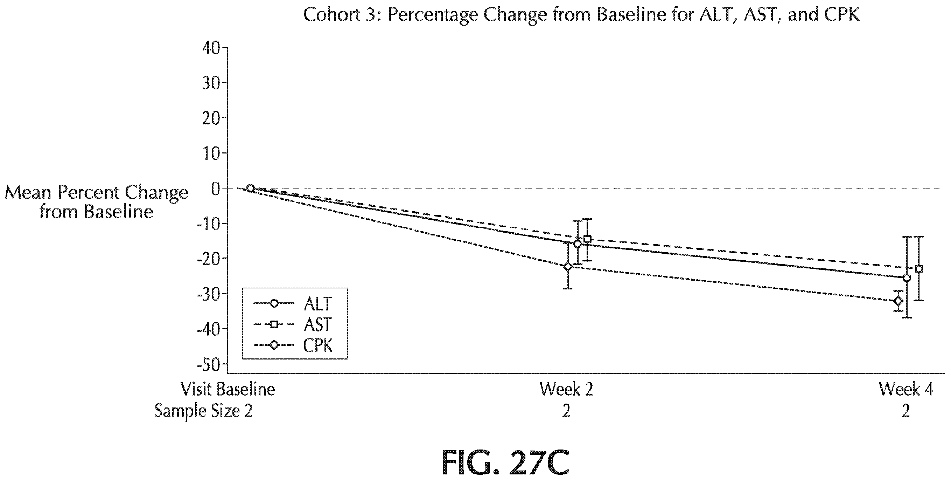

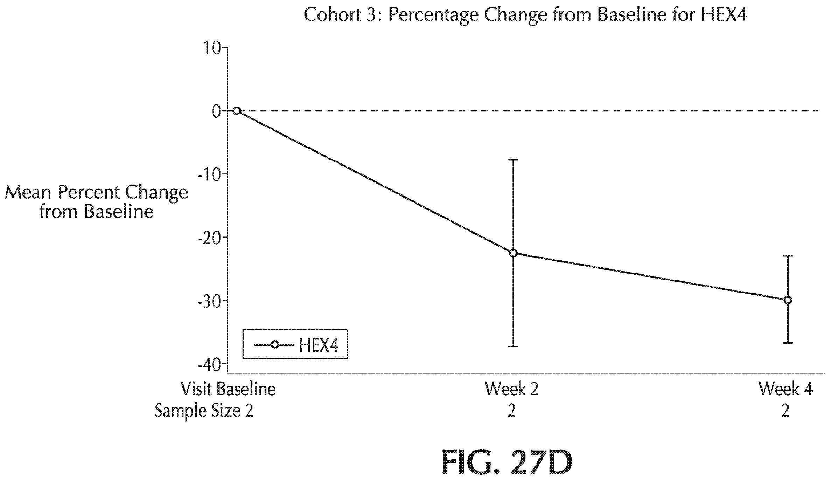

By week 4, all 4 biomarker levels decreased in the 2 patients in the treatment-naive cohort (Cohort 3) (Figures 27C and 27D).

In Figures 27A-27D, data are represented as mean ± standard error.

### Safety

- No serious adverse events (AEs) or infusion-associated reactions were reported after 155+ total infusions in all patients
- Treatment-emergent AEs, reported in 11/13 (84%) patients, were generally mild and transient.
- Treatment-related AEs reported in 7/13 (53%) patients: nausea (n=1), fatigue (n=1), headache (n=1), tremor (n=2), acne (n=1), tachycardia (n=1), and hypotension (n=1).

### Conclusions

- ATB200 alone and in combination with miglustat has been safe and well tolerated, with no infusion-associated reactions to date.
- ATB200 alone showed greater-than-dose-proportional increases in exposure, which was further enhanced with miglustat, suggesting a stabilizing effect of chaperone on ATB200.
- After switching from standard of care to ATB200/miglustat, patients generally showed an improvement in biomarkers of muscle damage, with many patients demonstrating normalization by week 18.
- The initial 2 treatment-naive patients treated with ATB200/miglustat demonstrated robust reduction in all biomarkers of muscle damage

## Claims

1. A method for producing purified recombinant human acid alpha-glucosidase (rhGAA), wherein the purified rhGAA comprises an amino acid sequence that is at least 98% identical to SEQ ID NO: 2, the method comprising:
in a bioreactor, culturing host cells that secrete the rhGAA;
removing media from the bioreactor;
filtering the media to provide a filtrate;
loading the filtrate onto an anion exchange chromatography (AEX) column to capture the rhGAA;
eluting the rhGAA from the AEX column;
loading the rhGAA eluted from the AEX column onto an immobilized metal affinity chromatography (IMAC) column; and
eluting the rhGAA from the IMAC column.

2. The method of claim 1, wherein the purified rhGAA comprises a first potential N-glycosylation site, a second potential N-glycosylation site, a third potential N-glycosylation site, a fourth potential N-glycosylation site, a fifth potential N-glycosylation site, a sixth potential N-glycosylation site, and a seventh potential N-glycosylation site.

3. The method of claim 1 or claim 2, further comprising:
loading the rhGAA eluted from the IMAC column onto a third chromatography column; and
eluting the rhGAA from the third chromatography column.

4. The method of claim 3, wherein the third chromatography column is selected from a cation exchange chromatography (CEX) column and a size exclusion chromatography (SEC) column.

5. The method of any of claims 1-4, wherein filtering the media is selected from alternating tangential flow filtration (ATF) and tangential flow filtration (TFF).

6. The method of any of claims 1-5, further comprising inactivating viruses in one or more of the rhGAA eluted from the AEX column, the rhGAA eluted from the IMAC column, and the rhGAA eluted from the third chromatography column.

7. The method of claim 3, further comprising filtering the rhGAA eluted from the IMAC column or the rhGAA eluted from the third chromatography column to provide a filtered product.

8. The method of claim 7, further comprising lyophilizing the filtered product.

9. The method of any of claims 1-8, wherein the host cells comprise Chinese hamster ovary (CHO) cells.

10. The method of any of claims 1-9, wherein:
(i) at least 90% of the purified rhGAA binds to cation-independent mannose-6-phosphate receptor (CIMPR); or
(ii) at least 90% of the purified rhGAA comprises an N-glycan carrying mono-mannose-6-phosphate (mono-M6P) or bis-mannose-6-phosphate (bis-M6P).

## Patentansprüche

1. Verfahren zur Herstellung von gereinigter rekombinanter humaner saurer Alpha-Glucosidase (rhGAA), wobei die gereinigte rhGAA eine Aminosäuresequenz umfasst, die zu mindestens 98 % mit SEQ ID NO: 2 identisch ist, wobei das Verfahren umfasst:
in einem Bioreaktor, Kultivieren von Wirtszellen, die die rhGAA sekretieren;
Entnehmen von Medium aus dem Bioreaktor;
Filtrieren des Mediums, um ein Filtrat bereitzustellen;
Laden des Filtrats auf eine Anionenaustauschchromatographie (AEX)-Säule, um die rhGAA einzufangen;
Eluieren der rhGAA von der AEX-Säule;
Laden der von der AEX-Säule eluierten rhGAA auf eine immobilisierte Metallaffinitätschromatographie (IMAC)-Säule; und
Eluieren der rhGAA von der IMAC-Säule.

2. Verfahren gemäß Anspruch 1, wobei die gereinigte rhGAA eine erste potenzielle N-Glykosylierungsstelle, eine zweite potenzielle N-Glykosylierungsstelle, eine dritte potenzielle N-Glykosylierungsstelle, eine vierte potenzielle N-Glykosylierungsstelle, eine fünfte potenzielle N-Glykosylierungsstelle, eine sechste potenzielle N-Glykosylierungsstelle und eine siebte potenzielle N-Glykosylierungsstelle umfasst.

3. Verfahren gemäß Anspruch 1 oder Anspruch 2, ferner umfassend:
Laden der von der IMAC-Säule eluierten rhGAA auf eine dritte Chromatographiesäule; und
Eluieren der rhGAA von der dritten Chromatographiesäule.

4. Verfahren gemäß Anspruch 3, wobei die dritte Chromatographiesäule ausgewählt ist von einer Kationenaustausch (CEX)-Chromatographiesäule und einer Größenausschluss (SEC)-Chromatographiesäule.

5. Verfahren gemäß irgendeinem der Ansprüche 1-4, wobei Filtrieren des Mediums ausgewählt ist aus alternierender Tangentialflussfiltration (ATF)
und Tangentialflussfiltration (TTF).

6. Verfahren gemäß irgendeinem der Ansprüche 1-5, ferner umfassend Inaktivieren von Viren in einer oder mehrerer der von der AEX-Säule eluierten rhGAA, der von der IMAC-Säule eluierten rhGAA und der von der dritten Chromatographiesäule eluierten rhGAA.

7. Verfahren gemäß Anspruch 3, ferner umfassend Filtrieren der von der IMAC-Säule eluierten rhGAA oder der von der dritten Chromatographiesäule eluierten rhGAA, um ein filtriertes Produkt bereitzustellen.

8. Verfahren gemäß Anspruch 7, ferner umfassend Lyophilisieren des filtrierten Produkts.

9. Verfahren gemäß irgendeinem der Ansprüche 1-8, wobei die Wirtszellen Chinesische Hamster Ovarialzellen (CHO Zellen) umfassen.

10. Verfahren gemäß irgendeinem der Ansprüche 1-9, wobei:
(i) mindestens 90% der gereinigten rhGAA an kationenunabhängigen Mannose-6-Phosphat-Rezeptor (CIMPR) bindet; oder
(ii) mindestens 90% der gereinigten rhGAA ein N-Glykan, das Mono-Mannose-6-Phosphat (Mono-M6P) oder Bis-Mannose-6-Phosphat (Bis-M6P) trägt, umfasst.

## Revendications

1. Procédé de production d'alpha-glucosidase acide humaine recombinante (rhGAA) purifiée, dans lequel la rhGAA purifiée comprend une séquence d'acides aminés qui présente une identité d'au moins 98 % avec la SEQ ID NO : 2, le procédé comprenant :
dans un bioréacteur, cultiver des cellules hôtes qui sécrètent la rhGAA ;
retirer le milieu du bioréacteur ;
filtrer le milieu afin de fournir un filtrat ;
charger le filtrat sur une colonne de chromatographie d'échange d'anions (AEX) pour capturer la rhGAA ;
éluer la rhGAA de la colonne AEX ;
charger la rhGAA éluée de la colonne AEX sur une colonne de chromatographie d'affinité sur ions métalliques immobilisés (IMAC) ; et
éluer la rhGAA de la colonne IMAC.

2. Procédé selon la revendication 1, dans lequel la rhGAA purifiée comprend un premier site potentiel de N-glycosylation, un deuxième site potentiel de N-glycosylation, un troisième site potentiel de N-glycosylation, un quatrième site potentiel de N-glycosylation, un cinquième site potentiel de N-glycosylation, un sixième site potentiel de N-glycosylation, et un septième site potentiel de N-glycosylation.

3. Procédé selon la revendication 1 ou la revendication 2, comprenant en outre :
charger la rhGAA éluée de la colonne IMAC sur une troisième colonne de chromatographie ; et
éluer la rhGAA de la troisième colonne de chromatographie.

4. Procédé selon la revendication 3, dans lequel la troisième colonne de chromatographie est sélectionnée parmi une colonne de chromatographie d'échange de cations (CEX) et une colonne de chromatographie d'exclusion de taille (SEC).

5. Procédé selon l'une quelconque des revendications 1 à 4, dans lequel la filtration du milieu est sélectionnée parmi une filtration à écoulement tangentiel alterné (ATF) et une filtration à écoulement tangentiel (TFF).

6. Procédé selon l'une quelconque des revendications 1 à 5, comprenant en outre l'inactivation de virus dans un ou plusieurs parmi la rhGAA éluée de la colonne AEX, la rhGAA éluée de la colonne IMAC, et la rhGAA éluée de la troisième colonne de chromatographie.

7. Procédé selon la revendication 3, comprenant en outre le filtrage de la rhGAA éluée de la colonne IMAC ou de la rhGAA éluée de la troisième colonne de chromatographie afin de fournir un produit filtré.

8. Procédé selon la revendication 7, comprenant en outre la lyophilisation du produit filtré.

9. Procédé selon l'une quelconque des revendications 1 à 8, dans lequel les cellules hôtes comprennent des cellules ovariennes de hamster chinois (CHO).

10. Procédé selon l'une quelconque des revendications 1 à 9, dans lequel :
(i) au moins 90 % de la rhGAA purifiée se lie au récepteur du mannose-6-phosphate indépendant des cations (CIMPR) ; ou
(ii) au moins 90 % de la rhGAA purifiée comprend un N-glycane portant du mono-mannose-6-phosphate (mono-M6P) ou du bis-mannose-6-phosphate (bis-M6P).
